(19)

Europäisches
Patentamt
European
Patent Office
Office européen
des brevets

(11) **EP 1 928 226 B1**

(12) **EUROPEAN PATENT SPECIFICATION**

(45) Date of publication and mention
of the grant of the patent:
**19.10.2011 Bulletin 2011/42**

(51) Int Cl.:
***C12N 15/82*** *(2006.01)* ***A01H 5/00*** *(2006.01)*

(21) Application number: **06814197.7**

(22) Date of filing: **31.08.2006**

(86) International application number:
**PCT/US2006/034615**

(87) International publication number:
**WO 2007/028165 (08.03.2007 Gazette 2007/10)**

(54) **Stress tolerance in plants**

Stresstoleranz in Pflanzen

Tolérance de plantes au stress

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **31.08.2005 US 713952 P**

(43) Date of publication of application:
**11.06.2008 Bulletin 2008/24**

(73) Proprietor: **Mendel Biotechnology, Inc.
Hayward, CA 94545 (US)**

(72) Inventors:
• **GUTTERSON, Neal, I
Oakland, California 94618 (US)**
• **RATCLIFFE, Oliver, J.
Oakland, California 94606 (US)**
• **REUBER, T., Lynne
San Mateo, California 94402 (US)**
• **CENTURY, Karen, S.
Albany, California 94706 (US)**
• **KROLIKOWSKI, Katherine
Richmond, California 94804 (US)**
• **COSTA, Jennifer, M.
Union City, California 94587 (US)**
• **CREELMAN, Robert, A.
Castro Valley, California 94546 (US)**
• **HEMPEL, Frederick, D.
Albany, California 94706 (US)**
• **KUMIMOTO, Roderick, W.
San Bruno, California 94066 (US)**
• **QUEEN, Emily, L.
San Bruno, California 94066 (US)**
• **REPETTI, Peter
Emeryville, California 94608 (US)**
• **ADAM, Luc
Hayward, California 94545 (US)**

(74) Representative: **Brasnett, Adrian Hugh et al
Mewburn Ellis LLP
33 Gutter Lane
London
EC2V 8AS (GB)**

(56) References cited:
WO-A-01/77311    WO-A-2004/031349
WO-A-2004/076638    WO-A-2005/033319
WO-A-2005/047516    WO-A-2007/044043
US-A1- 2004 019 925    US-A1- 2004 019 927

• LI X-Y ET AL: "EVOLUTIONARY VARIATION OF
THE CCAAT-BINDING TRANSCRIPTION
FACTOR NF-Y" NUCLEIC ACIDS RESEARCH,
OXFORD UNIVERSITY PRESS, SURREY, GB, vol.
20, no. 5, 11 March 1992 (1992-03-11), pages
1087-1091, XP000670347 ISSN: 0305-1048
• YI S Y ET AL: "The pepper transcription factor
CaPF1 confers pathogen and freezing tolerance
in Arabidopsis" PLANT PHYSIOLOGY,
AMERICAN SOCIETY OF PLANT
PHYSIOLOGISTS, ROCKVILLE, MD, US, vol. 136,
no. 1, 1 September 2004 (2004-09-01), pages
2862-2874, XP003005433 ISSN: 0032-0889
• JAGLO-OTTOSEN ET AL: "Arabidopsis thaliana
CBF1 overexpression induces COR genes and
enhances freezing tolerance" SCIENCE,
AMERICAN ASSOCIATION FOR THE
ADVANCEMENT OF SCIENCE, US,
WASHINGTON, DC, vol. 280, 3 April 1998
(1998-04-03), pages 104-106, XP002096424 ISSN:
0036-8075

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• KASUGA MIE ET AL: "Improving plant drought, salt, and freezing tolerance by gene transfer of a single stress-inducible transcription factor" NATURE BIOTECHNOLOGY, NATURE PUBLISHING GROUP, NEW YORK, NY, US, vol. 17, no. 3, 1 March 1999 (1999-03-01), pages 287-291, XP002173128 ISSN: 1087-0156

• PEARCE R S: "MOLECULAR ANALYSIS OF ACCLIMATION TO COLD" PLANT GROWTH REGULATION, DORDRECHT, vol. 29, no. 1/02, 1 September 1999 (1999-09-01), pages 47-76, XP008005545 ISSN: 0167-6903

• DATABASE GENBANK [Online] 20 November 2004 YAMAGUCHI-SHINOZAKI ET AL., XP008116860 Database accession no. (D13044)

• KASUGA M. ET AL.: 'Improving plant drought, salt, and freezing tolerance by gene transfer of a single stress-inducible transcription factor' NAT. BIOTECHNOL. vol. 17, no. 3, March 1999, pages 287 - 291, XP002173128

## Description

### FIELD OF THE INVENTION

The present invention relates to plant genomics and plant improvement.

### BACKGROUND OF THE INVENTION

Abiotic stress and yield. In the natural environment, plants often grow under unfavorable conditions, such as drought (low water availability), salinity, chilling, freezing, high temperature, flooding, or strong light. Any of these abiotic stresses can delay growth and development, reduce productivity, and in extreme cases, cause the plant to die. Enhanced tolerance to these stresses would lead to yield increases in conventional varieties and reduce yield variation in hybrid varieties. Of these stresses, low water availability is a major factor in crop yield reduction worldwide.

Water deficit is a common component of many plant stresses. Water deficit occurs in plant cells when the whole plant transpiration rate exceeds the water uptake. In addition to drought, other stresses, such as salinity and low temperature, produce cellular dehydration (McCue and Hanson, 1990).

Salt (and drought) stress signal transduction consists of ionic and osmotic homeostasis signaling pathways. The ionic aspect of salt stress is signaled via the SOS pathway where a calcium-responsive SOS3-SOS2 protein kinase complex controls the expression and activity of ion transporters such as SOS1. The pathway regulating ion homeostasis in response to salt stress has been reviewed recently by Xiong and Zhu (2002a).

The osmotic component of salt-stress involves complex plant reactions that are possibly overlapping with drought- and/or cold-stress responses. Common aspects of drought-, cold- and salt-stress response have been reviewed by Xiong and Zhu (2002). These include:

Abscisic acid (ABA) biosynthesis is regulated by osmotic stress at multiple steps. Both ABA-dependent and -independent osmotic stress signaling first modify constitutively expressed transcription factors, leading to the expression of early response transcriptional activators, which then activate downstream stress tolerance effector genes.

Based on the commonality of many aspects of cold, drought, and salt stress responses, it can be concluded that genes that increase tolerance to cold or salt stress can also improve drought stress protection. In fact, this has already been demonstrated for transcription factors (in the case of AtCBF/DREB1) and for other genes such as OsCDPK7 (Saijo et al. (2000)), or AVP1 (a vacuolar pyrophosphatase-proton-pump, Gaxiola et al. (2001)).

Transcription factors (TFs) and other genes involved in both abiotic and biotic stress resistance. Despite the evidence for negative cross-talk between drought and disease response pathways, a number of genes have been shown to function in both pathways, indicating possible convergence of the signal transduction pathways. There are numerous examples of genes that are inducible by multiple stresses. For instance, a global TxP analysis revealed classes of transcription factor that are mainly induced by abiotic stresses or disease, but also a class of transcription factors induced both by abiotic stress and bacterial infection (Chen et al. (2002a)).

Implications for crop improvement. Plant responses to drought and disease interact at a number of levels. Although dry conditions do not favor most pathogens, plant defenses may be weakened by metabolic stress or hormonal cross-talk, increasing vulnerability to pathogens that can infect under drought conditions. However, there is also evidence for convergence of abiotic and biotic stress response pathways, based on genes that confer tolerance to multiple stresses. Given our incomplete understanding of these signaling interactions, plants with positive alterations in one stress response should be examined carefully for possible alterations in other stress responses.

Plant transcription factors are disclosed in US 2004/019927; WO 01/77311; WO 2004/076638 A; and WO 2005/047516.

### SUMMARY OF THE INVENTION

The present invention pertains to the use of the transcription factor polynucleotides and the polypeptide , and expression vectors that comprise these sequences. The present invention provides the Use of a the comprising sequence

```
MAEAPASPGGGGGSHESGSPRGGGGGGGSVREQDRFLPIANISRIMKKAIPANG
KIAKDAKETVQECVSEFISFITSEASDKCQREKRKTINGDDLLWAMATLGFED
YIEPLKVYLQKYREMEGDSKLTAKSSDGSIKKDALGHVGASSSAAQGMGQQGA
YNQGMGYMQPQYHNGDISN   (SEQ ID NO: 8)
```

nucleotide sequence which encodes a polypeptide comprising SEQ ID NO:8 for producing a transgenic plant wherein over-expression of said polypeptide provides the trait of increased tolerance to cold; wherein said tolerance to cold is tolerance to chilling at 8˚C during germination. In one aspect, said nucleotide sequence which encodes a polypeptide is operably linked to a constitutive, inducible or tissue-active promoter. The plant may be an angiosperm.

The invention further provides a method for producing transgenic plant having the trait of increased tolerance to cold during germination, said method comprising: producing a transgenic plant having an expression vector comprising a nucleotide sequence which encodes a polypeptide comprising SEQ ID NO:8 wherein over-expression of said polypeptide provides the trait of increased tolerance to cold, wherein said tolerance to cold is tolerance to chilling at 8˚C during germination; and selecting a plant having said trait of increased cold tolerance caused by over-expression of said polypeptide. In one aspect, the nucleotide sequence which encodes the polypeptide is operably linked to a constitutive, inducible or tissue-active promoter. The plant may be an angiosperm.

Alternatively, the expression vector may comprise a polynucleotide that encodes a transcription factor polypeptide sequence fused to a GAL4 activation domain, thus creating either a C-terminal or an N-terminal GAL4 activation domain protein fusion.

Transgenic plants that are transformed with these expression vectors, and seed produced by these transgenic plants that comprise any of the sequences of the invention, are also described herein.

The invention may be used in methods for increasing the yield of a plant growing in conditions of stress, as compared to a wild-type plant of the same species growing in the same conditions of stress. In this case, the plant is transformed with a polynucleotide sequence encoding a transcription factor polypeptide, where the polynucleotide is operably linked to a constitutive, inducible or tissue-specific promoter. The transformed plant that ectopically expresses the transcription factor polypeptide is then selected, and this plant may have greater yield than a wild-type plant of the same species (that is, a non-transformed plant), when the transformed plant is grown in conditions of cold.

Brief Description of the Drawings

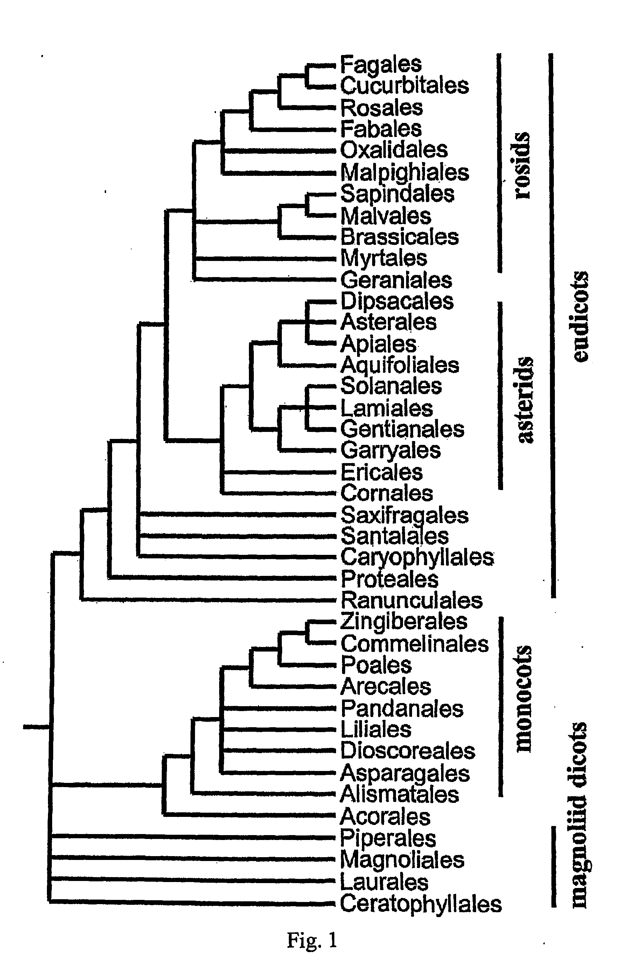

Figure 1 shows a conservative estimate of phylogenetic relationships among the orders of flowering plants (modified from Soltis et al. (1997)). Those plants with a single cotyledon (monocots) are a monophyletic clade nested within at least two major lineages of dicots; the eudicots are further divided into rosids and asterids. *Arabidopsis* is a rosid eudicot classified within the order Brassicales; rice is a member of the monocot order Poales. Figure 1 was adapted from Daly et al. (2001).

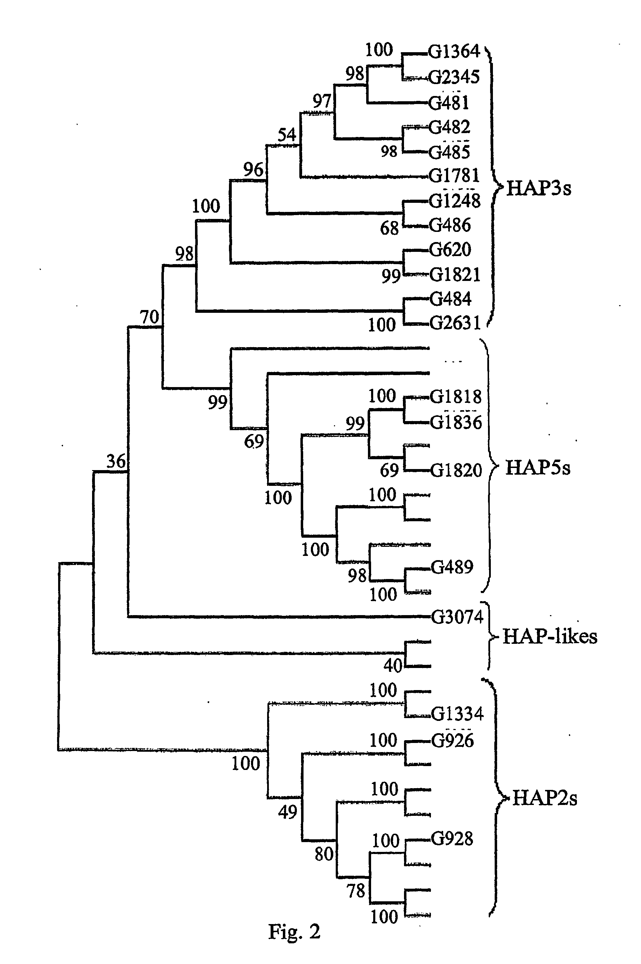

Figure 2: Phylogenetic tree of CAAT family proteins. There are three main sub-classes within the family: the HAP2 (also known as the NF-YA subclass), HAP3 (NF-YB subclass) and HAP5 (NF-YC subclass) related proteins. Three additional proteins were identified that did not clearly cluster with any of the three main groups and we have designated these as HAP-like proteins. G620, SEQ ID NO: 66, corresponds to *LEAFY COTYLEDON 1 (LEC1;* Lotan et al., 1998) and G1821, corresponds to *LEAFY COTYLEDON 1-LIKE (L1L;* Kwong et al., 2003). Other sequences shown in this tree include G1364 (SEQ ID NO: 14), G2345 (SEQ ID NO: 22), G481 (SEQ ID NO: 2), G482 (SEQ ID NO: 28), G485 (SEQ ID NO: 18), G1781 (SEQ ID NO: 56), G1248 (SEQ ID NO: 68), G486 (SEQ ID NO: 64), G484 (SEQ ID NO: 62), G2631 (SEQ ID NO: 70), G1818 (SEQ ID NO: 74), G1836 (SEQ ID NO: 48), G1820 (SEQ ID NO: 44), G489 (SEQ ID NO: 46), G3074 (SEQ ID NO: 76), G1334 (SEQ ID NO: 54), G926 (SEQ ID NO: 52), and G928 (SEQ ID NO: 72). The tree was based on a ClustalW alignment of full-length proteins using Mega 2 software (protein sequences are provided in the Sequence Listing).

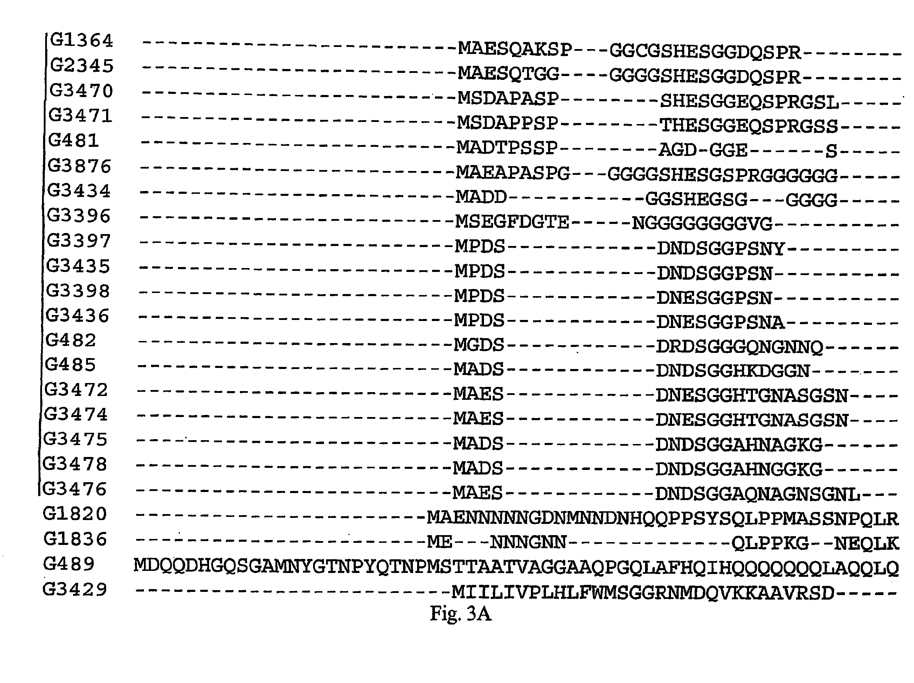

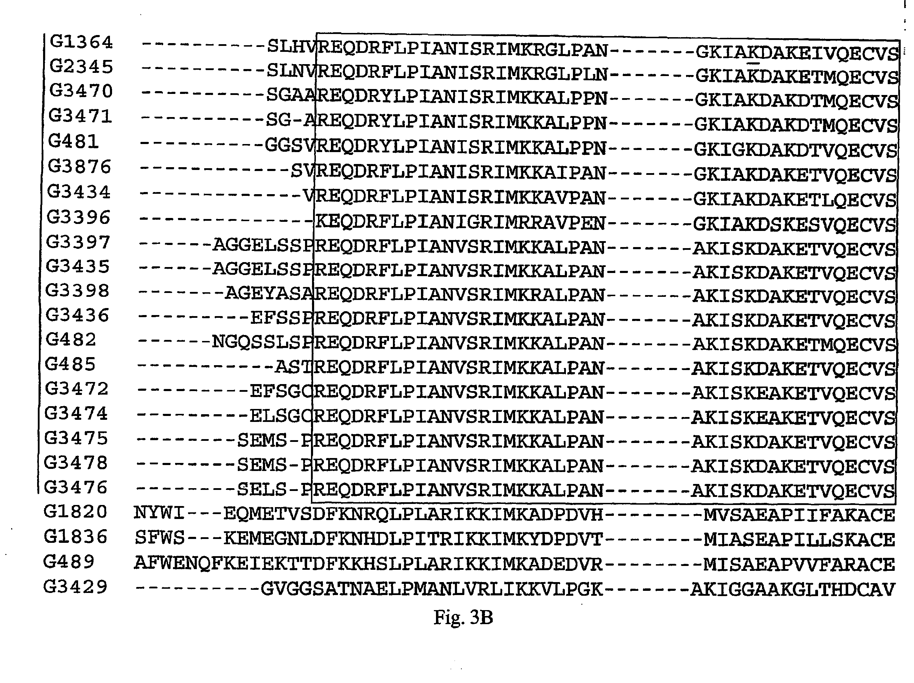

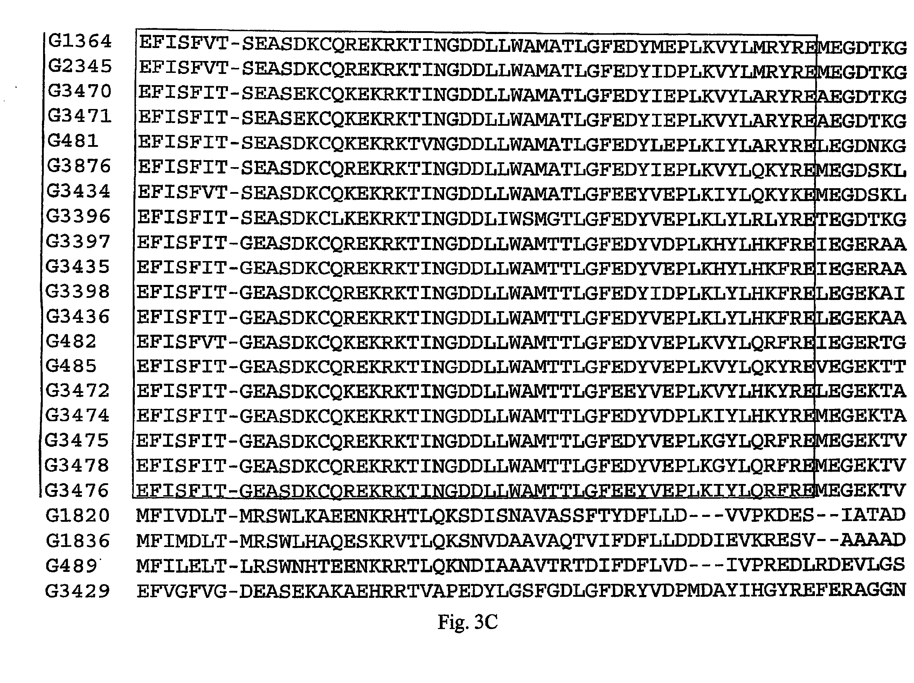

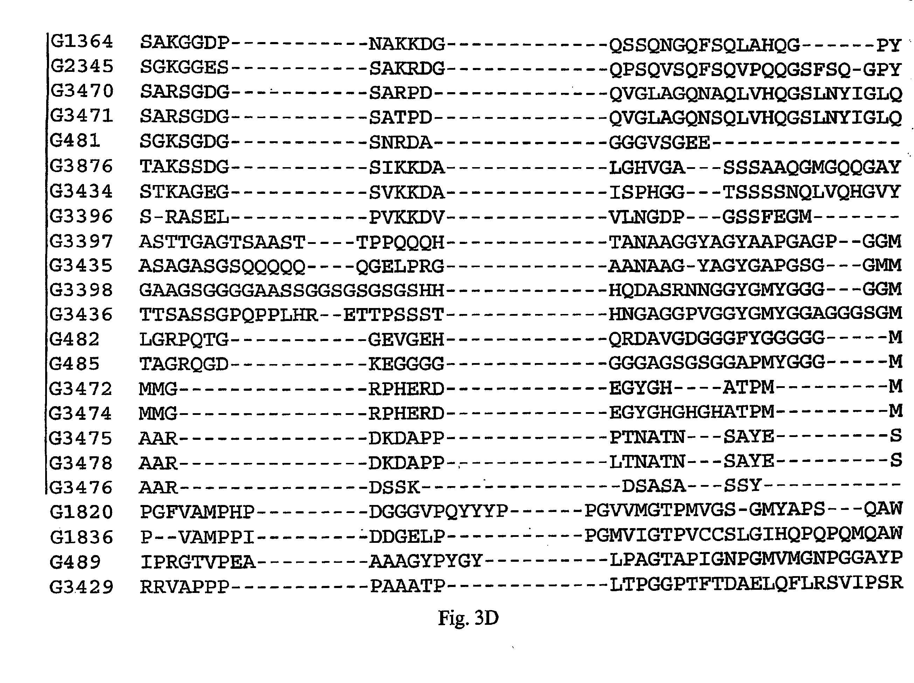

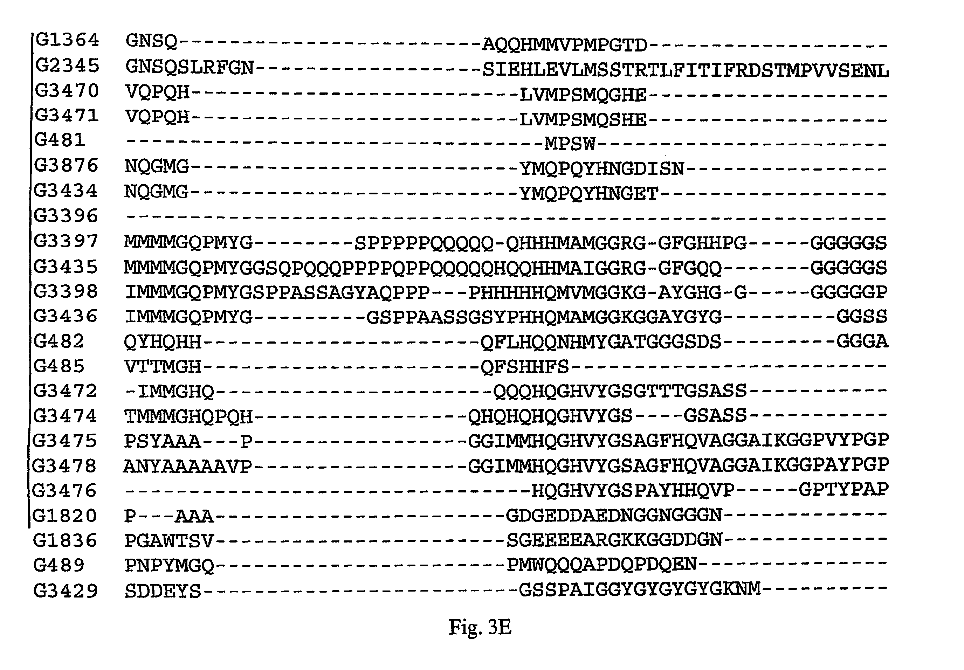

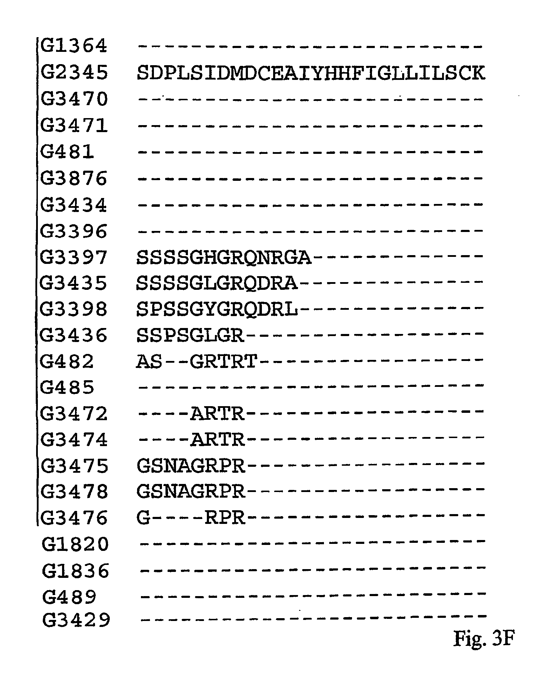

In Figures 3A-3F, the alignments of G481, G482, G485, G1364, G2345, G1781 and related sequences are presented. These sequences from *Arabidopsis* (At) are shown aligned with soybean (Gm), rice (Os) and corn (Zm) sequences with the B domains indicated by the large box that spans Figures 3B through 3C. The vertical line to the left in each page of the alignment indicates G482 clade members.

## DETAILED DESCRIPTION

As used herein and in the appended claims, the singular forms "a", "an", and "the" include the plural reference unless the context clearly dictates otherwise. Thus, for example, a reference to "a host cell" includes a plurality of such host cells, and a reference to "a stress" is a reference to one or more stresses and equivalents thereof known to those skilled in the art, and so forth.

DEFINITIONS

"Nucleic acid molecule" refers to an oligonucleotide, polynucleotide or any fragment thereof. It may be DNA or RNA of genomic or synthetic origin, double-stranded or single-stranded, and combined with carbohydrate, lipids, protein, or other materials to perform a particular activity such as transformation or form a useful composition such as a peptide nucleic acid (PNA).

"Polynucleotide" is a nucleic acid molecule comprising a plurality of polymerized nucleotides, e.g., at least about 15 consecutive polymerized nucleotides. A polynucleotide may be a nucleic acid, oligonucleotide, nucleotide, or any fragment thereof. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single-stranded or double-stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can be combined with carbohydrate, lipids, protein, or other materials to perform a particular activity such as transformation or form a useful composition such as a peptide nucleic acid (PNA). The polynucleotide can comprise a sequence in either sense or antisense orientations. "Oligonucleotide" is substantially equivalent to the terms amplimer, primer, oligomer, element, target, and probe and is preferably single-stranded.

"Gene" or "gene sequence" refers to the partial or complete coding sequence of a gene, its complement, and its 5' or 3' untranslated regions. A gene is also a functional unit of inheritance, and in physical terms is a particular segment or sequence of nucleotides along a molecule of DNA (or RNA, in the case of RNA viruses) involved in producing a polypeptide chain. The latter may be subjected to subsequent processing such as chemical modification or folding to obtain a functional protein or polypeptide. A gene may be isolated, partially isolated, or found with an organism's genome. By way of example, a transcription factor gene encodes a transcription factor polypeptide, which may be functional or require processing to function as an initiator of transcription.

Operationally, genes may be defined by the cis-trans test, a genetic test that determines whether two mutations occur in the same gene and that may be used to determine the limits of the genetically active unit (Rieger et al. (1976)). A gene generally includes regions preceding ("leaders"; upstream) and following ("trailers"; downstream) the coding region. A gene may also include intervening, non-coding sequences, referred to as "introns", located between individual coding segments, referred to as "exons". Most genes have an associated promoter region, a regulatory sequence 5' of the transcription initiation codon (there are some genes that do not have an identifiable promoter). The function of a gene may also be regulated by enhancers, operators, and other regulatory elements.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide, whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "polypeptide" is an amino acid sequence comprising a plurality of consecutive polymerized amino acid residues e.g., at least about 15 consecutive polymerized amino acid residues. In many instances, a polypeptide comprises a polymerized amino acid residue sequence that is a transcription factor or a domain or portion or fragment thereof. Additionally, the polypeptide may comprise: (i) a localization domain; (ii) an activation domain; (iii) a repression domain; (iv) an oligomerization domain; (v) a DNA-binding domain; or the like. The polypeptide optionally comprises modified amino acid residues, naturally occurring amino acid residues not encoded by a codon, non-naturally occurring amino acid residues.

"Protein" refers to an amino acid sequence, oligopeptide, peptide, polypeptide or portions thereof whether naturally occurring or synthetic.

"Portion", as used herein, refers to any part of a protein used for any purpose, but especially for the screening of a library of molecules which specifically bind to that portion or for the production of antibodies.

A "recombinant polypeptide" is a polypeptide produced by translation of a recombinant polynucleotide. A "synthetic polypeptide" is a polypeptide created by consecutive polymerization of isolated amino acid residues using methods well known in the art. An "isolated polypeptide," whether a naturally occurring or a recombinant polypeptide, is more enriched in (or out of) a cell than the polypeptide in its natural state in a wild-type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild-type plant. Alternatively, or additionally, the isolated polypeptide is separated from other cellular components with which it is typically associated, e.g., by any of the various protein purification methods herein.

"Homology" refers to sequence similarity between a reference sequence and at least a fragment of a newly sequenced clone insert or its encoded amino acid sequence.

"Identity" or "similarity" refers to sequence similarity between two polynucleotide sequences or between two polypeptide sequences, with identity being a more strict comparison. The phrases "percent identity" and "% identity" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100% similar, or any integer value therebetween. Identity or similarity can be determined by comparing a position in each sequence that may be aligned for purposes of comparison. When a position in the compared sequence is occupied by the same nucleotide base or amino acid, then the molecules are identical at that position. A degree of similarity or identity between polynucleotide sequences is a function of the number of identical, matching or corresponding nucleotides at positions shared by the polynucleotide sequences. A degree of identity of polypeptide sequences is a function of the number of identical amino acids at corresponding positions shared by the polypeptide sequences. A degree of homology or similarity of polypeptide sequences is a function of the number of amino acids at corresponding positions shared by the polypeptide sequences.

"Alignment" refers to a number of nucleotide bases or amino acid residue sequences aligned by lengthwise comparison so that components in common (i.e., nucleotide bases or amino acid residues at corresponding positions) may be visually and readily identified. The fraction or percentage of components in common is related to the homology or identity between the sequences. Alignments such as those of Figures 3A-3F may be used to identify conserved domains and relatedness within these domains. An alignment may suitably be determined by means of computer programs known in the art, such as MACVECTOR software (1999) (Accelrys, Inc., San Diego, CA).

A "conserved domain" or "conserved region" as used herein refers to a region in heterologous polynucleotide or polypeptide sequences where there is a relatively high degree of sequence identity between the distinct sequences. For example, an "AT-hook" domain", such as is found in a polypeptide member of AT-hook transcription factor family, is an example of a conserved domain. An "AP2" domain", such as is found in a polypeptide member of AP2 transcription factor family, is another example of a conserved domain. With respect to polynucleotides encoding presently disclosed transcription factors, a conserved domain is preferably at least nine base pairs (bp) in length. A conserved domain with respect to presently disclosed polypeptides refers to a domain within a transcription factor family that exhibits a higher degree of sequence homology, such as at least about 38% sequence identity including conservative substitutions, or at least about 55% sequence identity, or at least about 62% sequence identity, or at least about 65%, or at least about 70%, or at least about 75%, or at least about 78%, or at least about 80%, or at least about 82%, or at least about 85%, %, or at least about 90%, or at least about 95%, amino acid residue sequence identity, to a conserved domain of a polypeptide. Sequences that possess or encode for conserved domains that meet these criteria of percentage identity, and that have comparable biological activity to the present transcription factor sequences, thus being members of the G1073 clade of transcription factor polypeptides, are disclosed. A fragment or domain can be referred to as outside a conserved domain, outside a consensus sequence, or outside a consensus DNA-binding site that is known to exist or that exists for a particular transcription factor class, family, or sub-family. In this case, the fragment or domain will not include the exact amino acids of a consensus sequence or consensus DNA-binding site of a transcription factor class, family or sub-family, or the exact amino acids of a particular transcription factor consensus sequence or consensus DNA-binding site. Furthermore, a particular fragment, region, or domain of a polypeptide, or a polynucleotide encoding a polypeptide, can be "outside a conserved domain" if all the amino acids of the fragment, region, or domain fall outside of a defined conserved domain(s) for a polypeptide or protein. Sequences having lesser degrees of identity but comparable biological activity are considered to be equivalents.

As one of ordinary skill in the art recognizes, conserved domains may be identified as regions or domains of identity to a specific consensus sequence (see, for example, Riechmann et al. (2000a, 2000b)). Thus, by using alignment methods well known in the art, the conserved domains of the plant transcription factors, for example, for the AT-hook proteins (Reeves and Beckerbauer (2001); and Reeves (2001)), may be determined.

The conserved domains for many of the transcription factor sequences are listed in Tables 8 -17. Also, the polypeptides of Tables 8 -17 have conserved domains specifically indicated by amino acid coordinate start and stop sites. A comparison of the regions of these polypeptides allows one of skill in the art (see, for example, Reeves and Nissen (1995)) to identify domains or conserved domains for any of the polypeptides listed or referred to in this disclosure.

"Complementary" refers to the natural hydrogen bonding by base pairing between purines and pyrimidines. For example, the sequence A-C-G-T (5' -> 3') forms hydrogen bonds with its complements A-C-G-T (5' -> 3') or A-C-G-U (5' -> 3'). Two single-stranded molecules may be considered partially complementary, if only some of the nucleotides bond, or "completely complementary" if all of the nucleotides bond. The degree of complementarity between nucleic acid strands affects the efficiency and strength of hybridization and amplification reactions. "Fully complementary" refers to the case where bonding occurs between every base pair and its complement in a pair of sequences, and the two sequences have the same number of nucleotides.

The terms "highly stringent" or "highly stringent condition" refer to conditions that permit hybridization of DNA strands whose sequences are highly complementary, wherein these same conditions exclude hybridization of significantly mismatched DNAs. Polynucleotide sequences capable of hybridizing under stringent conditions with the polynucleotides described herein may be, for example, variants of the disclosed polynucleotide sequences, including allelic or splice variants, or sequences that encode orthologs or paralogs of presently disclosed polypeptides. Nucleic acid hybridization methods are disclosed in detail by Kashima et al. (1985), Sambrook et al. (1989), and by Haymes et al. (1985).

In general, stringency is determined by the temperature, ionic strength, and concentration of denaturing agents (e.g., formamide) used in a hybridization and washing procedure (for a more detailed description of establishing and determining stringency, see the section "Identifying Polynucleotides or Nucleic Acids by Hybridization", below). The degree to which two nucleic acids hybridize under various conditions of stringency is correlated with the extent of their similarity. Thus, similar nucleic acid sequences from a variety of sources, such as within a plant's genome (as in the case of paralogs) or from another plant (as in the case of orthologs) that may perform similar functions can be isolated on the basis of their ability to hybridize with known transcription factor sequences. Numerous variations are possible in the conditions and means by which nucleic acid hybridization can be performed to isolate transcription factor sequences having similarity to transcription factor sequences known in the art and are not limited to those explicitly disclosed herein. Such an approach may be used to isolate polynucleotide sequences having various degrees of similarity with disclosed transcription factor sequences, such as, for example, encoded transcription factors having 38% or greater identity with the conserved domain of disclosed transcription factors.

The terms "paralog" and "ortholog" are defined below in the section entitled "Orthologs and Paralogs". In brief, orthologs and paralogs are evolutionarily related genes that have similar sequences and functions. Orthologs are structurally related genes in different species that are derived by a speciation event. Paralogs are structurally related genes within a single species that are derived by a duplication event.

The term "equivalog" describes members of a set of homologous proteins that are conserved with respect to function since their last common ancestor. Related proteins are grouped into equivalog families, and otherwise into protein families with other hierarchically defined homology types. This definition is provided at the Institute for Genomic Research (TIGR) World Wide Web (www) website, " tigr.org " under the heading "Terms associated with TIGRFAMs".

In general, the term "variant" refers to molecules with some differences, generated synthetically or naturally, in their base or amino acid sequences as compared to a reference (native) polynucleotide or polypeptide, respectively. These differences include substitutions, insertions, deletions or any desired combinations of such changes in a native polynucleotide of amino acid sequence.

With regard to polynucleotide variants, differences between presently disclosed polynucleotides and polynucleotide variants are limited so that the nucleotide sequences of the former and the latter are closely similar overall and, in many regions, identical. Due to the degeneracy of the genetic code, differences between the former and latter nucleotide sequences may be silent (i.e., the amino acids encoded by the polynucleotide are the same, and the variant polynucleotide sequence encodes the same amino acid sequence as the presently disclosed polynucleotide. Variant nucleotide sequences may encode different amino acid sequences, in which case such nucleotide differences will result in amino acid substitutions, additions, deletions, insertions, truncations or fusions with respect to the similar disclosed polynucleotide sequences. These variations may result in polynucleotide variants encoding polypeptides that share at least one functional characteristic. The degeneracy of the genetic code also dictates that many different variant polynucleotides can encode identical and/or substantially similar polypeptides.

Also disclosed is a variant of a transcription factor nucleic acid , that is, one that encodes a functionally equivalent polypeptide (i.e., a polypeptide having some degree of equivalent or similar biological activity) but differs in sequence due to degeneracy in the genetic code. Included within this definition are polymorphisms that may or may not be readily detectable using a particular oligonucleotide probe of the polynucleotide encoding polypeptide, and improper or unexpected hybridization to allelic variants, with a locus other than the normal chromosomal locus for the polynucleotide sequence encoding polypeptide.

"Allelic variant" or "polynucleotide allelic variant" refers to any of two or more alternative forms of a gene occupying the same chromosomal locus. Allelic variation arises naturally through mutation, and may result in phenotypic polymorphism within populations. Gene mutations may be "silent" or may encode polypeptides having altered amino acid sequence. "Allelic variant" and "polypeptide allelic variant" may also be used with respect to polypeptides, and in this case the terms refer to a polypeptide encoded by an allelic variant of a gene.

"Splice variant" or "polynucleotide splice variant" as used herein refers to alternative forms of RNA transcribed from a gene. Splice variation naturally occurs as a result of alternative sites being spliced within a single transcribed RNA molecule or between separately transcribed RNA molecules, and may result in several different forms of mRNA transcribed from the same gene. Thus, splice variants may encode polypeptides having different amino acid sequences, which may or may not have similar functions in the organism. "Splice variant" or "polypeptide splice variant" may also refer to a polypeptide encoded by a splice variant of a transcribed mRNA.

As used herein, "polynucleotide variants" may also refer to polynucleotide sequences that encode paralogs and orthologs of the presently disclosed polypeptide sequences. "Polypeptide variants" may refer to polypeptide sequences that are paralogs and orthologs of the presently disclosed polypeptide sequences.

Differences between presently disclosed polypeptides and polypeptide variants are limited so that the sequences of the former and the latter are closely similar overall and, in many regions, identical. Presently disclosed polypeptide sequences and similar polypeptide variants may differ in amino acid sequence by one or more substitutions, additions, deletions, fusions and truncations, which may be present in any combination. These differences may produce silent changes and result in a functionally equivalent transcription factor. Thus, it will be readily appreciated by those of skill in the art, that any of a variety of polynucleotide sequences is capable of encoding the transcription factors and transcription factor homolog polypeptides of the invention. A polypeptide sequence variant may have "conservative" changes, wherein a substituted amino acid has similar structural or chemical properties. Deliberate amino acid substitutions may thus be made on the basis of similarity in polarity, charge, solubility, hydrophobicity, hydrophilicity, and/or the amphipathic nature of the residues, as long as a significant amount of the functional or biological activity of the transcription factor is retained. For example, negatively charged amino acids may include aspartic acid and glutamic acid, positively charged amino acids may include lysine and arginine, and amino acids with uncharged polar head groups having similar hydrophilicity values may include leucine, isoleucine, and valine; glycine and alanine; asparagine and glutamine; serine and threonine; and phenylalanine and tyrosine. More rarely, a variant may have "non-conservative" changes, e.g., replacement of a glycine with a tryptophan. Similar minor variations may also include amino acid deletions or insertions, or both. Related polypeptides may comprise, for example, additions and/or deletions of one or more N-linked or O-linked glycosylation sites, or an addition and/or a deletion of one or more cysteine residues. Guidance in determining which and how many amino acid residues may be substituted, inserted or deleted without abolishing functional or biological activity may be found using computer programs well known in the art, for example, DNASTAR software (see USPN 5,840,544).

"Fragment", with respect to a polynucleotide, refers to a clone or any part of a polynucleotide molecule that retains a usable, functional characteristic. Useful fragments include oligonucleotides and polynucleotides that may be used in hybridization or amplification technologies or in the regulation of replication, transcription or translation. A "polynucleotide fragment" refers to any subsequence of a polynucleotide, typically, of at least about 9 consecutive nucleotides, preferably at least about 30 nucleotides, more preferably at least about 50 nucleotides, of any of the sequences provided herein.

Exemplary fragments also include fragments that comprise a region that encodes an conserved domain of a transcription factor. Exemplary fragments also include fragments that comprise a conserved domain of a transcription factor. Exemplary fragments include fragments that comprise an conserved domain of a transcription factor, for example, amino acid residues 33-77 of G682 (SEQ ID NO: 60).

Fragments may also include subsequences of polypeptides and protein molecules, or a subsequence of the polypeptide. Fragments may have uses in that they may have antigenic potential. In some cases, the fragment or domain is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA-binding site or domain that binds to a DNA promoter region, an activation domain, or a domain for protein-protein interactions, and may initiate transcription. Fragments can vary in size from as few as 3 amino acid residues to the full length of the intact polypeptide, but are preferably at least about 30 amino acid residues in length and more preferably at least about 60 amino acid residues in length.

Described is also the production of DNA sequences that encode transcription factors and transcription factor derivatives, or fragments thereof, entirely by synthetic chemistry. After production, the synthetic sequence may be inserted into any of the many available expression vectors and cell systems using reagents well known in the art. Moreover, synthetic chemistry may be used to introduce mutations into a sequence encoding transcription factors or any fragment thereof.

"Derivative" refers to the chemical modification of a nucleic acid molecule or amino acid sequence. Chemical modifications can include replacement of hydrogen by an alkyl, acyl, or amino group or glycosylation, pegylation, or any similar process that retains or enhances biological activity or lifespan of the molecule or sequence.

The term "plant" includes whole plants, shoot vegetative organs/structures (for example, leaves, stems and tubers), roots, flowers and floral organs/structures (for example, bracts, sepals, petals, stamens, carpels, anthers and ovules), seed (including embryo, endosperm, and seed coat) and fruit (the mature ovary), plant tissue (for example, vascular tissue, ground tissue, and the like) and cells (for example, guard cells, egg cells, and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, horsetails, psilophytes, lycophytes, bryophytes, and multicellular algae.

A "control plant" as used in the present invention refers to a plant cell, seed, plant component, plant tissue, plant organ or whole plant used to compare against transgenic or genetically modified plant for the purpose of identifying an enhanced phenotype in the transgenic or genetically modified plant. A control plant may in some cases be a transgenic plant line that comprises an empty vector or marker gene, but does not contain the recombinant polynucleotide that is expressed in the transgenic or genetically modified plant being evaluated. In general, a control plant is a plant of the same line or variety as the transgenic or genetically modified plant being tested. A suitable control plant would include a genetically unaltered or non-transgenic plant of the parental line used to generate a transgenic plant herein.

A "transgenic plant" refers to a plant that contains genetic material not found in a wild-type plant of the same species, variety or cultivar. The genetic material may include a transgene, an insertional mutagenesis event (such as by transposon or T-DNA insertional mutagenesis), an activation'tagging sequence, a mutated sequence, a homologous recombination event or a sequence modified by chimeraplasty. Typically, the foreign genetic material has been introduced into the plant by human manipulation, but any method can be used as one of skill in the art recognizes.

A transgenic plant may contain an expression vector or cassette. The expression cassette typically comprises a polypeptide-encoding sequence operably linked (i.e., under regulatory control of) to appropriate inducible or constitutive regulatory sequences that allow for the controlled expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

"Wild type" or "wild-type", as used herein, refers to a plant cell, seed, plant component, plant tissue, plant organ or whole plant that has not been genetically modified or treated in an experimental sense. Wild-type cells, seed, components, tissue, organs or whole plants may be used as controls to compare levels of expression and the extent and nature of trait modification with cells, tissue or plants of the same species in which a transcription factor expression is altered, e.g., in that it has been knocked out, overexpressed, or ectopically expressed.

A "trait" refers to a physiological, morphological, biochemical, or physical characteristic of a plant or particular plant material or cell. In some instances, this characteristic is visible to the human eye, such as seed or plant size, or can be measured by biochemical techniques, such as detecting the protein, starch, or oil content of seed or leaves, or by observation of a metabolic or physiological process, e.g. by measuring tolerance to water deprivation or particular salt or sugar concentrations, or by the observation of the expression level of a gene or genes, e.g., by employing Northern analysis, RT-PCR, microarray gene expression assays, or reporter gene expression systems, or by agricultural observations such as hyperosmotic stress tolerance or yield. Any technique can be used to measure the amount of, comparative level of, or difference in any selected chemical compound or macromolecule in the transgenic plants, however.

"Trait modification" refers to a detectable difference in a characteristic in a plant ectopically expressing a polynucleotide or polypeptide of the present invention relative to a plant not doing so, such as a wild-type plant. In some cases, the trait modification can be evaluated quantitatively. For example, the trait modification can entail at least about a 2% increase or decrease, or an even greater difference, in an observed trait as compared with a control or wild-type plant. It is known that there can be a natural variation in the modified trait. Therefore, the trait modification observed entails a change of the normal distribution and magnitude of the trait in the plants as compared to control or wild-type plants.

When two or more plants have "similar morphologies", "substantially similar morphologies", "a morphology that is substantially similar", or are "morphologically similar", the plants have comparable forms or appearances, including analogous features such as overall dimensions, height, width, mass, root mass, shape, glossiness, color, stem diameter, leaf size, leaf dimension, leaf density, internode distance, branching, root branching, number and form of inflorescences, and other macroscopic characteristics, and the individual plants are not readily distinguishable based on morphological characteristics alone.

"Modulates" refers to a change in activity (biological, chemical, or immunological) or lifespan resulting from specific binding between a molecule and either a nucleic acid molecule or a protein.

The term "transcript profile" refers to the expression levels of a set of genes in a cell in a particular state, particularly by comparison with the expression levels of that same set of genes in a cell of the same type in a reference state. For example, the transcript profile of a particular transcription factor in a suspension cell is the expression levels of a set of genes in a cell knocking out or overexpressing that transcription factor compared with the expression levels of that same set of genes in a suspension cell that has normal levels of that transcription factor. The transcript profile can be presented as a list of those genes whose expression level is significantly different between the two treatments, and the difference ratios. Differences and similarities between expression levels may also be evaluated and calculated using statistical and clustering methods.

With regard to transcription factor gene knockouts as used herein, the term "knockout" refers to a plant or plant cell having a disruption in at least one transcription factor gene in the plant or cell, where the disruption results in a reduced expression or activity of the transcription factor encoded by that gene compared to a control cell. The knockout can be the result of, for example, genomic disruptions, including transposons, tilling, and homologous recombination, antisense constructs, sense constructs, RNA silencing constructs, or RNA interference. A T-DNA insertion within a transcription factor gene is an example of a genotypic alteration that may abolish expression of that transcription factor gene.

"Ectopic expression or altered expression" in reference to a polynucleotide indicates that the pattern of expression in, e.g., a transgenic plant or plant tissue, is different from the expression pattern in a wild-type plant or a reference plant of the same species. The pattern of expression may also be compared with a reference expression pattern in a wild-type plant of the same species. For example, the polynucleotide or polypeptide is expressed in a cell or tissue type other than a cell or tissue type in which the sequence is expressed in the wild-type plant, or by expression at a time other than at the time the sequence is expressed in the wild-type plant, or by a response to different inducible agents, such as hormones or environmental signals, or at different expression levels (either higher or lower) compared with those found in a wild-type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "overexpression" as used herein refers to a greater expression level of a gene in a plant, plant cell or plant tissue, compared to expression in a wild-type plant, cell or tissue, at any developmental or temporal stage for the gene. Overexpression can occur when, for example, the genes encoding one or more transcription factors are under the control of a strong promoter (e.g., the cauliflower mosaic virus 35S transcription initiation region). Overexpression may also under the control of an inducible or tissue specific promoter. Thus, overexpression may occur throughout a plant, in specific tissues of the plant, or in the presence or absence of particular environmental signals, depending on the promoter used.

Overexpression may take place in plant cells normally lacking expression of polypeptides functionally equivalent or identical to the present transcription factors. Overexpression may also occur in plant cells where endogenous expression of the present transcription factors or functionally equivalent molecules normally occurs, but such normal expression is at a lower level. Overexpression thus results in a greater than normal production, or "overproduction" of the transcription factor in the plant, cell or tissue.

The term "transcription regulating region" refers to a DNA regulatory sequence that regulates expression of one or more genes in a plant when a transcription fact having one or more specific binding domains binds to the DNA regulatory sequence. Transcription factors described herein possess an conserved domain. The transcription factors of the invention also comprise an amino acid subsequence that forms a transcription activation domain that regulates expression of one or more abiotic stress tolerance genes in a plant when the transcription factor binds to the regulating region.

DESCRIPTION OF THE SPECIFIC EMBODIMENTS

Transcription Factors Modify Expression of Endogenous Genes

The plant transcription factor to which the present invention relates belongs to the AT-hook transcription factor family (Reeves and Beckerbauer (2001); and Reeves (2001)).

Generally, the transcription factors are involved in cell differentiation and proliferation and the regulation of growth. Accordingly, one skilled in the art would recognize that by expressing the present sequences in a plant, one may change the expression of autologous genes or induce the expression of introduced genes. By affecting the expression of similar autologous sequences in a plant that have the biological activity of the present sequences, or by introducing the present sequences into a plant, one may alter a plant's phenotype to one with improved traits related to osmotic stresses. Sequences may also be used to transform a plant and introduce desirable traits not found in the wild-type cultivar or strain. Plants may then be selected for those that produce the most desirable degree of over- or under-expression of target genes of interest and coincident trait improvement.

The sequence for use in the present invention may be from any species, particularly plant species, in a naturally occurring form or from any source whether natural, synthetic, semi-synthetic or recombinant. Where "amino acid sequence" is recited to refer to an amino acid sequence of a naturally occurring protein molecule, "amino acid sequence" and like terms are not meant to limit the amino acid sequence to the complete native amino acid sequence associated with the recited protein molecule.

In addition to methods for modifying a plant phenotype by employing one or more polynucleotides and polypeptides described herein, the polynucleotides and polypeptides of SEQ ID NO:8 have a variety of additional uses. These uses include their use in the recombinant production (i.e., expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like; as substrates for cloning e.g., including digestion or ligation reactions; and for identifying exogenous or endogenous modulators of the transcription factors. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

Expression of genes that encode transcription factors that modify expression of endogenous genes, polynucleotides, and proteins are well known in the art. In addition, transgenic plants comprising isolated polynucleotides encoding transcription factors may also modify expression of endogenous genes, polynucleotides, and proteins. Examples include Peng et al. (1997) and Peng et al. (1999). In addition, many others have demonstrated that an *Arabidopsis* transcription factor expressed in an exogenous plant species elicits the same or very similar phenotypic response. See, for example, Fu et al. (2001); Nandi et al. (2000); Coupland (1995); and Weigel and Nilsson (1995)).

In another example, Mandel et al. (1992), and Suzuki et al. (2001), teach that a transcription factor expressed in another plant species elicits the same or very similar phenotypic response of the endogenous sequence, as often predicted in earlier studies of *Arabidopsis* transcription factors in *Arabidopsis* (see Mandel et al. (1992); Suzuki et al. (2001) ). Other examples include Müller et al. (2001); Kim et al. (2001); Kyozuka and Shimamoto (2002) ; Boss and Thomas (2002); He et al. (2000); and Robson et al. (2001).

In yet another example, Gilmour et al. (1998) teach an *Arabidopsis* AP2 transcription factor, CBF 1, which, when overexpressed in transgenic plants, increases plant freezing tolerance. Jaglo et al. (2001) further identified sequences in *Brassica napus* which encode CBF-like genes and that transcripts for these genes accumulated rapidly in response to low temperature. Transcripts encoding CBF-like proteins were also found to accumulate rapidly in response to low temperature in wheat, as well as in tomato. An alignment of the CBF proteins from *Arabidopsis, B. napus,* wheat, rye, and tomato revealed the presence of conserved consecutive amino acid residues, PKK/RPAGRxKFxETRHP and DSAWR, which bracket the AP2/EREBP DNA binding domains of the proteins and distinguish them from other members of the AP2/EREBP protein family. (Jaglo et al. (2001))

Transcription factors mediate cellular responses and control traits through altered expression of genes containing cis-acting nucleotide sequences that are targets of the introduced transcription factor. It is well appreciated in the art that the effect of a transcription factor on cellular responses or a cellular trait is determined by the particular genes whose expression is either directly or indirectly (e.g., by a cascade of transcription factor binding events and transcriptional changes) altered by transcription factor binding. In a global analysis of transcription comparing a standard condition with one in which a transcription factor is overexpressed, the resulting transcript profile associated with transcription factor overexpression is related to the trait or cellular process controlled by that transcription factor. For example, the PAP2 gene and other genes in the MYB family have been shown to control anthocyanin biosynthesis through regulation of the expression of genes known to be involved in the anthocyanin biosynthetic pathway (Bruce et al. (2000); and Borevitz et al. (2000)). Further, global transcript profiles have been used successfully as diagnostic tools for specific cellular states (e.g., cancerous vs. non-cancerous; Bhattacharjee et al. (2001); and Xu et al. (2001)). Consequently, it is evident to one skilled in the art that similarity of transcript profile upon overexpression of different transcription factors would indicate similarity of transcription factor function.

Polypeptides and Polynucleotides

The present invention relates to the use of the transcription factor (TF) of SEQ ID NO:8 and isolated or recombinant polynucleotides encoding this polypeptide.

DESCRIPTION OF THE SPECIFIC EMBODIMENTS

The data presented herein represent the results obtained in experiments with transcription factor polynucleotides and polypeptides that may be expressed in plants for the purpose of reducing yield losses that arise from biotic and abiotic stress.

Background Information for the G482 clade, including G481 and related sequences

G481 (SEQ ID NOs: 1 and 2; AT2G38880; also known as *HAP3A* and *NF-YB1*) *from Arabidopsis* is a member of the HAP3/NF-YB sub-group of the CCAAT binding factor family (CCAAT) of transcription factors (Figure 2). This gene was included based on the resistance to drought-related abiotic stress exhibited by 35S::G481 lines. The major goal of the current program is to define the mechanisms by which G481 confers drought tolerance, and to determine the extent to which other proteins from the CCAAT family, both in *Arabidopsis* and other plant species, have similar functions.

<u>Structural features and assembly of the NF-Y subunits.</u> NF-Y is one of the most heavily studied transcription factor complexes and an extensive literature has accumulated regarding its structure, regulation, and putative roles in various different organisms. Each of the three subunits comprises a region which has been evolutionarily conserved (Li et al. (1992); Mantovani (1999)). In the NF-YA subunits, this conserved region is at the C-terminus, in the NF-YB proteins it is centrally located, and in the NF-YC subunits it is at the N-terminus. The NF-YA and NF-YC subunits also have regions which are rich in glutamine (Q) residues that also show some degree of conservation; these Q-rich regions have an activation domain function. In fact it has been shown that NF-Y contains two transcription activation domains: a glutamine-rich, serine-threonine-rich domain present in the CBF-B (HAP2, NF-YA) subunit and a glutamine-rich domain in the CBF-C (HAP5, CBF-C) subunit (Coustry et al. (1995); Coustry et al. (1996); Coustry et al. (1998); Coustry et al. (2001)). In yeast, Q-regions are not present in the NF-Y subunits and the activation function is thought to be provided by an acidic region in HAP4 (Forsburg and Guarente (1989); Olesen and Guarente (1990); McNabb et al. (1997)), the subunit that is absent from mammals.

The NF-YB and NF-YC subunits bear some similarity to histones; the conserved regions of both these subunits contain a histone fold motif (HFM), which is an ancient domain of ~65 amino acids. The HFM has a high degree of structural conservation across all histones and comprises three or four $\alpha$-helices (four in the case of the NF-Y subunits) which are separated by short loops (L)/strand regions (Arents and Moudrianakis (1995)). In the histones, this HFM domain mediates dimerization and formation of non sequence-specific interactions with DNA (Arents and Moudrianakis (1995)).

Considerable knowledge has now accumulated regarding the biochemistry of NF-Y subunit association and DNA binding. The NF-YB-NF-YC subunits first form a tight dimer, which offers a complex surface for NF-YA association. The resulting trimer can then bind to DNA with high specificity and affinity (Kim and Sheffrey (1990); Bi et al. (1997); Mantovani (1999)). In addition to the NF-Y subunits themselves, a number of other proteins have been implicated in formation of the complex (Mantovani (1999)).

Using approaches such as directed mutagenesis, specific regions of the NF-Y proteins have been altered and inferences made about their specific role. In particular, it is has been found that the HFMs of NF-YB and NF-YC are critical for dimer formation, NF-YA association and CCAAT-binding (Sinha et al. (1996); Kim et al. (1996); Xing et al. (1993); Maity and de Crombrugghe (1998)). Specific amino acids in $\alpha 2$, L2 and $\alpha 3$ are required for dimerization between the NF-YB and NF-YC subunits. For NF-YA association, two conserved amino acids in $\alpha 2$ from NF-YB and several residues in NF-YC, within $\alpha 1$, $\alpha 2$ and at the C-terminus of $\alpha 3$ are required. For DNA binding, which is the most difficult feature to address since the two other functions need to be intact, the $\alpha 1$ and $\alpha 2$ of NF-YB and the $\alpha 1$ of NF-YC are necessary. These latter results do not rule out that other parts of the HFMs are necessary to make the trimer bind to DNA; most notably the positively charged residues in L2 may have such a role, as in histones (Luger et al. (1997); Mantovani (1999)).

Most of the sequence specific interactions within the NF-Y trimer appear to be conferred by NF-YA. In contrast to the B and C subunits, the conserved domain in the A subunit does not bear any resemblance to histones or any other well-characterized DNA-binding motif. However, like the B and C subunits, the A subunit has also been subject to saturation mutagenesis. The NF-YA conserved domain appears to comprise two distinct halves, each of ~20 amino acids; the N-terminal part of the conserved domain is required for association with the BC dimer, whereas the C-terminal portion of the NF-YA conserved domain is needed for DNA binding (Mantovani (1999)). Further structural insights into the function of NF-Y have now been obtained following solution of the crystal structure of the BC dimer (Romier et al. (2003)). This confirmed the role of the HFM motifs and the role of the conserved regions of NF-YA; a model for DNA interactions suggests that the NF-YA subunit binds the CCAAT box while the B and C subunits bend the DNA (Romier et al. (2003)).

There is very little sequence similarity between HAP3 proteins in the A and C domains; it is therefore reasonable to assume that the A and C domains could provide a degree of functional specificity to each member of the HAP3 subfamily. The B domain is the conserved region that specifies DNA binding and subunit association.

In Figures 3A-3F, HAP3 proteins from *Arabidopsis,* soybean, rice and corn are aligned with G481. The B domain of the non-LEC1-like clade (identified in the box spanning Figures 3B-3C) may be distinguished by the comprised amino acid residues:

Asn-(Xaa)$_{4-11}$-Lys-(Xaa)$_{33-34}$-Asn-Gly-(Xaa)$_2$-Leu;
where Xaa can be any amino acid. These residues in their present positions are uniquely found in the non-LEC1-like clade, and may be used to identify members of this clade.

The G482 subclade is distinguished by a B-domain comprising:

Ser/Glu-(Xaa)$_9$-Asn-(Xaa)$_{4-11}$-Lys-(Xaa)$_{33-34}$-Asn-Gly-(Xaa)$_2$-Leu.

Plant CCAAT binding factors are regulated at the level of transcription. In contrast to the NF-Y genes from mammals, members of the CCAAT family from *Arabidopsis* appear to be heavily regulated at the level of RNA abundance. Surveys of expression patterns *of Arabidopsis* CCAAT family members from a number of different studies have revealed complex patterns of expression, with some family members being specific to particular tissue types or conditions (Edwards et al. (1998); Gusmaroli et al. (2001); Gusmaroli et al. (2002)). During previous genomics studies, we also found that the expression patterns of many of the HAP-like genes in *Arabidopsis* were suggestive of developmental and/or conditional regulation. In particular *LEC1* (G620, SEQ ID NO: 65) and *L1L* (G1821) were very strongly expressed in siliques and embryos relative to other tissues. We used RT-PCR to analyze the endogenous expression of 31 of the 36 CCAAT-box genes. Our findings suggested that while many of the CCAAT-box gene transcripts are found ubiquitously throughout the plant, in more than half of the cases, the genes are predominantly expressed in flower, embryo and/or silique tissues.

Roles of CCAAT binding factors in plants. The specific roles of CCAAT-box elements and their binding factors in plants are still poorly understood. CCAAT-box elements have been shown to function in the regulation of gene expression (Rieping and Schoffl (1992); Kehoe et al. (1994); Ito et al. (1995)). Several reports have described the importance of the CCAAT-binding element for regulated gene expression; including the modulation of genes that are responsive to light (Kusnetsov et al. (1999); Carre and Kay (1995); Bezhani et al. (2001)) as well as stress (Rieping and Schoffl (1992)). Specifically, a CCAAT-box motif was shown to be important for the light regulated expression of the *CAB2* promoter in *Arabidopsis.* However, the proteins that bind to the site were not identified (Carre and Kay (1995)).

Role of LEC1-like proteins. The functions of only two of the *Arabidopsis* CCAAT-box genes have been genetically determined in the public domain. These genes, *LEAFY COTYLEDON 1 (LECI,* G620, SEQ ID NO: 65) and *LEAFY COTYLEDON 1-LIKE* (*L1L,* G1821) have critical roles in embryo development and seed maturation (Lotan et al. (1998); Kwong et al. (2003)) and encode proteins of the HAP3 (NF-YB) class. *LEC1* has multiple roles in and is critical for normal development during both the early and late phases of embryogenesis (Meinke (1992); Meinke et al. (1994); West et al. (1994); Parcy et al. (1997) ; Vicient et al. (2000)). Mutant *lec1* embryos have cotyledons that exhibit leaf-like characteristics such as trichomes. The gene is required to maintain suspensor cell fate and to specify cotyledon identity in the early morphogenesis phase. Through overexpression studies, LEC1 activity has been shown sufficient to initiate embryo development in vegetative cells (Lotan et al. (1998)). Additionally, *lec1* mutant embryos are desiccation intolerant and cannot survive seed dry-down (but can be artificially rescued in the laboratory). This phenotype reflects a role for LEC1 at later stages of seed maturation; the gene initiates and/or maintains the maturation phase, prevents precocious germination, and is required for acquisition of desiccation tolerance during seed maturation. *L1L* appears to be a paralog of, and partially redundant with *LEC1.* Like *LEC1, L1L* is expressed during embryogenesis, and genetic studies have demonstrated that *L1L* can complement *lec1* mutants (Kwong et al. (2003)).

Putative *LEC1* orthologs exist in a wide range of species and based on expression patterns, likely have a comparable function to the *Arabidopsis* gene. For example, the ortholog of *LEC1* has been identified recently in maize. The expression pattern of *ZmLEC1* in maize during somatic embryo development is similar to that of LEC1 in *Arabidopsis* during zygotic embryo development (Zhang et al. (2002)). A comparison of LEC1-like proteins with other proteins of the HAP3 sub-group indicates that the LEC1-like proteins form a distinct phylogenetic clade, and have a number of distinguishing residues, which set them apart from the non-LEC1-like HAP3 proteins (Kwong et al. (2003)). Thus it is likely that the LEC1 like proteins have very distinct functions compared to proteins of the non-LEC1-like HAP3 group.

HAP3 (NF-YB) proteins have a modular structure and are comprised of three distinct domains: an amino-terminal A domain, a central B domain and a carboxy-terminal C domain. There is very little sequence similarity between HAP3 proteins within the A and C domains suggesting that those regions could provide a degree of functional specificity to each member of the HAP3 subfamily. The B domain is a highly conserved region that specifies DNA binding and subunit association. Lee et al. (2003) performed an elegant series of domain swap experiments between the LEC1 and a non-LEC1 like HAP3 protein (At4g 14540, G485) to demonstrate that the B domain of LEC1 is necessary and sufficient, within the context of the rest of the protein, to confer its activity in embryogenesis. Furthermore, these authors identified a specific defining residue within the B domain (Asp-55) that is required for LEC1 activity and which is sufficient to confer LEC1 function to a non-LEC1 like B domain.

Discoveries made in earlier genomics programs. G481 is a member of the HAP3 (NF-YB) group of CCAAT-box binding proteins, and falls within the non-LEC1-like clade of proteins. G481 is equivalent to AtHAP3a, which was identified by Edwards et al. (1998), as an EST with extensive sequence homology to the yeast HAP3. Northern blot data from five different tissue samples indicated that G481 is primarily expressed in flower and/or silique, and root tissue. RT-PCR studies partially confirmed the published expression data; we detected relatively low levels of G481 expression in all of the tissues tested, with somewhat higher levels of expression being detected in flowers, siliques, and embryos. However, the differential expression of G481 in these relative to other tissues was much less dramatic than that which was seen for G620 (*LEC1,* 1, SEQ ID NO: 65) and G1821 (*L1L,*1), which function specifically in embryo development.

It was initially discovered that 35S::G481 lines display a hyperosmotic stress tolerance and/or sugar sensing phenotype on media containing high levels of sucrose, after which drought tolerance in a soil-based assay was demonstrated. In addition to G481, there are a further seven other non-LEC1-like proteins which lie on the same branch of the phylogenetic tree (Figure 2), and represent the phylogenetically related sequences G1364, G2345, G482, G485, G1781, G1248 and G486 (polypeptide SEQ ID NOs: 14, 22, 28, 18, 56, 68, and 64, respectively). Two other HAP3 proteins, G484 (polypeptide SEQ ID NO: 62) and G2631 (polypeptide SEQ ID NO: 70) appear to be rather more distantly related. G1364 and G2345 are the *Arabidopsis* proteins most closely related to G481; however, neither of these genes has been found to confer hyperosmotic stress tolerance.

G482 (polypeptide SEQ ID NO: 28) is slightly further diverged from G481 than G2345 and G1364 (Figure 2), but has an apparently similar function given that 35S::G482 lines analyzed during our initial genomics screens displayed an hyperosmotic stress response phenotype similar to 35S::G481. Another HAP3 gene, G485 (SEQ ID NO: 17 and 18), is most closely related to G482. G485 was not implicated in regulation of stress responses in our initial screens, but KO.G485 and 35S::G485 lines exhibited opposite flowering time phenotypes, with the mutant flowering late, and the overexpression lines flowering early. Thus, G485 functions as an activator of the floral transition. Interestingly, two of the other non-LEC1-like genes, G1781 (SEQ ID NO: 55) and G1248 (SEQ ID NO: 67), were also found to accelerate flowering when overexpressed, during our genomics program. However, overexpression lines for neither of those genes were found to show alterations in stress tolerance. G486 was also noted to produce effects on flowering time, but these were inconclusive and rather variable between different lines.

In addition to HAP3 (NF-YB) genes, a number of HAP5 (NF-YC) genes were found to influence abiotic stress responses during our initial genomics program. G489 (SEQ ID NO: 45), G1836 (SEQ ID NO: 47), and G1820 (SEQ ID NO: 43) are all HAP5-like proteins that generated hyperosmotic stress tolerance phenotypes when overexpressed. Thus, we surmised that these proteins might potentially be members of the same heteromeric complex as G481 or one or more of the other HAP3 proteins.

Potential mode of action of G481. The enhanced tolerance of 35S::G481 lines to sucrose seen in our genomics screens suggests that G481 could influence sugar sensing and hormone signaling. Several sugar sensing mutants have turned out to be allelic to ABA and ethylene mutants. On the other hand, the sucrose treatment (9.5% w/v) could have represented an hyperosmotic stress; thus, one might also interpret the results as indicating that G481 confers tolerance to hyperosmotic stress. *LEC1* (G620, polypeptide SEQ ID NO: 66), which is required for desiccation tolerance during seed maturation, is also ABA and drought inducible. This information, combined with the fact that CCAAT genes are disproportionately responsive to hyperosmotic stress suggests that the family could control pathways involved in both ABA response and desiccation tolerance. In particular, given their phylogenetic divergence, it is possible that LEC1-like proteins have evolved to confer desiccation tolerance specifically within the embryo, whereas other non-LEC1-like HAP3 proteins confer tolerance in non-embryonic tissues.

A role in sugar sensing also supports the possibility that, as in yeast, CCAAT-box factors from plants play a general role in the regulation of energy metabolism. Indeed, the fact that plants exhibit two modes of energy metabolism (in the form of photosynthesis and respiration) could account for the expansion of the family in the plant kingdom. Specifically, a mechanism that is currently being evaluated is that G481-related proteins regulate starch/sugar metabolism, and as such, influence both the osmotic balance of cells as well as the supply of photosynthate to sink areas. Such hypotheses can account for a number of the off-types, such as reduced yield (under well-watered conditions) and delayed senescence, seen in corn and soy field tests of G481 (and related genes) overexpression lines. The prospective involvement of CCAAT box factors in chloroplast development and retrograde signaling also suggests a further means by which G481-related genes could confer stress tolerance. The genes might act to maintain chloroplast function under unfavorable conditions. In fact, any effects on expression of chloroplast components could well be indirectly related to the putative effects on carbohydrate metabolism.

Table 1 shows a number of polypeptides of the disclosure and includes the amino acid residue coordinates for the conserved domains, the conserved domain sequences of the respective polypeptides, (sixth column); the identity in percentage terms to the conserved domain of the lead *Arabidopsis* sequence (the first transcription factor listed in each table), and whether the given sequence in each row was shown to confer increased biomass and yield or stress tolerance in plants (+) or has thus far not been shown to confer stress tolerance (-) for each given promoter::gene combination in our experiments. Percentage identities to the sequences listed in Tables 8-17 were determined using BLASTP analysis with defaults of wordlength (W) of 3, an expectation (E) of 10, and the BLOSUM62 scoring matrix Henikoff & Henikoff (1989) *Proc. Natl. Acad. Sci. USA* 89:10915).

Table 1. Conserved domains of G481 and closely related sequences

| Polypeptide SEQ ID NO: | Species/ GID No., Accession No., or Identifier | Domain Amino Acid Coordinates | B Domain | % ID to CCAAT-box binding conserved domain of G481 | Abiotic Stress Tolerance |
|---|---|---|---|---|---|
| 2 | At/G481 | 20-110 | REQDRYLPIANISRIMKKALPPNGKI GKDAKDTVQECVSEFISFITSEASD KCQKEKRKTVNGDDLLWAMATLG FEDYLEPLKIYLARYRE | 100% | + |
| 4 | At/G3470 | 27-117 | REQDRYLPIANISRIMKKALPPNGKI AKDAKDTMQECVSEFISFITSEASE KCQKEKRKTINGDDLLWAMATLG FEDYIEPLKVYLARYRE | 93% | + |
| 6 | At/G3471 | 26-116 | REQDRYLPIANISRIMKKALPPNGKI AKDAKDTMQECVSEFISFITSEASE KCQKEKRKTINGDDLLWAMATLG FEDYIEPLKVYLARYRE | 93% | + |
| 8 | Zm/G3876 | 30-120 | REQDRFLPIANISRIMKKAIPANGKI AKDAKETVQECVSEFISFITSEASDK CQREKRKTINGDDLLWAMATLGFE DYIEPLKVYLOKYRE | 87% | + |
| 10 | At/G3394 | 38-127 | RQDRFLPIANISRIMKKAIPANGKIA KDAKETVQECVSEFISFITSEASDKC QREKRKTINGDDLLWAMATLGFED YIEPLKVYLOKYRE | 87% | - |
| 12 | Zm/G3434 | 18-108 | REQDRFLPIANISRIMKKAVPANGKI AKDAKETLQECVSEFISFVTSEASD KCQKEKRKTINGDDLLWAMATLG FEEYVEPLKIYLOKYKE | 85% | + |
| 14 | At/G1364 | 29-119 | REQDRFLPIANISRIMKRGLPANGKI AKDAKEIVQECVSEFISFVTSEASD KCQREKRKTINGDDLLWAMATLGF EDYMEPLKVYLMRYRE | 85% | + |

| Polypeptide SEQ ID NO: | Species/ GID No., Accession No., or Identifier | Domain Amino Acid Coordinates | B Domain | % ID to CCAAT-box binding conserved domain of G481 | Abiotic Stress Tolerance |
|---|---|---|---|---|---|
| 16 | Gm/G3475 | 23-113 | REQDRFLPIANVSRIMKKALPANAK ISKDAKETVQECVSEFISFITGEASD KCQREKRKTINGDDLLWAMTTLGF EDYVEPLKGYLORFRE | 84% | + |
| 18 | At/G485 | 20-110 | REQDRFLPIANVSRIMKKALPANAK ISKDAKETVQECVSEFISFITGEASD KCQREKRKTINGDDLLWAMTTLGF EDYVEPLKVYLOKYRE | 84% | + |
| 20 | Gm/G3476 | 26-116 | REQDRFLPIANVSRIMKKALPANAK ISKDAKETVQECVSEFISFITGEASD KCQREKRKTINGDDLLWAMTTLGF EEYVEPLKIYLQRFRE | 84% | + |
| 22 | At/G2345 | 28-118 | REQDRFLPIANISRIMKRGLPLNGKI AKDAKETMQECVSEFISFVTSEASD KCQREKRKTINGDDLLWAMATLGF EDYIDPLKVYLMRYRE | 84% | + |
| 24 | Gm/G3474 | 25-115 | REQDRFLPIANVSRIMKKALPANAK ISKEAKETVQECVSEFISFITGEASD KCQKEKRKTINGDDLLWAMTTLGF EDYVDPLKIYLHKYRE | 84% | - |
| 26 | Gm/G3478 | 23-113 | REQDRFLPIANVSRIMKKALPANAK ISKDAKETVQECVSEFISFITGEASD KCQREKRKTINGDDLLWAMTTLGF EDYVEPLKGYLORFRE | 84% | - |
| 28 | At/G482 | 26-116 | REQDRFLPIANVSRIMKKALPANAK ISKDAKETMQECVSEFISFVTGEAS DKCQKEKRKTINGDDLLWAMTTL GFEDYVEPLKVYLORFRE | 83% | + |

EP 1 928 226 B1

| Polypeptide SEQ ID NO: | Species/ GID No., Accession No., or Identifier | Domain Amino Acid Coordinates | B Domain | % ID to CCAAT-box binding conserved domain of G481 | Abiotic Stress Tolerance |
|---|---|---|---|---|---|
| 30 | Zm/G3435 | 22-112 | REQDRFLPIANVSRIMKKALPANAK ISKDAKETVOECVSEFISFITGEASD | 83% | + |
| | | | KCQREKRKTINGDDLLWAMTTLGF EDYVEPLKHYLHKFRE | | |
| 32 | Gm/G3472 | 25-115 | REQDRFLPIANVSRIMKKALPANAK ISKEAKETVQECVSEFISFITGEASD KCQKEKRKTINGDDLLWAMTTLGF EEYVEPLKVYLHKYRE | 83% | + |
| 34 | Zm/G3436 | 20-110 | REQDRFLPIANVSRIMKKALPANAK ISKDAKETVQECVSEFISFITGEASD KCQREKRKTINGDDLLWAMTTLGF EDYVEPLKLYLHKFRE | 83% | + |
| 36 | Os/G3397 | 23-113 | REQDRFLPIANVSRIMKKALPANAK ISKDAKETVQECVSEFISFITGEASD KCQREKRKTINGDDLLWAMTTLGF EDYVDPLKHYLHKFRE | 82% | + |
| 38 | Os/G3395 | 19-109 | REQDRFLPIANISRIMKKAVPANGKI AKDAKETLQECVSEFISFVTSEASD KCQKEKRKTINGEDLLFAMGTLGF EEYVDPLKIYLHKYRE | 82% | + |
| 40 | Os/G3398 | 20-110 | REQDRFLPIANVSRIMKRALPANAK ISKDAKETVQECVSEFISFITGEASD KCQREKRKTINGDDLLWAMTTLGF EDYIDPLKLYLHKFRE | 81% | + |
| 42 | Os/G3396 | 20-111 | KEQDRFLPIANIGRIMRRAVPENGKI AKDSKESVQECVSEFISFITSEASDK CLKEKRKTINGDDLIWSMGTLGFE DYVEPLKLYLRLYRE | 78% | + |

EP 1 928 226 B1

17

| Polypeptide SEQ ID NO: | Species/ GID No., Accession No., or Identifier | Domain Amino Acid Coordinates | B Domain | % ID to CCAAT-box binding conserved domain of G481 | Abiotic Stress Tolerance |
|---|---|---|---|---|---|
| 58 | Os/G3429 | 37-125 | TNAELPMANLVRLIKKVLPGKAKI GGAAKGLTHDCAVEFVGFVGDEAS EKAKAEHRRTVAPEDYLGSFGDLG FDRYVDPMDAYIHGYRE | 43% | + |

Abbreviations for Table 1: *At - Arabidopsis thaliana;* Gm - *Glycine max;* Os - *Oryza* sativa; Zm-Zea *mays;* + more tolerant than control plant in abiotic or disease assay n/d - assay not yet done

Orthologs and Paralogs

Homologous sequences as described above can comprise orthologous or paralogous sequences. Several different methods are known by those of skill in the art for identifying and defining these functionally homologous sequences. Three general methods for defining orthologs and paralogs are described; an ortholog or paralog, including equivalogs, may be identified by one or more of the methods described below.

Within a single plant species, gene duplication may cause two copies of a particular gene, giving rise to two or more genes with similar sequence and often similar function known as paralogs. A paralog is therefore a similar gene formed by duplication within the same species. Paralogs typically cluster together or in the same clade (a group of similar genes) when a gene family phylogeny is analyzed using programs such as CLUSTAL (Thompson et al. (1994); Higgins et al. (1996)). Groups of similar genes can also be identified with pair-wise BLAST analysis (Feng and Doolittle (1987)). For example, a clade of very similar MADS domain transcription factors from *Arabidopsis* all share a common function in flowering time (Ratcliffe et al. (2001)), and a group of very similar AP2 domain transcription factors from *Arabidopsis* are involved in tolerance of plants to freezing (Gilmour et al. (1998)). Analysis of groups of similar genes with similar function that fall within one clade can yield sub-sequences that are particular to the clade. These sub-sequences, known as consensus sequences, can not only be used to define the sequences within each clade, but define the functions of these genes; genes within a clade may contain paralogous sequences, or orthologous sequences that share the same function (see also, for example, Mount (2001))

Speciation, the production of new species from a parental species, can also give rise to two or more genes with similar sequence and similar function. These genes, termed orthologs, often have an identical function within their host plants and are often interchangeable between species without losing function. Because plants have common ancestors, many genes in any plant species will have a corresponding orthologous gene in another plant species. Once a phylogenic tree for a gene family of one species has been constructed using a program such as CLUSTAL (Thompson et al. (1994); Higgins et al. (1996)) potential orthologous sequences can be placed into the phylogenetic tree and their relationship to genes from the species of interest can be determined. Orthologous sequences can also be identified by a reciprocal BLAST strategy. Once an orthologous sequence has been identified, the function of the ortholog can be deduced from the identified function of the reference sequence.

Transcription factor gene sequences are conserved across diverse eukaryotic species lines (Goodrich et al. (1993); Lin et al. (1991); Sadowski et al. (1988)). Plants are no exception to this observation; diverse plant species possess transcription factors that have similar sequences and functions.

Orthologous genes from different organisms have highly conserved functions, and very often essentially identical functions (Lee et al. (2002); Remm et al. (2001)). Paralogous genes, which have diverged through gene duplication, may retain similar functions of the encoded proteins. In such cases, paralogs can be used interchangeably with respect to certain embodiments of the instant invention (for example, transgenic expression of a coding sequence). An example of such highly related paralogs is the CBF family, with three well-defined members in *Arabidopsis* and at least one ortholog in *Brassica napus,* all of which control pathways involved in both freezing and drought stress (Gilmour et al. (1998); Jaglo et al. (2001)).

Distinct *Arabidopsis* transcription factors, including G28 (found in US Patent 6,664,446), G482 (found in US Patent Application 20040045049), G867 (found in US Patent Application 20040098764), and G1073 (found in US Patent 6,717,034), have been shown to confer stress tolerance or increased biomass when the sequences are overexpressed. The polypeptides sequences belong to distinct clades of transcription factor polypeptides that include members from diverse species. In each case, a significant number of clade member sequences derived from both dicots and monocots have been shown to confer increased biomass or tolerance to stress when the sequences were overexpressed (unpublished data). These references may serve to represent the many studies that demonstrate that conserved transcription factor genes from diverse species are likely to function similarly (i.e., regulate similar target sequences and control the same traits), and that transcription factors may be transformed into diverse species to confer or improve traits.

Orthologs and paralogs of presently disclosed transcription factors may be cloned using compositions provided by the present disclosure according to methods well known in the art. cDNAs can be cloned using mRNA from a plant cell or tissue that expresses one of the present transcription factors. Appropriate mRNA sources may be identified by interrogating Northern blots with probes designed from the present transcription factor sequences, after which a library is prepared from the mRNA obtained from a positive cell or tissue. Transcription factor-encoding cDNA is then isolated using, for example, PCR, using primers designed from a presently disclosed transcription factor gene sequence, or by probing with a partial or complete cDNA or with one or more sets of degenerate probes based on the disclosed sequences. The cDNA library may be used to transform plant cells. Expression of the cDNAs of interest is detected using, for example, microarrays, Northern blots, quantitative PCR, or any other technique for monitoring changes in expression. Genomic clones may be isolated using similar techniques to those.

Examples of orthologs of the *Arabidopsis* polypeptide sequences and their functionally similar orthologs are listed in the Sequence Listing. In addition to the sequences in the Sequence Listing, the disclosure relates to isolated nucleotide sequences that are phylogenetically and structurally similar to sequences listed in the Sequence Listing) and can function in a plant by increasing biomass, disease resistance and/or and abiotic stress tolerance when ectopically expressed in a plant. These polypeptide sequences represent transcription factors that show significant sequence similarity the polypeptides of the Sequence Listing particularly in their respective conserved domains, as identified in Table 1.

Since a significant number of these sequences are phylogenetically and sequentially related to each other and have been shown to increase a plant's biomass, disease resistance and/or abiotic stress tolerance, one skilled in the art would predict that other similar, phylogenetically related sequences falling within the present clades of transcription factors would also perform similar functions when ectopically expressed.

Identifying Polynucleotides or Nucleic Acids by Hybridization

Polynucleotides homologous to the sequences illustrated herein can be identified, e.g., by hybridization to each other under stringent or under highly stringent conditions. Single stranded polynucleotides hybridize when they associate based on a variety of well characterized physical-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number thereof), as described in more detail in the references cited below (e.g., Sambrook et al. (1989); Berger and Kimmel (1987); and Anderson and Young (1985)).

Encompassed by the disclosure are polynucleotide sequences that are capable of hybridizing to the claimed polynucleotide sequences, including any of the transcription factor polynucleotides within the Sequence Listing, and fragments thereof under various conditions of stringency (see, for example, Wahl and Berger (1987); and Kimmel (1987)). In addition, full length cDNA, orthologs, and paralogs of the present nucleotide sequences may be identified and isolated using well-known methods. The cDNA libraries, orthologs, and paralogs of the present nucleotide sequences may be screened using hybridization methods to determine their utility as hybridization target or amplification probes.

With regard to hybridization, conditions that are highly stringent, and means for achieving them, are well known in the art. See, for example, Sambrook et al. (1989); Berger (1987), pages 467-469; and Anderson and Young (1985).

Stability of DNA duplexes is affected by such factors as base composition, length, and degree of base pair mismatch. Hybridization conditions may be adjusted to allow DNAs of different sequence relatedness to hybridize. The melting temperature ($T_m$) is defined as the temperature when 50% of the duplex molecules have dissociated into their constituent single strands. The melting temperature of a perfectly matched duplex, where the hybridization buffer contains formamide as a denaturing agent, may be estimated by the following equations:

$$\text{(I) DNA-DNA:}$$

$$T_m(^\circ C)=81.5+16.6(\log [Na+])+0.41(\% \ G+C)-0.62(\% \ formamide)-500/L$$

$$\text{(II) DNA-RNA:}$$

$$T_m(^\circ C)=79.8+18.5(\log [Na+])+0.58(\% \ G+C)+0.12(\%G+C)^2-0.5(\% \ formamide)-820/L$$

$$\text{(III) RNA-RNA:}$$

$$T_m(^\circ C)=79.8+18.5(\log [Na+])+0.58(\% \ G+C)+0.12(\%G+C)^2-0.35(\% \ formamide)-820/L$$

where L is the length of the duplex formed, [Na+] is the molar concentration of the sodium ion in the hybridization or washing solution, and % G+C is the percentage of (guanine+cytosine) bases in the hybrid. For imperfectly matched hybrids, approximately 1° C is required to reduce the melting temperature for each 1% mismatch.

Hybridization experiments are generally conducted in a buffer of pH between 6.8 to 7.4, although the rate of hybridization is nearly independent of pH at ionic strengths likely to be used in the hybridization buffer (Anderson and Young (1985)). In addition, one or more of the following may be used to reduce non-specific hybridization: sonicated salmon sperm DNA or another non-complementary DNA, bovine serum albumin, sodium pyrophosphate, sodium dodecylsulfate (SDS), polyvinyl-pyrrolidone, ficoll and Denhardt's solution. Dextran sulfate and polyethylene glycol 6000 act to exclude DNA from solution, thus raising the effective probe DNA concentration and the hybridization signal within a given unit of time. In some instances, conditions of even greater stringency may be desirable or required to reduce non-specific and/or background hybridization. These conditions may be created with the use of higher temperature, lower ionic strength and higher concentration of a denaturing agent such as formamide.

Stringency conditions can be adjusted to screen for moderately similar fragments such as homologous sequences from distantly related organisms, or to highly similar fragments such as genes that duplicate functional enzymes from closely related organisms. The stringency can be adjusted either during the hybridization step or in the post-hybridization washes. Salt concentration, formamide concentration, hybridization temperature and probe lengths are variables that can be used to alter stringency (as described by the formula above). As a general guidelines high stringency is typically performed at $T_m$-5° C to $T_m$-20° C, moderate stringency at $T_m$-20° C to $T_m$-35° C and low stringency at $T_m$-35° C to $T_m$-50° C for duplex >150 base pairs. Hybridization may be performed at low to moderate stringency (25-50° C below $T_m$), followed by post-hybridization washes at increasing stringencies. Maximum rates of hybridization in solution are determined empirically to occur at $T_m$-25° C for DNA-DNA duplex and $T_m$-15° C for RNA-DNA duplex. Optionally, the degree of dissociation may be assessed after each wash step to determine the need for subsequent, higher stringency wash steps.

High stringency conditions may be used to select for nucleic acid sequences with high degrees of identity to the disclosed sequences. An example of stringent hybridization conditions obtained in a filter-based method such as a Southern or Northern blot for hybridization of complementary nucleic acids that have more than 100 complementary residues is about 5°C to 20°C lower than the thermal melting point ($T_m$) for the specific sequence at a defined ionic strength and pH. Conditions used for hybridization may include about 0.02 M to about 0.15 M sodium chloride, about 0.5% to about 5% casein, about 0.02% SDS or about 0.1% N-laurylsarcosine, about 0.001 M to about 0.03 M sodium citrate, at hybridization temperatures between about 50° C and about 70° C. More preferably, high stringency conditions are about 0.02 M sodium chloride, about 0.5% casein, about 0.02% SDS, about 0.001 M sodium citrate, at a temperature of about 50° C. Nucleic acid molecules that hybridize under stringent conditions will typically hybridize to a probe based on either the entire DNA molecule or selected portions, e.g., to a unique subsequence, of the DNA.

Stringent salt concentration will ordinarily be less than about 750 mM NaCl and 75 mM trisodium citrate. Increasingly stringent conditions may be obtained with less than about 500 mM NaCl and 50 mM trisodium citrate, to even greater stringency with less than about 250 mM NaCl and 25 mM trisodium citrate. Low stringency hybridization can be obtained in the absence of organic solvent, e.g., formamide, whereas high stringency hybridization may be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C with formamide present. Varying additional parameters, such as hybridization time, the concentration of detergent, e.g., sodium dodecyl sulfate (SDS) and ionic strength, are well known to those skilled in the art. Various levels of stringency are accomplished by combining these various conditions as needed.

The washing steps that follow hybridization may also vary in stringency; the post-hybridization wash steps primarily determine hybridization specificity, with the most critical factors being temperature and the ionic strength of the final wash solution. Wash stringency can be increased by decreasing salt concentration or by increasing temperature. Stringent salt concentration for the wash steps will preferably be less than about 30 mM NaCl and 3 mM trisodium citrate, and most preferably less than about 15 mM NaCl and 1.5 mM trisodium citrate.

Thus, hybridization and wash conditions that may be used to bind and remove polynucleotides with less than the desired homology to the nucleic acid sequences or their complements that encode the present transcription factors include, for example:

6X SSC at 65° C;
50% formamide, 4X SSC at 42° C; or
0.5X SSC, 0.1% SDS at 65° C;
with, for example, two wash steps of 10 - 30 minutes each. Useful variations on these conditions will be readily apparent to those skilled in the art.

A person of skill in the art would not expect substantial variation among polynucleotide species because the highly stringent conditions set forth in the above formulae yield structurally similar polynucleotides.

If desired, one may employ wash steps of even greater stringency, including about 0.2x SSC, 0.1% SDS at 65° C and washing twice, each wash step being about 30 minutes, or about 0.1 x SSC, 0.1% SDS at 65° C and washing twice for 30 minutes. The temperature for the wash solutions will ordinarily be at least about 25° C, and for greater stringency at least about 42° C. Hybridization stringency may be increased further by using the same conditions as in the hybridization steps, with the wash temperature raised about 3° C to about 5° C, and stringency may be increased even further by using the same conditions except the wash temperature is raised about 6° C to about 9° C. For identification of less closely related homologs, wash steps may be performed at a lower temperature, e.g., 50° C.

An example of a low stringency wash step employs a solution and conditions of at least 25° C in 30 mM NaCl, 3 mM trisodium citrate, and 0.1% SDS over 30 minutes. Greater stringency may be obtained at 42° C in 15 mM NaCl, with 1.5 mM trisodium citrate, and 0.1% SDS over 30 minutes. Even higher stringency wash conditions are obtained at 65° C -68° C in a solution of 15 mM NaCl, 1.5 mM trisodium citrate, and 0.1 % SDS. Wash procedures will generally employ at least two final wash steps. Additional variations on these conditions will be readily apparent to those skilled in the art (see, for example, US Patent Application No. 20010010913).

Stringency conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. It may be desirable to select conditions for a particular assay such that a higher signal to noise ratio, that is, about 15x or more, is obtained. Accordingly, a subject nucleic acid will hybridize to a unique coding oligonucleotide with at least a 2x or greater signal to noise ratio as compared to hybridization of the coding oligonucleotide to a nucleic acid encoding known polypeptide. The particular signal will depend on the label used in the relevant assay, e.g., a fluorescent label, a colorimetric label, a radioactive label, or the like. Labeled hybridization or PCR probes for detecting related polynucleotide sequences may be produced by oligolabeling, nick translation, end-labeling, or PCR amplification using a labeled nucleotide.

## EXAMPLES

It is to be understood that this invention is not limited to the particular devices, machines, materials and methods described. Although particular embodiments are described, equivalent embodiments may be used to practice the invention.

The invention, now being generally described, will be more readily understood by reference to the following examples, which are included merely for purposes of illustration of certain aspects and embodiments of the present invention and are not intended to limit the invention. It will be recognized by one of skill in the art that a transcription factor that is associated with a particular first trait may also be associated with at least one other, unrelated and inherent second trait which was not predicted by the first trait.

### Example I. Project Types

A variety of constructs are being used to modulate the activity of lead transcription factors, and to test the activity of orthologs and paralogs in transgenic plant material. This platform provides the material for all subsequent analysis.

Transgenic lines from each particular transformation "project" are examined for morphological and physiological phenotypes. An individual project is defined as the analysis of lines for a particular construct or knockout (for example this might be 35S lines for a lead gene, 35S lines for a paralog or ortholog, lines for an RNAi construct, lines for a GAL4 fusion construct, lines in which expression is driven from a particular tissue specific promoter, etc..) In the current lead advancement program, four main areas of analysis were pursued, spanning a variety of different project types (e.g., promoter-gene combinations).

### (1) Overexpression/tissue-specific/conditional expression

Expression of a given TF from a particular promoter is achieved either by a direct-promoter fusion construct in which that TF is cloned directly behind the promoter of interest or by a two component system. Details of transformation vectors used in these studies are shown in the Vector and Cloning Information (Example II). A list of all constructs (PIDs) included in this report, indicating the promoter fragment that is being used to drive the transgene, along with the cloning vector backbone, is provided in the following Table. Compilations of the sequences of promoter fragments and the expressed transgene sequences within the PIDs are provided in the Sequence Listing.

Table 2. Sequences of promoter fragments and the expressed transgene sequences

| GID | PID | SEQ ID NO: of PID | Promoter | Project type | Promoter_ID | Vector |
|---|---|---|---|---|---|---|
| G3876 | P25657 | 77 | 35S | Direct promoter-fusion | N2 | pMEN65 |

The vector backbone for most of the direct promoter-fusion overexpression constructs is pMEN65.

**Example II. Vector and Cloning Information**

Vector and Cloning Information: Expression Vectors.

A list of constructs (PIDs) included in this application, indicating the promoter fragment that was used to drive the transgene, along with the cloning vector backbone, is provided in Table 23. Compilations of the sequences of promoter fragments (SEQ ID NO: 79) and the expressed transgene sequences within the PIDs (SEQ ID NO: 77) are provided in the Sequence Listing. Plant Expression vectors that have been generated are summarized in the following table and more detailed description are provided below.

Table 3. Summary of Plant Expression Vectors

| Construct Name | Class | Construct Description | Selection | Description of the included sequence |
|---|---|---|---|---|
| pMEN65 | 35S expression vector | 35S::MCS::E9 | prNOS::NPTII::Nos | T-DNA segment (SEQ ID NO: 80) |

Table 3 Legend: E9: E9 3-prime UTR; GR: glucocorticoid receptor, MCS: multiple cloning site; Nos: Nopaline synthase 3-prime UTR; prNOS: Nopaline synthase promoter; NPTII: Kanamycin resistance.
Other Construct Element Sequences, which may be found in the table below and in the Sequence Listing, include: the 35S promoter (35S), the NOS promoter (prNOS), the minimal 35S (m35S), the omega Enhancer (oEnh), the Nos terminator (Nos), the E9 terminator (E9), and the NPmito::Sulfonamide element.

Table 4. Other Construct Element Sequences

| Element | Sequence |
|---|---|
| 35S promoter (35S) | gcggattccattgcccagctatctgtcactttattgtgaagatagtgaaaaagaaggtggctcctacaaatgccatcattgcgataaaggaaaggccatcgt tgaagatgcctctgccgacagtggtcccaaagatggaccccaacccacgaggagcatcgtggaaaaagaagacgttccaaccacgtcttcaaagcaa gtggattgatgtgatggtccgattgagactttttcaacaaagggtaatatccggaaacctcctcggattccattgcccagctatctgtcactttattgtgaagat agtggaaaaggaaggtggctcctacaaatgccatcattgcgataaaggaaaggccatcgttgaagatgcctctgccgacagtggtcccaaagatggac ccccacccacgaggagcatcgtggaaaaagaagacgttccaaccacgtcttcaaagcaagtggattgatgtgatatctccactgacgtaagggatgacg cacaatcccactatccttcgcaagacccttcctctatataaggaagttcatttcatttggagaggacacgctga |
| NOS promoter (prNOS) | tcgagatcatgagcggagaattaagggagtcacgttatgacccccgccgatgacgcgggacaagccgtttttacgtttggaactgacagaaccgcaacgt tgaaggagccactcagccgcgggtttctggagtttaatgagctaagcacatacgtcagaaaccattattgcgcgttcaaaagtcgcctaaggtcactatca gctagcaaatatttcttgtcaaaaatgctccactgacgttccataaattcccctcggtatccaattagagtctcatattcactctcaatccaaataatctgcaccg gatctggatcgtttcgc |
| minimal 35S (m35S) | cgcaagacccttcctctatataaggaagttcatttcatttggagaggacacgctc |
| omega Enhancer (oEnh) | atttttacaacaattaccaacaacaacaaacaacaaacaacattacaattacatttacaattacca |
| Nos terminator (Nos) | gcgggactctggggttcgaaatgaccgaccaagcgacgcccaacctgccatcacgagatttcgattccaccgccgccttctatgaaaggttgggcttcg gaatcgtttccgggacgccggctggatgatcctccagcgcggggatctcatgctggagttcttcgcccacgggatctctgcggaacaggcggtcgaag gtgccgatatcattacgacagcaacggccgacaagcacaacgccacgatcctgagcgacaatatgatcgggcccggcgtccacatcaacggcgtcgg cggcgactgcccaggcaagaccgagatgcaccgcgatatcttgctgcgttcggatattttcgtggagttcccgccacagacccggatgatccccgatcg ttcaaacatttggcaataaagtttcttaagattgaatcctgttgccggtcttgcgatgattatcatataatttctgttgaattacgttaagcatgtaataattaacatg taatgcatgacgttatttatgagatgggtttttatgattagagtcccgcaattatacatttaatacgcgatagaaaacaaaatatagcgcgcaaactaggataa attatcgcgcgcggtgtcatctatgttactagatcggg |

(continued)

| Element | Sequence |
|---|---|
| E9 terminator (E9) | gatcctctagctagagctttcgttcgtatcatcggtttcgacaacgttcgtcaagttcaatgcatcagtttcattgcgcacacaccagaatcctactgagtttga gtattatggcattgggaaaactgtttttcttgtaccatttgttgtgcttgtaatttactgtgttttttattcggttttcgctatcgaactgtgaaatggaaatggatgga gaagagttaatgaatgatatggtccttttgttcattctcaaattaatattatttgttttttctcttatttgttgtgtgttgaatttgaaattataagagatatgcaaacattt tgttttgagtaaaaatgtgtcaaatcgtggcctctaatgaccgaagttaatatgaggagtaaaacacttgtagttgtaccattatgcttattcactaggcaacaa atatattttcagacctagaaaagctgcaaatgttactgaatacaagtatgtcctcttgtgtgttttagacatttatgaactttcctttatgtaattttccagaatccttgtc agattctaatcattgctttataattatagttatactcatggatttgtagttgagtatgaaaatattttttaatgcattttatgacttgccaattgattgacaacatgcatc aatcgacctgcagccactcgaagcggccggccgccac |
| NPmito::Sul fonamide | agctcatttttacaacaattaccaacaacaacaaacaacaaacaacattacaattacatttacaattatcgatggcttctcggaggcttctcgcctctctcctcc gtcaatcggctcaacgtggcggcggtctaatttcccgatcgttaggaaactccatccctaaatccgcttcacgcgcctcttcacgcgcatcccctaaggga ttcctcttaaaccgcgccgtacagtacgctacctccgcagcggcaccggcatctcagccatcaacaccaccaaagtccggcagtgaaccgtccggaaa aattaccgatgagttcaccggcgctggttcgatcggtgccatggataaatcgctcatcattttcggcatcgtcaacataacctcggacagtttctccgatgga ggccggtatctggcgccagacgcagccattgcgcaggcgcgtaagctgatggccgaggggggcagatgtgatcgacctcggtccggcatccagcaat cccgacgccgcgcctgtttcgtccgacacagaaatcgcgcgtatcgcgccggtgctggacgcgctcaaggcagatggcattcccgtctcgctcgacag ttatcaacccgcgacgcaagcctatgccttgtcgcgtggtgtggcctatctcaatgatattcgcggttttccagacgctgcgttctatccgcaattggcgaaa tcatctgccaaactcgtcgttatgcattcggtgcaagacgggcaggcagatcggcgcgaggcacccgctggcgacatcatggatcacattgcggcgttc |
|  | tttgacgcgcgcatcgcggcgctgacggggtgccggtatcaaacgcaaccgccttgtccttgatcccggcatggggttttttctgggggctgctcccgaaa cctcgctctcggtgctggcgcggttcgatgaattgcggctgcgcttcgatttgccggtgcttctgtctgtttcgcgcaaatcctttctgcgcgcgctcacagg ccgtggtccgggggatgtcggggccgcgacactcgctgcagagcttgccgccgccgcaggtggagctgacttcatccgcacacacgagccgcgccc cttgcgcgacgggctggcggtattggcggcgctgaaagaaaccgcaaggattcgttaa |

35S Expression Vectors

pMEN001 is a derivative ofpBI121 in which kanamycin resistance gene is driven by the *Nos* promoter. pMEN001 was used for the initial cloning of a number of *Arabidopsis* transcription factors. (Sequence of pMEN001 polylinker = SEQ ID NO: 78)

pMEN20 is an earlier version of pMEN65 in which the kanamycin resistance gene is driven by the 35S promoter rather than the *nos* promoter. It is the base vector for P5381, P5425, P5375, and some of the older *Arabidopsis* transcription factor overexpression constructs. (Sequence of pMEN20 polylinker = SEQ ID NO: 79)

pMEN65 is a derivative of pMON10098. The only differences between pMEN65 and pMON10098 are the polylinker and the fact that the kanamycin gene is driven by the *nos* promoter. pMEN65 is the base vector for the majority of the transcription factor overexpression clones. (Sequence of pMEN65 = SEQ ID NO: 80);

pMEN65 primers:

| 35S | gcaagtggattgatgtgatatc |
|---|---|
| 05183 | tttggagaggacacgctgacaa |
| 06344 | atccggtacgaggcctgtctagag |
| E9 | caaactcagtaggattctggtgtgt |

pMEN65 polvlinker:

```
                    gcaagtggattgatgtgatatc->primer 35S
CCAACCACGTCTTCAAAGCAAGTGGATTGATGTGATATCTCCACTGACGTAAGGGATGACGCACAATCCCACTATCCTTCGCAAGAC
CCT
GGTTGGTGCAGAAGTTTCGTTCACCTAACTACACTATAGAGGTGACTGCATTCCCTACTGCGTGTTAGGGTGATAGGAAGCGTTCTG
GGA


                    tttggagaggacacgctgacaa->primer O5183
TCCTCTATATAAGGAAGTTCATTTCATTTGGAGAGGACACGCTGACAAGCTGACTCTAGCAGATCTGGTACCGTCGACGGTGAGCTC
CGC
AGGAGATATATTCCTTCAAGTAAAGTAAACCTCTCCTGTGCGACTGTTCGACTGAGATCGTCTAGACCATGGCAGCTGCCACTCGAG
GCG
                                            --------pMEN65 MCS------------


GGCCGCTCTAGACAGGCCTCGTACCGGATCCTCTAGCTAGAGCTTTCGTTCGTATCATCGGTTTCGACAACGTTCGTCAAGTTCAAT
GCA
CCGGCGAGATCTGTCCGGAGCATGGCCTAGGAGATCGATCTCGAAAGCAAGCATAGTAGCCAAAGCTGTTGCAAGCAGTTCAAGTTA
CGT
    <-gagatctgtccggagcatggccta primer O6344


TCAGTTTCATTGCGCACACACCAGAATCCTACTGAGTTTGAGTATTATGGCATT
AGTCAAAGTAACGCGTGTGTGGTCTTAGGATGACTCAAACTCATAATACCGTAA


                    <-tgtgtggtcttaggatgactcaaac  primer E9
```

**Example IV. Transformation**

Transformation of Arabidopsis was performed by an *Agrobacterium-mediated* protocol based on the method of Bechtold and Pelletier (1998). Unless otherwise specified, all experimental work was done using the Columbia ecotype.

Plant preparation. *Arabidopsis* seeds were sown on mesh covered pots. The seedlings were thinned so that 6-10 evenly spaced plants remained on each pot 10 days after planting. The primary bolts were cut off a week before transformation to break apical dominance and encourage auxiliary shoots to form. Transformation was typically performed at 4-5 weeks after sowing.

Bacterial culture preparation. *Agrobacterium* stocks were inoculated from single colony plates or from glycerol stocks and grown with the appropriate antibiotics and grown until saturation. On the morning of transformation, the saturated cultures were centrifuged and bacterial pellets were re-suspended in Infiltration Media (0.5X MS, 1X B5 Vitamins, 5% sucrose, 1 mg/ml benzylaminopurine riboside, 200 μl/L Silwet L77) until an A600 reading of 0.8 is reached.

Transformation and seed harvest. The *Agrobacterium* solution was poured into dipping containers. All flower buds and rosette leaves of the plants were immersed in this solution for 30 seconds. The plants were laid on their side and wrapped to keep the humidity high. The plants were kept this way overnight at 4 °C and then the pots were turned upright, unwrapped, and moved to the growth racks.

The plants were maintained on the growth rack under 24-hour light until seeds were ready to be harvested. Seeds were harvested when 80% of the siliques of the transformed plants are ripe (approximately 5 weeks after the initial transformation). This seed was deemed T0 seed, since it was obtained from the T0 generation, and was later plated on selection plates (either kanamycin or sulfonamide, see Example VI). Resistant plants that were identified on such selection plates comprised the T1 generation.

**Example V. Morphology**

Morphological analysis was performed to determine whether changes in transcription factor levels affect plant growth and development. This was primarily carried out on the T1 generation, when at least 10-20 independent lines were examined. However, in cases where a phenotype required confirmation or detailed characterization, plants from subsequent generations were also analyzed.

Primary transformants were selected on MS medium with 0.3% sucrose and 50 mg/l kanamycin. T2 and later generation plants were selected in the same manner, except that kanamycin was used at 35 mg/l. In cases where lines carry a sulfonamide marker (as in all lines generated by super-transformation), seeds were selected on MS medium with 0.3% sucrose and 1.5 mg/l sulfonamide. KO lines were usually germinated on plates without a selection. Seeds were cold-treated (stratified) on plates for 3 days in the dark (in order to increase germination efficiency) prior to transfer to growth cabinets. Initially, plates were incubated at 22°C under a light intensity of approximately 100 microEinsteins for 7 days. At this stage, transformants were green, possessed the first two true leaves, and were easily distinguished from bleached kanamycin or sulfonamide-susceptible seedlings. Resistant seedlings were then transferred onto soil (Sunshine potting mix). Following transfer to soil, trays of seedlings were covered with plastic lids for 2-3 days to maintain humidity while they became established. Plants were grown on soil under fluorescent light at an intensity of 70-95 microEinsteins and a temperature of 18-23°C. Light conditions consisted of a 24-hour photoperiod unless otherwise stated. In instances where alterations in flowering time was apparent, flowering may was re-examined under both 12-hour and 24-hour light to assess whether the phenotype was photoperiod dependent. Under our 24-hour light growth conditions, the typical generation time (seed to seed) was approximately 14 weeks.

Because many aspects of *Arabidopsis* development are dependent on localized environmental conditions, in all cases plants were evaluated in comparison to controls in the same flat. As noted below, controls for transgenic lines were wild-type plants, plants overexpressing CBF4, or transgenic plants harboring an empty transformation vector selected on kanamycin or sulfonamide. Careful examination was made at the following stages: seedling (1 week), rosette (2-3 weeks), flowering (4-7 weeks), and late seed set (8-12 weeks). Seed was also inspected. Seedling morphology was assessed on selection plates. At all other stages, plants were macroscopically evaluated while growing on soil. All significant differences (including alterations in growth rate, size, leaf and flower morphology, coloration and flowering time) were recorded, but routine measurements were not be taken if no differences were apparent. In certain cases, stem sections were stained to reveal lignin distribution. In these instances, hand-sectioned stems were mounted in phloroglucinol saturated 2M HCl (which stains lignin pink) and viewed immediately under a dissection microscope.

Note that for a given project (gene-promoter combination, GAL4 fusion lines, RNAi lines etc.), ten lines were typically examined in subsequent plate based physiology assays.

**Example VI. Physiology Experimental Methods**

Plate Assays. Twelve different plate-based physiological assays (shown below), representing a variety of drought-stress related conditions, were used as a pre-screen to identify top performing lines from each project (i.e. lines from transformation with a particular construct), that may be tested in subsequent soil based assays. Typically, ten lines were subjected to plate assays, from which the best three lines were selected for subsequent soil based assays. However, in projects where significant stress tolerance was not obtained in plate based assays, lines were not submitted for soil assays.

In addition, some projects were subjected to nutrient limitation studies. A nutrient limitation assay was intended to find genes that allow more plant growth upon deprivation of nitrogen. Nitrogen is a major nutrient affecting plant growth and development that ultimately impacts yield and stress tolerance. These assays monitor primarily root but also rosette growth on nitrogen deficient media. In all higher plants, inorganic nitrogen is first assimilated into glutamate, glutamine, aspartate and asparagine, the four amino acids used to transport assimilated nitrogen from sources (e.g. leaves) to sinks (e.g. developing seeds). This process is regulated by light, as well as by C/N metabolic status of the plant. We used a C/N sensing assay to look for alterations in the mechanisms plants use to sense internal levels of carbon and nitrogen metabolites which could activate signal transduction cascades that regulate the transcription of N-assimilatory genes. To determine whether these mechanisms are altered, we exploited the observation that wild-type plants grown on media containing high levels of sucrose (3%) without a nitrogen source accumulate high levels of anthocyanins. This sucrose induced anthocyanin accumulation can be relieved by the addition of either inorganic or organic nitrogen. We used glutamine as a nitrogen source since it also serves as a compound used to transport N in plants.

Germination assays. NaCl (150 mM), mannitol (300 mM), sucrose (9.4%), ABA (0.3$\mu$M), Heat (32°C), Cold (8°C), -N is basal media minus nitrogen plus 3% sucrose and -N/+Gln is basal media minus nitrogen plus 3% sucrose and 1 mM glutamine.

Growth assays. Growth assays consisted of severe dehydration (plate-based desiccation or drought), heat (32°C for 5 days followed by recovery at 22°C), chilling (8°C), root development (visual assessment of lateral and primary roots, root hairs and overall growth). For the nitrogen limitation assay, all components of MS medium remained constant except nitrogen was reduced to 20 mg/L of $NH_4NO_3$. Note that 80% MS had 1.32 g/L $NH_4NO_3$ and 1.52 g/L $KNO_3$.

Unless otherwise stated, all experiments were performed with the *Arabidopsis thaliana* ecotype Columbia (col-0). Assays were usually performed on non-selected segregating T2 populations (in order to avoid the extra stress of selection). Control plants for assays on lines containing direct promoter-fusion constructs were Col-0 plants transformed an empty transformation vector (pMEN65). Controls for 2-component lines (generated by supertransformation) were the background promoter-driver lines (i.e. promoter::LexA-GAL4TA lines), into which the supertransformations were initially performed.

All assays were performed in tissue culture. Growing the plants under controlled temperature and humidity on sterile medium produced uniform plant material that had not been exposed to additional stresses (such as water stress) which could cause variability in the results obtained. All assays were designed to detect plants that were more tolerant or less tolerant to the particular stress condition and were developed with reference to the following publications: Jang et al. (1997), Smeekens (1998), Liu and Zhu (1997), Saleki et al. (1993), Wu et al. (1996), Zhu et al. (1998), Alia et al. (1998), Xin and Browse, (1998), Leon-Kloosterziel et al. (1996). Where possible, assay conditions were originally tested in a blind experiment with controls that had phenotypes related to the condition tested.

Procedures

Prior to plating, seed for all experiments were surface sterilized in the following manner: (1) 5 minute incubation with mixing in 70 % ethanol, (2) 20 minute incubation with mixing in 30% bleach, 0.01% triton-X 100, (3) 5X rinses with sterile water, (4) Seeds were re-suspended in 0.1% sterile agarose and stratified at 4 °C for 3-4 days.

All germination assays follow modifications of the same basic protocol. Sterile seeds were sown on the conditional media that had a basal composition of 80% MS + Vitamins. Plates were incubated at 22 °C under 24-hour light (120-130 $\mu$E m$^{-2}$ s$^{-1}$) in a growth chamber. Evaluation of germination and seedling vigor was performed 5 days after planting. For assessment of root development, seedlings germinated on 80% MS + Vitamins + 1% sucrose were transferred to square plates at 7 days. Evaluation was done 5 days after transfer following growth in a vertical position. Qualitative differences were recorded including lateral and primary root length, root hair number and length, and overall growth.

For chilling (8 °C) and heat sensitivity (32 °C) growth assays, seeds were germinated and grown for 7 days on MS + Vitamins + 1% sucrose at 22 °C and then were transferred to chilling or heat stress conditions. Heat stress was applied for 5 days, after which the plants were transferred back to 22 °C for recovery and evaluated after a further 5 days. Plants were subjected to chilling conditions (8 °C) and evaluated at 10 days and 17 days.

For plate-based severe dehydration assays (sometimes referred to as desiccation assays), seedlings were grown for 14 days on MS+ Vitamins + 1% Sucrose at 22 °C. Plates were opened in the sterile hood for 3 hr for hardening and then seedlings were removed from the media and dried for 2 h in the hood. After this time they were transferred back to plates and incubated at 22 °C for recovery. Plants were evaluated after another 5 days.

Data interpretation

At the time of evaluation, plants were given one of the following scores:

(++) Substantially enhanced performance compared to controls. The phenotype was very consistent and growth was significantly above the normal levels of variability observed for that assay.
(+) Enhanced performance compared to controls. The response was consistent but was only moderately above the normal levels of variability observed for that assay.
(wt) No detectable difference from wild-type controls.
(-) Impaired performance compared to controls. The response was consistent but was only moderately above the normal levels of variability observed for that assay.
(- -) Substantially impaired performance compared to controls. The phenotype was consistent and growth was significantly above the normal levels of variability observed for that assay.
(n/d) Experiment failed, data not obtained, or assay not performed.

**Example VI. Soil Drought (Clay Pot)**

The soil drought assay (performed in clay pots) was based on that described by Haake et al. (2002).

Experimental Procedure.

Previously, we performed clay-pot assays on segregating T2 populations, sown directly to soil. However, in the current procedure, seedlings were first germinated on selection plates containing either kanamycin or sulfonamide.
Seeds were sterilized by a 2 minute ethanol treatment followed by 20 minutes in 30% bleach / 0.01% Tween and five washes in distilled water. Seeds were sown to MS agar in 0.1% agarose and stratified for 3 days at 4 °C, before transfer to growth cabinets with a temperature of 22 °C. After 7 days of growth on selection plates, seedlings were transplanted to 3.5 inch diameter clay pots containing 80g of a 50:50 mix of vermiculite:perlite topped with 80g of ProMix. Typically, each pot contains 14 seedlings, and plants of the transgenic line being tested are in separate pots to the wild-type controls. Pots containing the transgenic line versus control pots were interspersed in the growth room, maintained under 24-hour light conditions (18 - 23 °C, and 90 - 100 $\mu$E m$^{-2}$ S$^{-1}$) and watered for a period of 14 days. Water was then withheld and pots were placed on absorbent paper for a period of 8-10 days to apply a drought treatment. After this period, a visual qualitative "drought score" from 0-6 was assigned to record the extent of visible drought stress symptoms. A score of "6" corresponded to no visible symptoms whereas a score of "0" corresponded to extreme wilting and the leaves having a "crispy" texture. At the end of the drought period, pots were re-watered and scored after 5-6 days; the number of surviving plants in each pot was counted, and the proportion of the total plants in the pot that survived was calculated.
Split-pot method. A variation of the above method was sometimes used, whereby plants for a given transgenic line were compared to wild-type controls in the same pot. For those studies, 7 wild-type seedlings were transplanted into one half of a 3.5 inch pot and 7 seedlings of the line being tested were transplanted into the other half of the pot.
Analysis of results. In a given experiment, we typically compared 6 or more pots of a transgenic line with 6 or more pots of the appropriate control. (In the split pot method, 12 or more pots are used.) The mean drought score and mean proportion of plants surviving (survival rate) were calculated for both the transgenic line and the wild-type pots. In each case a p-value* was calculated, which indicated the significance of the difference between the two mean values. The results for each transgenic line across each planting for a particular project were then presented in a results table.
Calculation of p-values. For the assays where control and experimental plants were in separate pots, survival was analyzed with a logistic regression to account for the fact that the random variable was a proportion between 0 and 1. The reported *p*-value was the significance of the experimental proportion contrasted to the control, based upon regressing the logit-transformed data.
Drought score, being an ordered factor with no real numeric meaning, was analyzed with a non-parametric test between the experimental and control groups. The p-value was calculated with a Mann-Whitney rank-sum test.
For the split-pot assays, matched control and experimental measurements were available for both variables. In lieu of a direct transformed regression technique for these data, the logit-transformed proportions were analyzed by parametric methods. The p-value was derived from a paired-*t*-test on the transformed data. For the paired score data, the *p*-value from a Wilcoxon test was reported.

**Example VIII. Soil Drought (Single Pot)**

These experiments determined the physiological basis for the drought tolerance conferred by each lead and were typically performed under soil grown conditions. Usually, the experiment was performed under photoperiodic conditions of 10-hr or 12-hr light. Where possible, a given project (gene/promoter combination or protein variant) was represented by three independent lines. Plants were usually at late vegetative/early reproductive stage at the time measurements were taken. Typically we assayed three different states: a well-watered state, a mild-drought state and a moderately severe drought state. In each case, we made comparisons to wild-type plants with the same degree of physical stress symptoms (wilting). To achieve this, staggered samplings were often required. Typically, for a given line, ten individual plants were assayed for each state.

The following physiological parameters were routinely measured: relative water content, ABA content, proline content, and photosynthesis rate. In some cases, measurements of chlorophyll levels, starch levels, carotenoid levels, and chlorophyll fluorescence were also made.

Analysis of results. In a given experiment, for a particular parameter, we typically compared about 10 samples from a given transgenic line with about 10 samples of the appropriate wild-type control at each drought state. The mean values for each physiological parameter were calculated for both the transgenic line and the wild-type pots. In each case, a P-value (calculated via a simple t-test) was determined, which indicated the significance of the difference between the two mean values. The results for each transgenic line across each planting for a particular project were then presented in a results table.

A typical procedure is described below; this corresponds to method used for the drought time-course experiment which we performed on wild-type plants during our baseline studies at the outset of the drought program.

Procedure. Seeds were stratified for 3 days at 4° C in 0.1% agarose and sown on Metromix 200 in 2.25 inch pots (square or round). Plants were maintained in individual pots within flats grown under short days (10:14 L:D). Seedlings were watered as needed to maintain healthy plant growth and development. At 7 to 8 weeks after planting, plants were used in drought experiments.

Plants matched for equivalent growth development (rosette size) were removed from plastic flats and placed on absorbent paper. Pots containing plants used as well-watered controls were placed within a weigh boat and the dish placed on the absorbent paper. The purpose of the weigh boat was to retain any water that might leak from well-watered pots and affect pots containing plants undergoing the drought stress treatment.

On each day of sampling, up to 18 droughted plants and 6 well-watered controls (from each transgenic line) were picked from a randomly generated pool (given that they passed quality control standards). Biochemical analysis for photosynthesis, ABA, and proline was performed on the next three youngest, most fully expanded leaves. Relative water content was analyzed using the remaining rosette tissue.

**Example IX. Soil Drought (Biochemical and Physiological Assays)**

Background. The purpose of these measurements was to determine the physiological state of plants in soil drought experiments.

Measurement of Photosynthesis. Photosynthesis was measured using a LICOR LI-6400. The LI-6400 uses infrared gas analyzers to measure carbon dioxide to generate a photosynthesis measurement. This method is based upon the difference of the $CO_2$ reference (the amount put into the chamber) and the $CO_2$ sample (the amount that leaves the chamber). Since photosynthesis is the process of converting $CO_2$ to carbohydrates, we expected to see a decrease in the amount of $CO_2$ sample. From this difference, a photosynthesis rate can be generated. In some cases, respiration may occur and an increase in $CO_2$ detected. To perform measurements, the LI-6400 was set-up and calibrated as per LI-6400 standard directions. Photosynthesis was measured in the youngest most fully expanded leaf at 300 and 1000 ppm $CO_2$ using a metal halide light source. This light source provided about 700 $\mu$E m$^{-2}$ s$^{-1}$.

Fluorescence was measured in dark and light adapted leaves using either a LI-6400 (LICOR) with a leaf chamber fluorometer attachment or an OS-1 (Opti-Sciences) as described in the manufacturer's literature. When the LI-6400 was used, all manipulations were performed under a dark shade cloth. Plants were dark adapted by placing in a box under this shade cloth until used. The OS-30 utilized small clips to create dark adapted leaves.

Measurement of Abscisic Acid and Proline. The purpose of this experiment was to measure ABA and proline in plant tissue. ABA is a plant hormone believed to be involved in stress responses and proline is an osmoprotectant.

Three of the youngest, most fully expanded mature leaves were harvested, frozen in liquid nitrogen, lyophilized, and a dry weight measurement taken. Plant tissue was then homogenized in methanol to which 500 ng of d6-ABA had been added to act as an internal standard. The homogenate was filtered to removed plant material and the filtrate evaporated to a small volume. To this crude extract, approximately 3 ml of 1% acetic acid was added and the extract was further evaporated to remove any remaining methanol. The volume of the remaining aqueous extract was measured and a small aliquot (usually 200 to 500 $\mu$l) removed for proline analysis (Protocol described below). The remaining extract was then partitioned twice against ether, the ether removed by evaporation and the residue methylated using ethereal diazomethane. Following removal of any unreacted diazomethane, the residue was dissolved in 100 to 200 $\mu$l ethyl acetate and analyzed by gas chromatography-mass spectrometry. Analysis was performed using an HP 6890 GC coupled to an HP 5973 MSD using a DB-5ms gas capillary column. Column pressure was 20 psi. Initially, the oven temperature was 150 ˚C. Following injection, the oven was heated at 5 ˚C/min to a final temperature of 250 ˚C. ABA levels were estimated using an isotope dilution equation and normalized to tissue dry weight.

Free proline content was measured according to Bates (Bates et al., 1973). The crude aqueous extract obtained above was brought up to a fmal volume of 500 $\mu$l using distilled water. Subsequently, 500 $\mu$l of glacial acetic was added followed by 500 $\mu$l of Chinard's Ninhydrin. The samples were then heated at 95 to 100 ˚C for 1 hour. After this incubation period, samples were cooled and 1.5 ml of toluene were added. The upper toluene phase was removed and absorbance measured at 515 nm. Amounts of proline were estimated using a standard curve generated using L-proline and normalized to tissue dry weight.

[n.b. Chinard's Ninhydrin was prepared by dissolving 2.5 g ninhydrin (triketohydrindene hydrate) in 60 ml glacial acetic acid at 70 ˚C to which 40 ml of 6 M phosphoric acid was added.]

Measurement of Relative Water Content (RWC). Relative Water Content (RWC) indicates the amount of water that is stored within the plant tissue at any given time. It was obtained by taking the field weight of the rosette minus the dry weight of the plant material and dividing by the weight of the rosette saturated with water minus the dry weight of the plant material. The resulting RWC value can be compared from plant to plant, regardless of plant size.

$$\text{Relative Water Content} = \frac{\text{Field Weight - Dry Weight}}{\text{Turgid Weight - Dry Weight}} \times 100$$

After tissue had been removed for array and ABA/proline analysis, the rosette was cut from the roots using a small pair of scissors. The field weight was obtained by weighing the rosette. The rosette was then immersed in cold water and placed in an ice water bath in the dark. The purpose of this was to allow the plant tissue to take up water while preventing any metabolism which could alter the level of small molecules within the cell. The next day, the rosette was carefully removed, blotted dry with tissue paper, and weighed to obtain the turgid weight. Tissue was then frozen, lyophilized, and weighed to obtain the dry weight.

Starch determination. Starch was estimated using a simple iodine based staining procedure. Young, fully expanded leaves were harvested either at the end or beginning of a 12 h light period and placed in tubes containing 80% ethanol or 100% methanol. Leaves were decolorized by incubating tubes in a 70 to 80 C water bath until chlorophyll had been removed from leaf tissue. Leaves were then immersed in water to displace any residual methanol which may be present in the tissue. Starch was then stained by incubating leaves in an iodine stain (2 g KI, 1 g $I_2$ in 100 ml water) for one min and then washing with copious amounts of water. Tissue containing large amounts of starch stained dark blue or black; tissues depleted in starch were colorless.

Chlorophyll/carotenoid determination. For some experiments, chlorophyll was estimated in methanolic extracts using the method of Porra et al. (1989). Carotenoids were estimated in the same extract at 450 nm using an A(1%) of 2500. We currently are measuring chlorophyll using a SPAD-502 (Minolta). When the SPAD-502 was being used to measure chlorophyll, both carotenoid and chlorophyll content and amount could also be determined via HPLC. Pigments were extracted from leave tissue by homogenizing leaves in acetone:ethyl acetate (3:2). Water was added, the mixture centrifuged, and the upper phase removed for HPLC analysis. Samples were analyzed using a Zorbax C18 (non-endcapped) column (250 x 4.6) with a gradient of acetonitrile:water (85:15) to acetonitrile:methanol (85:15) in 12.5 minutes. After holding at these conditions for two minutes, solvent conditions were changed to methanol:ethyl acetate (68:32) in two minutes.

Carotenoids and chlorophylls were quantified using peak areas and response factors calculated using lutein and beta-carotene as standards.

Quantification of protein level. Protein level quantification was performed for 35S::G481 and related projects. Plants were plated on selective MS media, and transplanted to vertical MS plates after one week of growth. After 17 days of growth (24 h light, 22 C), tissues were harvested from the vertical plates. The shoot tissue from 1 plant was harvested as one biological replicate for each line, and the root tissue from 2 plants were combined as 1 biological replicate. For each line analyzed, two biological replicates each of shoot and root tissue were analyzed. Whole cell protein extracts were prepared in a 96 well format and separated on a 4-20% SDS-PAGE gel, transferred to PVDF membrane for western blotting, and probed with a 1:2000 dilution of anti-G481 antibody in a 1% blocking solution in TBS-T. Protein levels for various samples were estimated by setting a level of one for pMEN65 wild type and three for line G481-6 to describe the amount of G481 protein visible on the blot. The protein level for each of the other lines tested was visually estimated on each blot relative to the pMEN65 and G481-6 standards.

Nuclear and cytoplasmically-enriched fractions. We developed a platform to prepare nuclear and cytoplasmic protein extracts in a 96-well format using a tungsten carbide beads for cell disruption in a mild detergent and a sucrose cushion to separate cytoplasmic from nuclear fractions. We used histone antibodies to demonstrate that this method effectively separated cytoplasmic from nuclear-enriched fractions. An alternate method (spun only) used the same disruption procedure, but simply pelleted the nuclei to separate them from the cytoplasm without the added purification of a sucrose cushion.

Quantification of mRNA level. Three shoot and three root biological replicates were typically harvested for each line, as described above in the protein quantification methods section. RNA was prepared using a 96-well format protocol, and cDNA synthesized from each sample. These preparations were used as templates for RT-PCR experiments. We measured the levels of transcript for a gene of interest (such as G481) relative to 18S RNA transcript for each sample using an ABI 7900 Real-Time RT-PCR machine with SYBR Green technology.

Phenotypic Analysis: Flowering time. Plants were grown in soil. Flowering time was determined based on either or both of (i) number to days after planting to the first visible flower bud. (ii) the total number of leaves (rosette or rosette plus cauline) produced by the primary shoot meristem.

Phenotypic Analysis: Heat stress. In preliminary experiments described in this report, plants were germinated growth chamber at 30 C with 24 h light for 11 d. Plants were allowed to recover in 22 C with 24 h light for three days, and photographs were taken to record health after the treatment. In a second experiment, seedlings were grown at 22 C for four days on selective media, and the plates transferred to 32 C for one week. They were then allowed to recover at 22 C for three days. Forty plants from two separate plates were harvested for each line, and both fresh weight and chlorophyll content measured.

Phenotypic Analysis: Dark-induced senescence. In preliminary experiments described in this report, plants were grown on soil for 27-30 days in 12h light at 22 C. They were moved to a dark chamber at 22 C, and visually evaluated for senescence after 10-13 days. In some cases we used Fv/Fm as a measure of chlorophyll (Pourtau et al., 2004) on the youngest most fully-expanded leaf on each plant. The Fv/Fm mean for the 12 plants from each line was normalized to the Fv/Fm mean for the 12 matched controls.

Microscopy. Light microscopy was performed by us. Electron and confocal microscopy were performed using the facilities at University of California, Berkeley.

Various definitions used in this report:

$$RWC = \text{Relative water content (field wt.} - \text{dry weight)/(turgid wt.} - \text{dry wt.)} \times 100$$

ABA = Abscisic acid, $\mu$g/gdw

Proline = Proline, $\mu$mole/gdw

A 300 = net assimilation rate, $\mu$mole $CO_2/m^2$/s at 300 ppm $CO_2$

A 1000 = net assimilation rate, $\mu$mole $CO_2/m^2$/s at 1000 ppm $CO_2$

Chl SPAD = Chlorophyll estimated by a Minolta SPAD-502, ratio of 650 nm to 940 nm

Total Chl = mg/gfw, estimated by HPLC

Carot = mg/gfw, estimated by HPLC

Fo = minimal fluorescence of a dark adapted leaf

Fm = maximal fluorescence of a dark adapted leaf

Fo' = minimal fluorescence of a light adapted leaf

Fm' = maximal fluorescence of a light adapted leaf

Fs = steady state fluorescence of a light adapted leaf

Psi 1f = water potential (Mpa) of a leaf

Psi p = turgor potential (Mpa) of a leaf

Psi pi = osmotic potential (Mpa) of a leaf

Fv/Fm = (Fm - Fo)/Fm; maximum quantum yield of PSII

Fv'/Fm' = (Fm' - Fo')/Fm'; efficiency of energy harvesting by open PSII reaction centers

PhiPS2 = (Fm' - Fs)/Fm', actual quantum yield of PSII

ETR = PhiPS2 x light intensity absorbed x 0.5; we use 100 $\mu$E/m$^2$/s for an average light intensity and 85% as the amount of light absorbed

qP = (Fm' - Fs)/(Fm'- Fo'); photochemical quenching (includes photosynthesis and photorespiration); proportion of open PSII

qN = (Fm - Fm')/(Fm - Fo'); non- photochemical quenching (includes mechanisms like heat dissipation)

NPQ = (Fm - Fm')/Fm'; non-photochemical quenching (includes mechanisms like heat dissipation)

**Example X. Experimental Results**

The following table summarizes the experimental results that have yielded new phenotypic traits in morphological, physiological or disease assays in *Arabidopsis.* The last column lists the trait that was experimentally observed in plants after transforming with each transcription factor polynucleotide GID (Gene IDentifier, found in the first column) under the listed regulatory control mechanism (found in the fifth or "Project Column").

Table 5. Phenotypic traits conferred by *Arabidopsis* transcription factors in morphological, physiological or disease assays in *Arabidopsis*

| GID | SEQ ID NO: | Species from which GID was obtained | Clade | Project | Trait Category | Experimental observation (trait compared to controls) |
|---|---|---|---|---|---|---|
| G3876 | 8 | *Oryza sativa* | G481 | Constitutive 35S | Cold tolerance | Increased tolerance to cold |
| G3876 | 8 | *Oryza sativa* | G481 | Constitutive 35S | Drought tolerance | Increased tolerance to dehydration |

In this Example, unless otherwise indicted, morphological and physiological traits are disclosed in comparison to wild-type control plants. That is, a transformed plant that is described as large and/or drought tolerant is large and more tolerant to drought with respect to a wild-type control plant. When a plant is said to have a better performance than controls, it generally showed less stress symptoms than control plants. The better performing lines may, for example, produce less anthocyanin, or be larger, green, or more vigorous in response to a particular stress, as noted below. Better performance generally implies greater tolerance to a particular biotic or abiotic stress, less sensitivity to ABA, or better recovery from a stress (as in the case of a drought treatment) than controls.

G3876 (SEQ ID NO: 7 and 8: *Oryza sativa*) - Constitutive 35S

Background. G3876 is a maize gene which is a member of the HAP3 (NF-YB) subfamily of the CCAAT-box binding transcription factors. In phylogenetic analyses, the protein lies within the same sub-clade as G481.
Morphological Observations. Two sets of 35S::G3876 lines have been selected. No clear-cut alterations in morphology were noted, although a few of the lines were noted to have slight changes in flowering time. Line 302 was slightly late flowering, whereas #305 and #311 were slightly early flowering. Fifteen other lines appeared wild type in morphology and development.

Physiology (Plate assays) Results. Six of 10 lines tested were more tolerant to cold during germination than wild type, and 4 of 10 lines were more tolerant to desiccation in plate-based assays.

Discussion. A minority of 35S::G3876 lines showed slight alterations in flowering time. Perhaps more significantly, improvements in cold germination and desiccation tolerance were noted in plants that had wild-type development and morphology.

Potential applications. Based on the results obtained so far, G3876 could be applied to modify flowering time. The gene may also be used to improve drought and cold tolerance without causing undesirable morphological or developmental defects.

**Example XI. Transformation of dicots to produce increased biomass, disease resistance or abiotic stress tolerance**

Crop species including tomato and soybean plants that overexpress any of a considerable number of the transcription factor polypeptides of the disclosure have been shown experimentally to produce plants with increased drought tolerance and/or biomass in field trials. For example, tomato plants overexpressing the G2153 polypeptide have been found to be larger than wild-type control tomato plants. For example, soy plants overexpressing a number of G481, G682, G867 and G1073 orthologs have been shown to be more drought tolerant than control plants. These observations indicate that these genes, when overexpressed, will result in larger yields than non-transformed plants in both stressed and non-stressed conditions.

Thus, transcription factor polynucleotide sequences listed in the Sequence Listing recombined into, for example, one of the expression vectors of the disclosure, or another suitable expression vector, may be transformed into a plant for the purpose of modifying plant traits for the purpose of improving yield and/or quality. The expression vector may contain a constitutive, tissue-specific or inducible promoter operably linked to the transcription factor polynucleotide. The cloning vector may be introduced into a variety of plants by means well known in the art such as, for example, direct DNA transfer or *Agrobacterium tumefaciens-mediated* transformation. It is now routine to produce transgenic plants using most dicot plants (see Weissbach and Weissbach, (1989); Gelvin et al. (1990); Herrera-Estrella et al. (1983); Bevan (1984); and Klee (1985)). Methods for analysis of traits are routine in the art and examples are disclosed above.

Numerous protocols for the transformation of tomato and soy plants have been previously described, and are well known in the art. Gruber et al. (1993), and Glick and Thompson (1993) describe several expression vectors and culture methods that may be used for cell or tissue transformation and subsequent regeneration. For soybean transformation, methods are described by Miki et al. (1993) ; and U.S. Pat. No. 5,563,055, (Townsend and Thomas), issued Oct. 8, 1996.

There are a substantial number of alternatives to Agrobacterium-mediated transformation protocols, other methods for the purpose of transferring exogenous genes into soybeans or tomatoes. One such method is microprojectile-mediated transformation, in which DNA on the surface of microprojectile particles is driven into plant tissues with a biolistic device (see, for example, Sanford et al. (1987); Christou et al. (1992); Sanford (1993); Klein et al. (1987); U.S. Pat. No. 5,015,580 (Christou et al), issued May 14, 1991; and U.S. Pat. No. 5,322,783 (Tomes et al.), issued Jun. 21, 1994).

Alternatively, sonication methods (see, for example, Zhang et al. (1991)); direct uptake of DNA into protoplasts using $CaCl_2$ precipitation, polyvinyl alcohol or poly-L-ornithine (Hain et al. (1985); Draper et al. (1982)); liposome or spheroplast fusion (see, for example, Deshayes et al. (1985); Christou et al. (1987)); and electroporation of protoplasts and whole cells and tissues (see, for example, Donn et al.(1990); D'Halluin et al. (1992); and Spencer et al. (1994)) have been used to introduce foreign DNA and expression vectors into plants.

After a plant or plant cell is transformed (and the latter regenerated into a plant), the transformed plant may be crossed with itself or a plant from the same line, a non-transformed or wild-type plant, or another transformed plant from a different transgenic line of plants. Crossing provides the advantages of producing new and often stable transgenic varieties. Genes and the traits they confer that have been introduced into a tomato or soybean line may be moved into distinct line of plants using traditional backcrossing techniques well known in the art. Transformation of tomato plants may be conducted using the protocols of Koornneef et al (1986), and in U.S. Patent 6,613,962, the latter method described in brief here. Eight day old cotyledon explants are precultured for 24 hours in Petri dishes containing a feeder layer of Petunia hybrida suspension cells plated on MS medium with 2% (w/v) sucrose and 0.8% agar supplemented with 10 $\mu$M $\alpha$-naphthalene acetic acid and 4.4 $\mu$M 6-benzylaminopurine. The explants are then infected with a diluted overnight culture *of Agrobacterium tumefaciens* containing an expression vector comprising a polynucleotide of the disclosure for 5-10 minutes, blotted dry on sterile filter paper and cocultured for 48 hours on the original feeder layer plates. Culture conditions are as described above. Overnight cultures of *Agrobacterium tumefaciens* are diluted in liquid MS medium with 2% (w/v/) sucrose, pH 5.7) to an $OD_{600}$ of 0.8.

Following cocultivation, the cotyledon explants are transferred to Petri dishes with selective medium comprising MS medium with 4.56 μM zeatin, 67.3 μM vancomycin, 418.9 μM cefotaxime and 171.6 μM kanamycin sulfate, and cultured under the culture conditions described above. The explants are subcultured every three weeks onto fresh medium. Emerging shoots are dissected from the underlying callus and transferred to glass jars with selective medium without zeatin to form roots. The formation of roots in a kanamycin sulfate-containing medium is a positive indication of a successful transformation.

Transformation of soybean plants may be conducted using the methods found in, for example, U.S. Patent 5,563,055 (Townsend et al., issued October 8, 1996), described in brief here. In this method soybean seed is surface sterilized by exposure to chlorine gas evolved in a glass bell jar. Seeds are germinated by plating on 1/10 strength agar solidified medium without plant growth regulators and culturing at 28° C. with a 16 hour day length. After three or four days, seed may be prepared for cocultivation. The seedcoat is removed and the elongating radicle removed 3-4 mm below the cotyledons.

Overnight cultures of *Agrobacterium tumefaciens* harboring the expression vector comprising a polynucleotide of the disclosure are grown to log phase, pooled, and concentrated by centrifugation. Inoculations are conducted in batches such that each plate of seed was treated with a newly resuspended pellet of *Agrobacterium.* The pellets are resuspended in 20 ml inoculation medium. The inoculum is poured into a Petri dish containing prepared seed and the cotyledonary nodes are macerated with a surgical blade. After 30 minutes the explants are transferred to plates of the same medium that has been solidified. Explants are embedded with the adaxial side up and level with the surface of the medium and cultured at 22° C. for three days under white fluorescent light. These plants may then be regenerated according to methods well established in the art, such as by moving the explants after three days to a liquid counter-selection medium (see U.S. Patent 5,563,055).

The explants may then be picked, embedded and cultured in solidified selection medium. After one month on selective media transformed tissue becomes visible as green sectors of regenerating tissue against a background of bleached, less healthy tissue. Explants with green sectors are transferred to an elongation medium. Culture is continued on this medium with transfers to fresh plates every two weeks. When shoots are 0.5 cm in length they may be excised at the base and placed in a rooting medium.

Example XII: Transformation of monocots to produce increased biomass, disease resistance or abiotic stress tolerance

Cereal plants such as, but not limited to, corn, wheat, rice, sorghum, or barley, may be transformed with the present polynucleotide sequences, including monocot or dicot-derived sequences such as those presented in the present Tables, cloned into a vector such as pGA643 and containing a kanamycin-resistance marker, and expressed constitutively under, for example, the CaMV 35S or COR15 promoters, or with tissue-specific or inducible promoters. The expression vectors may be one found in the Sequence Listing, or any other suitable expression vector may be similarly used. For example, pMEN020 may be modified to replace the NptII coding region with the BAR gene of *Streptomyces hygroscopicus* that confers resistance to phosphinothricin. The KpnI and BglII sites of the Bar gene are removed by site-directed mutagenesis with silent codon changes.

The cloning vector may be introduced into a variety of cereal plants by means well known in the art including direct DNA transfer or *Agrobacterium tumefaciens-mediated* transformation. The latter approach may be accomplished by a variety of means, including, for example, that of U.S. Patent No. 5,591,616, in which monocotyledon callus is transformed by contacting dedifferentiating tissue with the *Agrobacterium* containing the cloning vector.

The sample tissues are immersed in a suspension of $3x10^{-9}$ cells *of Agrobacterium* containing the cloning vector for 3-10 minutes. The callus material is cultured on solid medium at 25° C in the dark for several days. The calli grown on this medium are transferred to Regeneration medium. Transfers are continued every 2-3 weeks (2 or 3 times) until shoots develop. Shoots are then transferred to Shoot-Elongation medium every 2-3 weeks. Healthy looking shoots are transferred to rooting medium and after roots have developed, the plants are placed into moist potting soil.

The transformed plants are then analyzed for the presence of the NPTII gene/ kanamycin resistance by ELISA, using the ELISA NPTII kit from 5Prime-3Prime Inc. (Boulder, CO).

It is also routine to use other methods to produce transgenic plants of most cereal crops (Vasil (1994)) such as corn, wheat, rice, sorghum (Cassas et al. (1993)), and barley (Wan and Lemeaux (1994)). DNA transfer methods such as the microprojectile method can be used for corn (Fromm et al. (1990); Gordon-Kamm et al. (1990); Ishida (1990)), wheat (Vasil et al. (1992); Vasil et al. (1993); Weeks et al. (1993)), and rice (Christou (1991); Hiei et al. (1994); Aldemita and Hodges (1996); and Hiei et al. (1997)). For most cereal plants, embryogenic cells derived from immature scutellum tissues are the preferred cellular targets for transformation (Hiei et al. (1997); Vasil (1994)). For transforming corn embryogenic cells derived from immature scutellar tissue using microprojectile bombardment, the A188XB73 genotype is the preferred genotype (Fromm et al. (1990); Gordon-Kamm et al. (1990)). After microprojectile bombardment the tissues are selected on phosphinothricin to identify the transgenic embryogenic cells (Gordon-Kamm et al. (1990)). Transgenic plants are regenerated by standard corn regeneration techniques (Fromm et al. (1990); Gordon-Kamm et al. (1990)).

Example XVI: Transcription factor expression and analysis of disease resistance or abiotic stress tolerance

Northern blot analysis, RT-PCR or microarray analysis of the regenerated, transformed plants may be used to show expression of a transcription factor polypeptide or the disclosure and related genes that are capable of inducing disease resistance, abiotic stress tolerance, and/or larger size.

To verify the ability to confer stress resistance, mature plants overexpressing a transcription factor of the disclosure, or alternatively, seedling progeny of these plants, may be challenged by a stress such as a disease pathogen, drought, heat, cold, high salt, or desiccation. Alternatively, these plants may challenged in a hyperosmotic stress condition that may also measure altered sugar sensing, such as a high sugar condition. By comparing control plants (for example, wild type) and transgenic plants similarly treated, the transgenic plants may be shown to have greater tolerance to the particular stress.

After a dicot plant, monocot plant or plant cell has been transformed (and the latter regenerated into a plant) and shown to have greater size or tolerance to abiotic stress, or produce greater yield relative to a control plant under the stress conditions, the transformed monocot plant may be crossed with itself or a plant from the same line, a non-transformed or wild-type monocot plant, or another transformed monocot plant from a different transgenic line of plants.

These experiments would demonstrate that transcription factor polypeptides can be identified and shown to confer larger size, greater yield, greater disease resistance and/or abiotic stress tolerance in dicots or monocots, including tolerance or resistance to multiple stresses.

**Example XIV: Sequences that Confer Significant Improvements to non-*Arabidopsis* species**

The function of specific transcription factors of the disclosure, including closely-related orthologs, have been analyzed and may be further characterized and incorporated into crop plants. The ectopic overexpression of these sequences may be regulated using constitutive, inducible, or tissue specific regulatory elements. Genes that have been examined and have been shown to modify plant traits (including increasing biomass, disease resistance and/or abiotic stress tolerance) encode transcription factor polypeptides described herein. In addition to these sequences, it is expected that newly discovered polynucleotide and polypeptide sequences closely related to these polynucleotide and polypeptide sequences can also confer alteration of traits in similar manner to the sequences described herein, when transformed into a any of a considerable variety of plants of different species, and including dicots and monocots. The polynucleotide and polypeptide sequences derived from monocots (e.g., the rice sequences) may be used to transform both monocot and dicot plants, and those derived from dicots (e.g., the *Arabidopsis* and soy genes) may be used to transform either group, although it is expected that some of these sequences will function best if the gene is transformed into a plant from the same group as that from which the sequence is derived.

As an example of a first step to determine drought-related tolerance, seeds of these transgenic plants are subjected to germination assays to measure sucrose sensing. Sterile monocot seeds, including, but not limited to, corn, rice, wheat, rye and sorghum, as well as dicots including, but not limited to soybean and alfalfa, are sown on 80% MS medium plus vitamins with 9.4% sucrose; control media lack sucrose. All assay plates are then incubated at 22° C under 24-hour light, 120-130 $\mu$Ein/m$^2$/s, in a growth chamber. Evaluation of germination and seedling vigor is then conducted three days after planting. Plants overexpressing sequences of the disclosure may be found to be more tolerant to high sucrose by having better germination, longer radicles, and more cotyledon expansion. These methods have been used to show that overexpressors of numerous sequences of the disclosure are involved in sucrose-specific sugar sensing. It is expected that structurally similar orthologs of these sequences, including those found in the Sequence Listing, are also involved in sugar sensing, an indication of altered osmotic stress tolerance.

Plants overexpressing the transcription factor sequences of the disclosure may also be subjected to soil-based drought assays to identify those lines that are more tolerant to water deprivation than wild-type control plants. A number of the lines of plants overexpressing transcription factor polypeptides of the disclosure, including newly discovered closely-related species, will be significantly larger and greener, with less wilting or desiccation, than wild-type control plants, particularly after a period of water deprivation is followed by rewatering and a subsequent incubation period. The sequence of the transcription factor may be overexpressed under the regulatory control of constitutive, tissue specific or inducible promoters, or may comprise a GAL4 transactivation domain fused to either the N- or the C terminus of the polypeptide. The results presented in Examples above indicate that these transcription factors may confer disease resistance or abiotic stress tolerance when they are overexpressed under the regulatory control of non-constitutive promoters or a transactivation domain fused to the clade member, without having a significant adverse impact on plant morphology and/or development. The lines that display useful traits may be selected for further study or commercial development.

Monocotyledonous plants, including rice, corn, wheat, rye, sorghum, barley and others, may be transformed with a plasmid containing a transcription factor polynucleotide. The transcription factor gene sequence may include dicot or monocot-derived sequences such as those presented herein. These transcription factor genes may be cloned into an expression vector containing a kanamycin-resistance marker, and then expressed constitutively or in a tissue-specific or inducible manner.

The cloning vector may be introduced into monocots by, for example, means described in the previous Example, including direct DNA transfer or *Agrobacterium tumefaciens*-mediated transformation. The latter approach may be accomplished by a variety of means, including, for example, that of U.S. Patent No. 5,591,616, in which monocotyledon callus is transformed by contacting dedifferentiating tissue with the *Agrobacterium* containing the cloning vector.

The sample tissues are immersed in a suspension of $3\times10^{-9}$ cells of *Agrobacterium* containing the cloning vector for 3-10 minutes. The callus material is cultured on solid medium at 25° C in the dark for several days. The calli grown on this medium are transferred to Regeneration medium. Transfers are continued every 2-3 weeks (2 or 3 times) until shoots develop. Shoots are then transferred to Shoot-Elongation medium every 2-3 weeks. Healthy looking shoots are transferred to rooting medium and after roots have developed, the plants are placed into moist potting soil.

The transformed plants are then analyzed for the presence of the NPTII gene/ kanamycin resistance by ELISA, using the ELISA NPTII kit from 5Prime-3Prime Inc. (Boulder, CO).

Northern blot analysis, RT-PCR or microarray analysis of the regenerated, transformed plants may be used to show expression of a transcription factor polypeptide of the disclosure that is capable of conferring abiotic stress tolerance, disease resistance, or increased size or yield, in the transformed plants.

To verify the ability to confer abiotic stress tolerance, mature plants or seedling progeny of these plants expressing a monocot-derived equivalog gene may be challenged using methods described in the above Examples. By comparing wild type plants and the transgenic plants, the latter are shown be more tolerant to abiotic stress, more resistant to disease, and/or have increased biomass, as compared to wild type control plants similarly treated.

It is expected that the same methods may be applied to identify other useful and valuable sequences of the present transcription factor clades, and the sequences may be derived from a diverse range of species.

References cited:

Abe et al. (1997) Plant Cell 9: 1859-1868

Abe et al. (2003) Plant Cell 15: 63-78

Affolter et al. (1990) Curr. Opin. Cell. Biol. 2: 485-495

Agrios, G.N. (1997) Plant Pathology. 4th edition. (Academic Press, San Diego, New York)

Aldemita and Hodges (1996) Planta 199: 612-617

Alia et al. (1998) Plant J. 16: 155-161

Allen (1998) EMBO J. 17: 5484-5496.

Altschul (1990) J Mol. Biol. 215: 403-410

Altschul (1993) J. Mol. Evol. 36: 290-300

Anderson and Young (1985) "Quantitative Filter Hybridisation", In: Hames and Higgins, ed., Nucleic Acid Hybridisation, A Practical Approach. Oxford, IRL Press, 73-111

Anderson et al. (2004) Plant Cell 16: 3460-3479.

Aravind and Landsman (1998) Nucleic Acids Res. 26: 4413-4421

Arents and Moudrianakis (1995) Proc. Natl. Acad. Sci. USA 92: 11170-11174

Atchley and Fitch (1997) Proc. Natl. Acad. Sci. USA 94: 5172-5176

Atchley et al. (1999) J. Mol. Evol. 48: 501-516

Ausubel et al. (1997) Short Protocols in Molecular Biology, John Wiley & Sons, New York, NY, unit 7.7

Bailey et al. (2003) Plant Cell 15: 2497-2502

Bairoch et al. (1997) Nucleic Acids Res. 25: 217-221

Bänzinger et al. (2000) Breeding for drought and nitrogen stress tolerance in maize. From theory to practice. (Mexico: CIMMYT (The International Maize and Wheat Improvement Center))

Barthelemy et al. (1996) Biochem. Biophys. Res. Commun. 224: 870-876

Bates et al. (1973) Plant Soil 39: 205-207

Baudino and Cleveland (2001) Mol. Cell. Biol. 21: 691-702

Bechtold and Pelletier (1998) Methods Mol. Biol. 82: 259-266

Berger and Kimmel (1987) "Guide to Molecular Cloning Techniques", in Methods in Enzymology, vol. 152, Academic Press, Inc., San Diego, CA

Berger et al. (1998) Curr. Biol. 8: 421-430

Berrocal-Lobo et al. (2002) Plant J. 29: 23-32

Berrocal-Lobo and Molina (2004) Mol. Plant Microbe Interact. 17: 763-770

Bevan (1984) Nucleic Acids Res. 12: 8711-8721

Bezhani et al. (2001) J. Biol. Chem. 276: 23785-23789

Bhattacharjee et al. (2001) Proc. Natl. Acad. Sci. USA 98: 13790-13795

Bi et al. (1997) J. Biol. Chem. 272: 26562-26572

Bianchi and Beltrame (2000) EMBO Rep. 1: 109-114
Boter (2004) Genes Dev. 18: 1577-1591
Borevitz et al. (2000) Plant Cell 12: 2383-2393
Boss and Thomas (2002) Nature 416: 847-850
Boyer (1995) Annu. Rev. Phytopathol. 33: 251-274.
Brown et al. (2003) Plant Physiol. 132: 1020-1032
Brownlie et al. (1997) Structure 5: 509-520
Bruce et al. (2000) Plant Cell 12: 65-79
Bucher and Trifonov (1988) J. Biomol. Struct. Dyn. 5: 1231-1236
Bucher (1990) J. Mol. Biol. 212: 563-578
Buck and Atchley (2003) J. Mol. Evol. 56: 742-750
Burglin (1997) Nucleic Acids Res. 25: 4173-4180
Burglin (1998) Dev. Genes Evol. 208: 113-116
Byrne (2000) Nature 408: 967-971
Caretti et al. (2003) J. Biol. Chem. 278: 30435-30440
Carre and Kay (1995) Plant Cell 7: 2039-2051
Carroll (2000) Cell 101: 577-580
Carson et al. (1997) Plant J. 12: 1231-1240
Cassas et al. (1993) Proc. Natl. Acad. Sci. USA 90: 11212-11216
Chae et al. (2004) Oncogene 23: 4084-4088
Chakravarthy et al. (2003) Plant Cell 15: 3033-3050
Chang and Liu (1994) J. Biol. Chem. 269: 17893-17898
Chase et al. (1993) Ann. Missouri Bot. Gard. 80: 528-580
Chen et al. (2002a) Plant Cell 14: 559-574.
Chen and Chen (2002) Plant Physiol. 129: 706-716
Cheong et al. (2002) Plant Physiol. 129: 661-677
Cheong et al. (2003) Plant Physiol. 132: 1961-1972
Chini et al. (2004) Plant J. 38: 810-822.
Chinnusamy et al. (2003) Genes Dev. 17: 1043-1054
Chinthapalli et al. (2002) in Reviews in Plant Biochemistry and Biotechnology, Goyal, A. et al (eds.) pp. 143-159
Christou et al. (1987) Proc. Natl. Acad. Sci. USA 84: 3962-3966
Christou (1991) Bio/Technol. 9:957-962
Christou et al. (1992) Plant. J. 2: 275-281
Ciarapica et al. (2003) J. Biol. Chem. 278: 12182-12190
Costa and Dolan (2003) Development 130: 2893-2901
Coupland (1995) Nature 377: 482-483
Coustry et al. (1995) J. Biol. Chem. 270: 468-475
Coustry et al. (1996) J. Biol. Chem. 271: 14485-14491
Coustry et al. (1998) Biochem J. 331(Pt 1): 291-297
Coustry et al. (2001) J. Biol. Chem. 276: 40621-40630.
Crawford et al. (2004) Plant Physiol. 135: 244-253
Crozatier et al. (1996) Curr. Biol. 6: 707-718
Cubas et al. (1999) Plant J. 18: 215-222
Currie (1997) J. Biol. Chem. 272: 30880-30888
Daly et al. (2001) Plant Physiol. 127: 1328-1333
Dang et al. (1992) Proc. Natl. Acad. Sci. USA 89: 599-602
Dang et al. (1996) J. Bacteriol. 178: 1842-1849
de Pater et al. (1996) Nucleic Acids Res. 24: 4624-4631
Dellagi et al. (2000) Mol. Plant Microbe Interact. 13: 1092-110
Deshayes et al. (1985) EMBOJ.: 4: 2731-2737
D'Halluin et al. (1992) Plant Cell 4: 1495-1505
Di Cristina et al. (1996) Plant J. 10: 393-402
Doebley and Lukens (1998) Plant Cell 10: 1075-1082
Donn et al.(1990) in Abstracts of VIIth International Congress on Plant Cell and Tissue Culture IAPTC, A2-38: 53
Doolittle, ed. (1996) Methods in Enzymology, vol. 266: "Computer Methods for Macromolecular Sequence Analysis"
Academic Press, Inc., San Diego, Calif., USA
Draper et al. (1982) Plant Cell Physiol. 23: 451-458
Du and Chen (2000) Plant J. 24: 837-847

Duboule (1994), (ed.) Guidebook to the homeobox genesOxford University Press, Oxford

Duckett et al. (1994) Development 120: 3247-3255

Ecker (1995) Science 268: 667-675

Eddy (1996) Curr. Opin. Str. Biol. 6: 361-365

Edwards et al. (1998) Plant Physiol. 117: 1015-1022.

Eimert et al. (1995) Plant Cell 7: 1703-1712

Ellenberger et al. (1994) Genes Dev. 8: 970-980

Eulgem et al. (1999) EMBO J. 18: 4689-4699

Eulgem (2000) Trends Plant Sci. 5: 199-206.

Ezcurra et al. (2000) Plant J. 24: 57-66

Fairchild et al. (2000) Genes Dev. 14: 2377-2391

Fairman et al. (1993) Proc. Natl. Acad. Sci. USA 90: 10429-10433

Falvo et al. (1995) Cell 83: 1101-1111

Feng and Doolittle (1987) J. Mol. Evol. 25: 351-360

Ferre-D'Amare et al. (1994) EMBO J. 13: 180-189

Finkelstein et al. (1998) Plant Cell 10: 1043-1054

Fischer and Droge-Laser (2004) Mol. Plant Microbe Interact. 17: 1162-1171

Fisher and Goding (1992) EMBO J. 11: 4103-4109

Fisher and Caudy (1998) Bioessays 20: 298-306

Forsburg and Guarente (1988) Genes Dev. 3: 1166-117

Forzani et al. (2001) J. Biol. Chem. 276: 16731-16738

Fowler et al. (2002) Plant Cell 14: 1675-1679

Fowler and Thomashow (2002) Plant Cell 14: 1675-1690

Frampton et al. (1991) Protein Eng. 4: 891-901

Frank et al. (2000) Plant Cell 12: 111-124.

Freeling and Hake (1985) Genetics 111: 617-634

Friedrichsen et al. (2002) Genetics 162: 1445-1456

Fromm et al. (1990) Bio/Technol. 8: 833-839

Fu et al. (2001) Plant Cell 13: 1791-1802

Fuji et al. (2000) Nat. Struct. Biol. 7: 889-893

Fujimoto et al. (2000) Plant Cell 12: 393-404

Fujimoto et al. (2004) Plant Mol. Biol. 56: 225-239

Galigniana et al. (1998) Mol. Endocrinol. 12:1903-1913

Galway et al. (1994) Dev. Biol. 166: 740-754

Gampala et al. (2004). International Conference on Arabidopsis Research. Berlin. Abstract # T04-085

Gancedo (1998) Microbiol. Mol. Biol. Rev. 62: 334-361.

Gaxiola et al. (2001) Proc. Natl. Acad. Sci. U S A 98: 11444-11449.

Gelinas et al. (1985) Prog. Clin. Biol. Res. 191: 125-139

Gelvin et al. (1990) Plant Molecular Biology Manual, Kluwer Academic Publishers

Gilmour et al. (1998) Plant J. 16: 433-442

Giraudat et al. (1992) Plant Cell 4: 1251-1261

Glick and Thompson, eds. (1993) Methods in Plant Molecular Biology and Biotechnology. CRC Press., Boca Raton, FL

Goff et al. (1992) Genes Dev. 6: 864-875

Good and Chen (1996) Biol Signals 5: 163-169

Goodrich et al. (1993) Cell 75: 519-530

Gordon-Kamm et al. (1990) Plant Cell 2: 603-618

Graf (1992) Curr. Opin. Genet. Dev. 2: 249-255.

Grandori et al. (2000) Ann. Rev. Cell. Dev. Biol. 16: 653-699

Grant et al. (2003) Mol. Plant Microbe Interact. 16: 669-680.

Grasser (1995) Plant J. 7: 185-192

Grasser (2003) Plant Mol. Biol. 53: 281-295

Gruber et al. ((1993) in Methods in Plant Molecular Biology and Biotechnology, p. 89-119

Gu et al. (2000) Plant Cell 12: 771-786

Gu et al. (2002) Plant Cell 14: 817-831

Guiltinan et al. (1990) Science 250: 267-271

Guo et al. (2004) Plant Mol. Biol. 55: 607-618.

Gupta et al (1997a) Plant Mol. Biol. 35: 987-992

Gupta et al. (1997b) Plant Mol. Biol. 34: 529-536

Gusmaroli et al. (2001) Gene 264: 173-185

Gusmaroli et al. (2002) Gene 283: 41-48

Haake et al. (2002) Plant Physiol. 130: 639-648

Hain et al. (1985) Mol. Gen. Genet. 199: 161-168

Hall et al. (2000) Plant Physiol. 123: 1449-1458.

Halliday et al. (1999) Proc. Natl. Acad. Sci. USA 96: 5832-5837

Haymes et al. "Nucleic Acid Hybridization: A Practical Approach", IRL Press, Washington, D.C. (1985)

Hanes and Brent (1989) Cell 57: 1275-1283

Hanes and Brent (1991) Science 251: 426-430

Hao et al. (1998) J. Biol. Chem. 273: 26857-26861

Hao (2002) Biochemistry 41: 4202-4208

Harper (2002) WO0216655

Hasegawa et al. (2000) Annu. Rev. Plant Mol. Plant Physiol. 51: 463-499.

Hatch (1987) Biochim. Biophys. Acta 895: 81-106

Hattori et al. (1992) Genes Dev. 6: 609-618

Hayashi and Scott (1990) Cell 63: 883-894

He et al. (2000) Transgenic Res. 9: 223-227

He et al. (2001) Mol. Plant Microbe Interact. 14: 1453-1457

Heim et al. (2003) Mol. Biol. Evol. 20: 735-747

Hein (1990) Methods Enzymol. 183: 626-645

Heisler et al. (2001) Development 128: 1089-1098

Hempel (1997) Development 124: 3845-3853

Henikoff and Henikoff (1991) Nucleic Acids Res. 19: 6565-6572

Herrera-Estrella et al. (1983) Nature 303: 209

Hiei et al. (1994) Plant J. 6:271-282

Hiei et al. (1997) Plant Mol. Biol. 35:205-218

Higgins and Sharp (1988) Gene 73: 237-244

Higgins et al. (1996) Methods Enzymol. 266: 383-402

Hirano et al. (2002) Gene 290: 107-114

Hobo et al. (1999) Proc. Natl. Acad. Sci. USA 96: 15348-15353

Hoecker et al. (1995) Genes Dev. 9: 2459-2469

Hsieh et al. (1998) Proc. Natl. Acad. Sci. USA 95: 13965-13970

Hu et al. (2003) Plant Cell 15: 1951-1961

Hu et al. (2004) Cell Res. 14: 8-15

Hung et al. (1998) Plant Physiol. 117: 73-84

Huq and Quail (2002) EMBO J. 21: 2441-2450

Huth et al. (1997) Nat. Struct. Biol. 4: 657-665

Hwang and Goodman (1995) Plant J. 8: 37-43

Ishida (1990) Nature Biotechnol. 14:745-750

Ishiguro and Nakamura (1994) Mol. Gen. Genet. 244: 563-571

Ito et al. (1995) Plant Cell Physiol. 36: 1281-1289

Jaglo et al. (2001) Plant Physiol. 127: 910-917

Jaglo-Ottosen et al. (1998) Science. 280:104-106

Jakoby et al. (2002) Trends Plant Sci. 7: 106-111

Jaglo et al. (2001) Plant Physiol. 127: 910-917

Jang et al. (1997) Plant Cell 9: 5-19

Jofuku et al. (1994) Plant Cell 6: 1211-1225

Johnson and McKnight (1989) Ann. Rev. Biochem. 58: 799-839

Johnson et al. (2002) Plant Cell 14: 1359-1375

Kagaya et al. (1999) Nucleic Acids Res. 27: 470-478

Kaiser et al. (1998) Science 281: 1202-1206

Kashima et al. (1985) Nature 313: 402-404

Kasuga et al. (1999) Nature Biotechnol. 17: 287-291

Kehoe et al. (1994) Plant Cell 6: 1123-1134

Keith et al. (1994) Plant Cell 6: 589-600

Kerstetter et al. (1994) Plant Cell 6: 1877-1887

Kerstetter et al. (1997) Development 124: 3045-3054

Kim and ShefFrey (1990) J. Biol. Chem. 265: 13362-13369

Kim et al. (1996) Mol. Cell. Biol .16: 4003-4013

Kim et al. (2001) Plant J. 25: 247-259

Kim (2004) Plant Mol. Biol. 55: 883-904

Kimmel (1987) Methods Enzymol. 152: 507-511

Kirik et al. (2004a) Dev. Biol. 268: 506-513

Kirik et al. (2004b) Plant Mol. Biol. 55: 389-398

Kissinger et al. (1990) Cell 63: 579-590

Klee (1985) Bio/Technology 3: 637-642

Klein et al. (1987) Nature 327: 70-73

Knight (2000a) Int. Rev. Cytol. 195: 269-324.

Koornneef et al (1986) In Tomato Biotechnology: Alan R. Liss, Inc., 169-178

Krizek (1999) Dev. Genet. 25: 224-236

Ku et al. (2000) Proc. Natl. Acad Sci. USA 97: 9121-9126

Kunst et al. (2000) Biochem Soc. Trans. 28: 651-654.

Kusnetsov et al. (1999) J. Biol. Chem. 274: 36009-36014

Kwak et al. (2005) Science 307: 1111-1113

Kwong (2003) Plant Cell 15: 5-18

Kyozuka and Shimamoto (2002) Plant Cell Physiol. 43: 130-135

Lapik and Kaufman (2003) Plant Cell 15: 1578-1590

Larkin et al. (2003) Ann. Rev. Plant Biol. 54: 403-430

Lebel et al. (1998) Plant J. 16: 223-233

Ledent and Vervoort (2001) Genome Res. 11: 754-770

Lee and Schiefelbein (1999) Cell 99: 473-483

Lee et al. (2002) Genome Res. 12: 493-502

Lee and Schiefelbein (2002) Plant Cell 14: 611-618

Lee et al. (2003) Proc. Natl. Acad. Sci. U S A 100: 2152-2156.

Lee et al. (2004) Plant Mol. Biol. 55: 61-81.

Lefstin and Yamamoto (1998) Nature 392: 885-888

Leon-Kloosterziel et al. (1996) Plant Physiol. 110: 233-240

Levens (2003) Genes Dev. 17: 1071-1077

Li et al. (1992) Nucleic Acids Res. 20: 1087-1091

Li et al. (1998) EMBOJ. 17: 6300-6315

Lin et al. (1991) Nature 353: 569-571

Lincoln et al. (1990) Plant Cell 2: 1071-1080

Liscum and Reed (2002) Plant Mol. Biol.49: 387-400

Littlewood and Evan (1998) Helix-Loon-Helix Transcription Factors (New York: Oxford University Press)

Liu and Zhu (1997) Proc. Natl. Acad. Sci. USA 94: 14960-14964

Liu et al. (1999) Eur. J. Biochem. 262: 247-257

Livingston et al. (2004) Economic and policy implications of wind-borne entry of Asian soybean rust into the United States. http://www.ers.usda.gov/Features/SoyBeanRust/

Long et al. (1996) Nature 379: 66-69

Long and Barton (2000) Dev. Biol. 218: 341-353

Lorenzo et al. (2003) Plant Cell 15: 165-178

Lorenzo et al. (2004) Plant Cell 16: 1938-1950

Lotan et al. (1998) Cell 93: 1195-1205.

Loulergue et al. (1998) Gene 225: 47-57

Ludwig et al. (1989) Proc. Natl. Acad. Sci. USA 86: 7092-7096

Ludwig et al. (1990) Cell 62: 849-851

Luerssen et al. (1998) Plant J. 15: 755-764

Luger et al. (1997) Nature 389: 251-260

Lynch et al. (2002) Phytopathol. 92: S33.

Ma et al. (1994) Cell 77: 451-459

Mackay and Crossley (1998) Trends Biochem. Sci. 23: 1-4

Maity and de Crombrugghe (1998) Trends Biochem. Sci. 23: 174-178

Maleck (2000) Nat. Genet. 26: 403-410

Mandel (1992) Nature 360: 273-277

Mandel et al. (1992) Cell 71-133-143

Mantovani (1998) Nucleic Acids Res. 26: 1135-1143

Mantovani (1999) Gene 239: 15-27.

Mare et al. (2004) Plant Mol. Biol. 55: 399-416

Martin and Paz-Ares (1997) Trends Genet. 13: 67-73

Martinez-Garcia and Quail (1999) Plant J. 18: 173-183

Martinez-Garcia et al. (2000) Science 288: 859-863

Masiero et al. (2002) J. Biol. Chem. 277: 26429-26435

Massari and Murre (2000) Mol. Cell. Biol. 20: 429-440

Masucci J. et al. (1996) Development 122: 1253-1260

Mazon et al. (1982) Eur. J. Biochem. 127: 605-608

McCarty et al. (1989) Plant Cell 1: 523-532

McCarty et al. (1991) Cell 66: 895-905

McCue and Hanson (1990) Trends Biotechnol. 8: 358-362

McNabb et al. (1995) Genes Dev. 9: 47-58

McNabb et al. (1997) Mol. Cell. Biol. 17: 7008-7018

Meijer et al. (1996) Plant Mol. Biol. 31: 607-618

Meinke (1992) Science 258: 1647-1650

Meinke et al. (1994) Plant Cell 6: 1049-1064

Merlot et al. (2001) Plant J. 25: 295-303.

Mewes et al. (2002) Nucleic Acids Res. 30: 31-34

Meyers (1995) Molecular Biology and Biotechnology, Wiley VCH, New York, NY, p 856-853

Miki et al. (1993) in Methods in Plant Molecular Biology and Biotechnology, p. 67-88, Glick and Thompson, eds., CRC Press, Inc., Boca Raton

Miles et al. (2003) Soybean rust: is the U.S. soybean crop at risk? http://www.apsnet.org/online/feature/rust/

Miyoshi et al. (2003) Plant J. 36: 532-540

Mizukama and Fischer (2000) Proc. Natl. Acad. Sci. USA 97: 942-947

Mizukami (2001) Curr Opinion Plant Biol. 4: 533-539

Mohr and Cahill (2003) Functional Plant Biology 30: 461-469

Mount (2001), in Bioinformatics: Sequence and Genome Analysis, Cold Spring Harbor Laboratory Press, Cold Spring Harbor, New York, page 543

Müller et al. (2001) Plant J. 28: 169-179

Munkvold (2003) Annu. Rev. Phytopathol. 41: 99-116.

Murre et al. (1989) Cell 56: 777-783

Myers et al. (1986) Science 232: 613-618

Nair and Burley (2000) Nature 404: 715: 717-718

Nakshatri (1996) J. Biol. Chem. 271: 28784-28791.

Nambara et al. (1995) Development 121: 629-636

Nandi et al. (2000) Curr. Biol. 10: 215-218

Nath et al. (2003) Science 299: 1404-1407

Nesi et al. (2000) Plant Cell 12: 1863-1878

Ni et al. (1998) Cell 95: 657-667

Nieto-Sotelo and Quail (1994) Biochem. Soc. Symp. 60: 265-275

North Dakota State University Extension Service. (2002). Managing Row Crop Diseases in Drought Years. http://www.ag.ndsu.nodak.edu/drought/ds-10-97.htm

North Dakota State University Extension Service. (2004). Small Grain Diseases: Management of Those More Common and Severe in Dry Years. http://www.ag.ndsu.nodak.edu/drought/ds-01-02.htm

Novillo et al. (2004) Proc. Natl. Acad. Sci. USA 101: 3985-3990

Odell (1985) Nature 313: 810-812

Ohme-Takagi and Shinshi (1995) Plant Cell 7: 173-182

Ohta et al. (2001) Plant Cell 13: 1959-1968

Okamuro et al. (1997) Proc. Natl. Acad. Sci. USA 94: 7076-7081

Olesen and Guarente (1990) Genes Dev. 4: 1714-1729

Onate et al. (1994) Mol. Cell. Biol. 14: 3376-3391

Onate-Sanchez and Singh (2002) Plant Physiol. 128: 1313-1322

Ooms et al. (1993) Plant Physiol. 102: 1185-1191

Palatnik et al. (2003) Nature 425: 257-263

Parcy and Giraudat (1997) Plant J. 11: 693-702

Parcy et al. (1997) Plant Cell 9: 1265-1277
Park (2001) Plant Cell 13: 1035-1046.
Payne et al. (2000) Genetics 156: 1349-1362
Peng et al. (1997) Genes Development 11: 3194-3205)
Peng et al. (1999) Nature: 400: 256-261
Pinkham and Guarente (1985) Mol. Cell Biol. 5: 3410-3416.
Pnueli et al. (2002) Plant J. 31: 319-330
Porra et al. (1989) Biochim. Biophys. Acta 975: 384-394
Pourtau et al. (2004) Planta 219: 765-772
Putterill et al (1995) Cell 80: 847-857
Rajani and Sundaresan (2001) Curr. Biol. 11: 1914-1922
Ratcliffe et al. (2001) Plant Physiol. 126: 122-132
Reeves and Nissen (1990) J. Biol. Chem. 265: 8573-8582
Reeves (2001) Gene 277: 63-81.
Reeves and Beckerbauer (2001) Biochim Biophys Acta 1519: 13-29.
Reidt et al. (2000) Plant J. 21: 401-408
Remm et al. (2001) J. Mol. Biol. 314: 1041-1052
Reuber (1998) Plant J. 16: 473-485.
Riechmann and Meyerowitz (1998) Biol. Chem. 379: 633-646
Riechmann et al. (2000a) Science 290: 2105-2110
Riechmann and Ratcliffe (2000b) Curr. Opin. Plant Biol. 3: 423-434
Rieger et al. (1976) Glossary of Genetics and Cytogenetics: Classical and Molecular, 4th ed., Springer Verlag, Berlin
Rieping and Schoffl (1992) Mol. Gen. Genet. 231: 226-232
Rigaut et al. (1999) Nat. Biotechnol. 17: 1030-1032
Robatzek and Somssich (2002) Genes Dev. 16: 1139-1149
Robinson et al. (2000) Nucleic Acids Res. 28: 4460-4466
Robson et al. (2001) Plant J. 28: 619-631
Rohila et al. (2004) Plant J. 38: 172-181
Romier et al. (2003) J. Biol. Chem. 278: 1336-1345
Rushton et al. (1995) Plant Mol. Biol. 29: 691-702
Rushton et al. (1996) EMBO J. 15: 5690-5700
Sadowski et al. (1988) Nature 335: 563-564
Saijo et al. (2000) Plant J. 23: 319-327.
Sakuma et al. (2002) Biochem. Biophys. Res. Comm. 290: 998-1009
Saleki et al. (1993) Plant Physiol. 101: 839-845
Salsi et al. (2003) J. Biol. Chem. 278: 6642-6650
Sambrook et al. (1989) Molecular Cloning: A Laboratory Manual, 2nd Ed., Cold Spring Harbor Laboratory, Cold Spring Harbor, N.Y.
Sanchez and Cejudo (2003) Plant Physiol. 132: 949-957
Sanders et al. (1999) Plant Cell 11: 691-706
Sanford et al. (1987) Part. Sci. Technol. 5:27-37
Sanford (1993) Methods Enzymol. 217: 483-509
Schaffer et al. (1998) Cell 93: 1219-1229
Schellmann et al. (2002) EMBO J. 21: 5036-5046
Schindler et al. (1993) Plant J. 4: 137-150
Schnittger et al. (1998) Development 125: 2283-2289
Schnittger et al. (1999) Plant Cell 11: 1105-1116
Schoof et al. (2000) Cell 100: 635-644
Sessa et al. (1994) Molecular genetic analysis of plant development and metabolism. (Berlin: Springer Verlag).
Sharp and LeNoble (2002) J. Exp. Bot. 53: 33-37.
Sheen (1999) Ann. Rev. Plant Physiol. Plant Mol. Biol. 50: 187-217
Shimizu et al. (1997) EMBO J. 16: 4689-4697
Shin et al. (2002) Mol Plant Microbe Interact 15: 983-989.
Shirakata et al. (1993) Genes Dev. 7: 2456-2470
Shpaer (1997) Methods Mol. Biol. 70: 173-187
Silver et al. (2003) Mol. Cell. Biol. 23: 5989-5999
Sinha et al. (1996) Mol. Cell. Biol. 16: 328-337
Sivamani et al. (2000) Plant Science 155: 1-9

Smalle et al (1998) Proc. Natl. Acad. Sci. USA. 95:3318-3322
Smeekens (1998) Curr. Opin. Plant Biol. 1: 230-234
Smith et al. (1992) Protein Engineering 5: 35-51
Smolen et al. (2002) Genetics 161: 1235-1246
Soltis et al. (1997) Ann. Missouri Bot. Gard. 84: 1-49
Solano et al. (1998) Genes Dev. 12: 3703-3714
Sonnhammer et al. (1997) Proteins 28: 405-420
Sorensen et al. (2003) Plant J. 33: 413-423
Spencer et al. (1994) Plant Mol. Biol. 24: 51-61
Spollen et al. (2000) Plant Physiol. 122: 967-976.
Stockinger et al. (1997) Proc. Natl. Acad. Sci. USA 94: 1035-1040
Stone et al. (2001) Proc. Natl. Acad. Sci. USA 98: 11806-11811
Surpin et al. (2002) Plant Cell 14 Suppl: S327-S338
Suzuki et al. (1997) Plant Cell 9: 799-807
Suzuki et al. (2001) Plant J. 28: 409-418
Suzuki et al. (2003) Plant Physiol. 132: 1664-1677
Svensson et al. (2003) Arch. Biochem. Biophys. 414: 180-188
Tahtiharju and Palva (2001) Plant J 26: 461-470.
Tamminen et al. (2001) Plant J. 25: 1-8
Tanimoto et al. (1995) Plant J. 8: 943-948
Tasanen et al. (1992) J. Biol. Chem. 267: 11513-11519
Taylor and Scheuring (1994) Mol. Gen. Genet. 243: 148-157
Tepperman et al. (2001) Proc. Natl. Acad. Sci. USA 98: 9437-9442
Thaler and Bostock (2003) Ecology 85: 48-58.
Thoma (1994) Plant Physiol. 105: 35-45
Thompson et al. (1994) Nucleic Acids Res. 22: 4673-4680
Tiwari et al. (2001) Plant Cell 13: 2809-2822
Tiwari et al. (2003) Plant Cell 15: 533-543
Toledo-Ortiz et al. (2003) Plant Cell 15: 1749-1770
Tournier et al. (2003) FEBS Lett. 550: 149-154
Toyama et al. (1999) Plant Cell Physiol. 40: 1087-1092
Truemit and Sauer (1995) Planta 196: 564-570
Tudge (2000) in The Variety of Life, Oxford University Press, New York, NY pp. 547-606
Ulmasov et al. (1997) Science 276: 1865-1868
Vasil et al. (1992) Bio/Technol. 10:667-674
Vasil et al. (1993) Bio/Technol. 11:1553-1558
Vasil (1994) Plant Mol. Biol. 25: 925-937
Verslues and Sharp (1999) Plant Physiol. 119: 1349-1360
Vicient et al. (2000) J. Exp. Bot. 51: 995-1003
Vollbrecht et al. (1991) Nature 350: 241-243
Wada et al. (1997) Science 277: 1113-1116
Wada et. al. (2002) Development 129: 5409-5419
Wahl and Berger (1987) Methods Enzymol. 152: 399-407
Wan and Lemeaux (1994) Plant Physiol. 104: 37-48
Wang et al. (1997) Plant Cell 9: 491-507
Wang (1998) Plant J. 16: 515-522
Wanner and Gruissem (1991) Plant Cell 3: 1289-1303
Waterhouse et al. (2001) Trends Plant Sci. 6: 297-301
Waterston et al. (2002) Nature 420: 520-562
Weeks et al. (1993) Plant Physiol. 102:1077-1084
Weigel et al. (1992) Cell 69: 843-859
Weigel (1995) Plant Cell 7: 388-389
Weigel et al. (2000) Plant Physiol. 122: 1003-1013
Weigel and Nilsson (1995) Nature 377: 482-500
Weissbach and Weissbach (1989) Methods for Plant Molecular Biology, Academic Press
Wendler et al. (1997) J. Biol. Chem. 272: 8482-8489
Wesley et al. (2001) Plant J. 27: 581-590
West et al. (1994) Plant Cell 6: 1731-1745

Westhoff and Gowik (2004) Ann. Bot. (London) 93: 13-23

Windhovel (2001) Plant Mol. Biol. 45: 201-214.

Wobus and Weber (1999) Curr. Opin. Plant Biol. 2: 33-38

Wolberger et al. (1991) Cell 67: 517-528

Wrather and Sweets (2004) Aflatoxin in Corn. http://aes.missouri.edu/delta/croppest/aflacorn.stm

Wu et al. (1996) Plant Cell 8: 617-627

Xin and Browse (1998) Proc. Natl. Acad Sci. USA 95: 7799-7804

Xing et al. (1993) EMBO J. 12: 4647-4655

Xiong et al. (2001a) Genes Dev. 15: 1971-1984.

Xiong and Zhu (2002) Plant Cell Environ. 25: 131-139.

Xiong and Yang (2003) Plant Cell 15: 745-759.

Xu et al. (1996) Plant Physiol. 110: 249-257

Xu et al. (2001) Proc. Natl. Acad Sci USA 98: 15089-15094

Yamada et al. (1999a) FEBS Lett. 460: 41-45

Yamada et al. (1999b) Biochem. Biophys. Res. Commun. 261: 614-621

Yamada et al. (2003) Biochem J. 373: 167-178

Yamaguchi-Shinozaki and Shinozaki (1993) Mol. Gen. Genet. 236: 331-340

Yamasaki et al. (2005) Plant Cell 17: 944-956

Yang et al. (1999) Plant J. 18: 141-149

Yi et al. (2004) Plant Physiol. 136: 2862-2874

Yu et al. (2001) Plant Cell 13: 1527-1540

Yun et al. (2003) J. Biol. Chem. 278: 36966-36972

Zhang et al. (1991) Bio/Technology 9: 996-997

Zhang et al. (2002) Planta 215:: 191-194

Zhang et al. (2003) Development 130: 4859-4869

Zhang and Wang (2005) BMC Evol. Biol. 5: 1

Zhong and Ye (2001) Plant Phys. 126: 549-563

Zhou et al. (1995a) Nature 376: 771-774

Zhou et al. (1995b) Cell 83: 925-935

Zhou et al. (1997) EMBO J. 16: 3207-3218

Zhou and Lee (1998) J. Natl. Cancer Inst. 90: 381-388

Zhu et al. (1998) Plant Cell 10: 1181-1191

Zou et al. (2004) J. Biol. Chem. 279: 55770-5577

**Claims**

1. Use of a nucleotide sequence which encodes a polypeptide comprising the sequence

```
MAEAPASPGGGGGSHESGSPRGGGGGGGSVREQDRFLPIANISRIMKKAIPANG
KIAKDAKETVQECVSEFISFITSEASDKCQREKRKTINGDDLLWAMATLGFED
YIEPLKVYLQKYREMEGDSKLTAKSSDGSIKKDALGHVGASSSAAQGMGQQGA
YNQGMGYMQPQYHNGDISN >
```

for producing a transgenic plant wherein over-expression of said polypeptide provides the trait of increased tolerance to cold; wherein said tolerance to cold is tolerance to chilling at 8°C during germination.

2. Use of any claim 1 wherein said nucleotide sequence which encodes a polypeptide is operably linked to a constitutive, inducible or tissue-active promoter.

3. Use of claim 1 or 2 wherein said plant is a an angiosperm.

4. A method for producing transgenic plant having the trait of increased tolerance to cold during germination, said method comprising:

producing a transgenic plant having an expression vector comprising a nucleotide sequence which encodes a polypeptide comprising the sequence

```
MAEAPASPGGGGGSHESGSPRGGGGGGSVREQDRFLPIANISRIMKKAIPANG
KIAKDAKETVQECVSEFISFITSEASDKCQREKRKTINGDDLLWAMATLGFED
YIEPLKVYLQKYREMEGDSKLTAKSSDGSIKKDALGHVGASSSAAQGMGQQGA
YNQGMGYMQPQYHNGDISN >
```

wherein over-expression of said polypeptide provides the trait of increased tolerance to cold, wherein said tolerance to cold is tolerance to chilling at 8°C during germination; and

selecting a plant having said trait of increased cold tolerance caused by over-expression of said polypeptide.

5. The method of claim 4 wherein said nucleotide sequence which encodes a polypeptide is operably linked to a constitutive, inducible or tissue-active promoter.

6. The method of claim 4 or 5 wherein said plant is an angiosperm.


**Patentansprüche**

1. Verwendung einer Nucleotidsequenz, die für ein Polypeptid kodiert, welches die Sequenz:

MAEAPASPGGGGGSHESGSPRGGGGGGSVREQDRFLPIANISRIMKKAIPANGKIA

KDAKETVQECVSEFISFITSEASDKCQREKRKTINGDDLLWAMATLGFEDYIEPLKVY

LQKYREMEGDSKLTAKSSDGSIKKDALGHVGASSSAAQGMGQQGAYNQGMGYM

QPQYHNGDISN

umfasst, zur Herstellung einer transgenen Pflanze, worin Überexpression des Polypeptids das Merkmal erhöhter Toleranz gegenüber Kälte hervorbringt; worin die Toleranz gegenüber Kälte Toleranz gegenüber Kühlung bei 8 °C während der Keimbildung ist.

2. Verwendung nach Anspruch 1, worin die Nucleotidsequenz, die für das Polypeptid kodiert, operabel an einen konstitutiven, induzierbaren oder gewebeaktiven Promotor gebunden ist.

3. Verwendung nach Anspruch 1 oder Anspruch 2, worin die Pflanze eine Angiosperme ist.

4. Verfahren zur Herstellung einer transgenen Pflanze mit dem Merkmal erhöhter Toleranz gegenüber Kälte während der Keimbildung, wobei das Verfahren Folgendes umfasst:

das Herstellen einer transgenen Pflanze mit einem Expressionsvektor, der eine Nucleotidsequenz umfasst, die für ein Polypeptid kodiert, welches die Sequenz:

MAEAPASPGGGGGSHESGSPRGGGGGGSVREQDRFLPIANISRIMKKAIPANGKIA

KDAKETVQECVSEFISFITSEASDKCQREKRKTINGDDLLWAMATLGFEDYIEPLKVY

LQKYREMEGDSKLTAKSSDGSIKKDALGHVGASSSAAQGMGQQGAYNQGMGYM

QPQYHNGDISN

umfasst, worin Überexpression des Polypeptids das Merkmal erhöhter Toleranz gegenüber Kälte hervorbringt, worin die Toleranz gegenüber Kälte Toleranz gegenüber Kühlung bei 8 °C während der Keimbildung ist; und das Auswählen einer Pflanze mit dem Merkmal erhöhter Kältetoleranz, die durch Überexpression des Polypeptids verursacht wurde.

5. Verfahren nach Anspruch 4, worin die Nucleotidsequenz, die für das Polypeptid kodiert, operabel an einen konstitutiven, induzierbaren oder gewebeaktiven Promotor gebunden ist.

6. Verfahren nach Anspruch 4 oder 5, worin die Pflanze eine Angiosperme ist.


**Revendications**

1. Utilisation d'une séquence de nucléotides qui code pour un polypeptide comprenant la séquence

```
MAEAPASPGGGGGSHESGSPRGGGGGGSVREQDRFLPIANISRIMKKAIPANG
KIAKDAKETVQECVSEFISFITSEASDKCQREKRKTINGDDLLWAMATLGFED
YIEPLKVYLQKYREMEGDSKLTAKSSDGSIKKDALGHVGASSSAAQGMGQQGA
YNQGMGYMQPQYHNGDISN
```

pour produire une plante transgénique, où la surexpression dudit polypeptide fournit le trait d'une plus grande tolérance au froid; où ladite tolérance au froid est une tolérance à la réfrigération à 8°C durant la germination.

2. Utilisation selon la revendication 1, où ladite séquence de nucléotides qui code pour un polypeptide est fonctionnellement liée à un promoteur constitutif, inductible ou tissu-actif.

3. Utilisation selon la revendication 1 ou 2, où ladite plante est un angiosperme.

4. Méthode de production d'une plante transgénique ayant le trait d'une plus grande tolérance au froid durant la germination, ladite méthode comprenant:

produire une plante transgénique ayant un vecteur d'expression comprenant une séquence de nucléotides qui code pour un polypeptide comprenant la séquence

```
MAEAPASPGGGGGSHESGSPRGGGGGGSVREQDRFLPIANISRIMKKAIPANG
KIAKDAKETVQECVSEFISFITSEASDKCQREKRKTINGDDLLWAMATLGFED
YIEPLKVYLQKYREMEGDSKLTAKSSDGSIKKDALGHVGASSSAAQGMGQQGA
YNQGMGYMQPQYHNGDISN,
```

où la sur-expression dudit polypeptide fournit le trait d'une plus grande tolérance au froid, où ladite tolérance au froid est une tolérance à la réfrigération à 8°C durant la germination; et
sélectionner une plante ayant ledit trait d'une plus grande tolérance au froid provoqué par la surexpression dudit polypeptide.

5. Méthode selon la revendication 4, où ladite séquence de nucléotides qui code pour un polypeptide est fonctionnellement liée à un promoteur constitutif, inductible ou tissu-actif.

6. Méthode selon la revendication 4 ou 5, où ladite plante est un angiosperme.

Fig. 1

Fig. 2

G1364      --MAESQAKSP----GGCGSHESGGDQSPR----
G2345      --MAESQTGG----GGGGSHESGGDQSPR----
G3470      --MSDAPASP-------SHESGGEQSPRGSL----
G3471      --MSDAPPSP------THESGGEQSPRGSS----
G481       --MADTPSSP---AGD-GGE-----S----
G3876      --MAEAPASPG--GGGGSHESGSPRGGGGGG----
G3434      --MADD-----GGSHEGSG---GGGG----
G3396      --MSEGFDGTE------NGGGGGGGVG----
G3397      ---MPDS-------DNDSGGPSNY----
G3435      ---MPDS-------DNDSGGPSN----
G3398      ---MPDS-------DNESGGPSN----
G3436      ---MPDS-------DNESGGPSNA----
G482       ---MGDS-------DRDSGGQNGNNQ----
G485       ---MADS-------DNDSGGHKDGGN----
G3472      ---MAES-------DNESGGHTGNASGSN----
G3474      ---MAES-------DNESGGHTGNASGSN----
G3475      ---MADS-------DNDSGGAHNAGKG----
G3478      ---MADS-------DNDSGGAHNGGKG----
G3476      ---MAES-------DNDSGGAQNAGNSGNL----
G1820      ---MAENNNNNNGDNMNNDNHQQPPSYSQLPPMASSNPQLR
G1836      ---ME---NNNGNN-----QLPPKG--NEQLK
G489       MDQQDHGQSGAMNYGTNPYQTNPMSTTAATVAGGAAQPGQLAFHQIHQQQQQLAQQLQ
G3429      ---MILIVPLHLFWMSGGRNMDQVKKAAVRSD----

Fig. 3A

```
G1364   -----------SLHVREQDRFLPIANISRIMKRGLPAN-------GKIAKDAKEIVQECVS
G2345   -----------SLNVREQDRFLPIANISRIMKRGLPLN-------GKIAKDAKETMQECVS
G3470   -----------SGAAREQDRYLPIANISRIMKKALPPN-------GKIAKDAKDTMQECVS
G3471   -----------SG-AREQDRYLPIANISRIMKKALPPN-------GKIAKDAKDTMQECVS
G481    -----------GGSVREQDRYLPIANISRIMKKALPPN-------GKIGKDAKDTVQECVS
G3876   -------------SVREQDRFLPIANISRIMKKAIPAN-------GKIAKDAKETVQECVS
G3434   --------------VREQDRFLPIANISRIMKKAVPAN-------GKIAKDAKETLQECVS
G3396   ---------------KEQDRFLPIANIGRIMRRAVPEN-------GKIAKDSKESVQECVS
G3397   ------AGGELSSPREQDRFLPIANVSRIMKKALPAN-------AKISKDAKETVQECVS
G3435   ------AGGELSSPREQDRFLPIANVSRIMKKALPAN-------AKISKDAKETVQECVS
G3398   -------AGEYASAREQDRFLPIANVSRIMKRALPAN-------AKISKDAKETVQECVS
G3436   ---------EFSSPREQDRFLPIANVSRIMKKALPAN-------AKISKDAKETVQECVS
G482    ------NGQSSLSPREQDRFLPIANVSRIMKKALPAN-------AKISKDAKETMQECVS
G485    -----------ASTREQDRFLPIANVSRIMKKALPAN-------AKISKDAKETVQECVS
G3472   ---------EFSGCREQDRFLPIANVSRIMKKALPAN-------AKISKEAKETVQECVS
G3474   ---------ELSGCREQDRFLPIANVSRIMKKALPAN-------AKISKEAKETVQECVS
G3475   ---------SEMS-PREQDRFLPIANVSRIMKKALPAN-------AKISKDAKETVQECVS
G3478   ---------SEMS-PREQDRFLPIANVSRIMKKALPAN-------AKISKDAKETVQECVS
G3476   ---------SELS-PREQDRFLPIANVSRIMKKALPAN-------AKISKDAKETVQECVS
G1820   NYWI---EQMETVSDFKNRQLPLARIKKIMKADPDVH-------MVSAEAPIIFAKACE
G1836   SFWS---KEMEGNLDFKNHDLPITRIKKIMKYDPDVT-------MIASEAPILLSKACE
G489    AFWENQFKEIEKTTDFKKHSLPLARIKKIMKADEDVR-------MISAEAPVVFARACE
G3429   -----------GVGGSATNAELPMANLVRLIKKVLPGK-------AKIGGAAKGLTHDCAV
```

Fig. 3B

EP 1 928 226 B1

```
G1364   EFISFVT-SEASDKCQREKRKTINGDDLLWAMATLGFEDYMEPLKVYLMRYREMEGDTKG
G2345   EFISFVT-SEASDKCQREKRKTINGDDLLWAMATLGFEDYIDPLKVYLMRYREMEGDTKG
G3470   EFISFIT-SEASEKCQKEKRKTINGDDLLWAMATLGFEDYIEPLKVYLARYREAEGDTKG
G3471   EFISFIT-SEASEKCQKEKRKTINGDDLLWAMATLGFEDYIEPLKVYLARYREAEGDTKG
G481    EFISFIT-SEASDKCQKEKRKTVNGDDLLWAMATLGFEDYLEPLKIYLARYRELEGDNKG
G3876   EFISFIT-SEASDKCQREKRKTINGDDLLWAMATLGFEDYIEPLKVYLQKYREMEGDSKL
G3434   EFISFVT-SEASDKCQKEKRKTINGDDLLWAMATLGFEEYVEPLKIYLQKYKEMEGDSKL
G3396   EFISFIT-SEASDKCLKEKRKTINGDDLIWSMGTLGFEDYVEPLKLYLRLYRETEGDTKG
G3397   EFISFIT-GEASDKCQREKRKTINGDDLLWAMTTLGFEDYVDPLKHYLHKFREIEGERAA
G3435   EFISFIT-GEASDKCQREKRKTINGDDLLWAMTTLGFEDYVEPLKHYLHKFREIEGERAA
G3398   EFISFIT-GEASDKCQREKRKTINGDDLLWAMTTLGFEDYIDPLKLYLHKFRELEGEKAI
G3436   EFISFIT-GEASDKCQREKRKTINGDDLLWAMTTLGFEDYVEPLKLYLHKFRELEGEKAA
G482    EFISFVT-GEASDKCQKEKRKTINGDDLLWAMTTLGFEDYVEPLKVYLQRFREIEGERTG
G485    EFISFIT-GEASDKCQREKRKTINGDDLLWAMTTLGFEDYVEPLKVYLQKYREVEGEKTT
G3472   EFISFIT-GEASDKCQKEKRKTINGDDLLWAMTTLGFEEYVEPLKVYLHKYRELEGEKTA
G3474   EFISFIT-GEASDKCQKEKRKTINGDDLLWAMTTLGFEDYVDPLKIYLHKYREMEGEKTA
G3475   EFISFIT-GEASDKCQREKRKTINGDDLLWAMTTLGFEDYVEPLKGYLQRFREMEGEKTV
G3478   EFISFIT-GEASDKCQREKRKTINGDDLLWAMTTLGFEDYVEPLKGYLQRFREMEGEKTV
G3476   EFISFIT-GEASDKCQREKRKTINGDDLLWAMTTLGFEEYVEPLKIYLQRFREMEGEKTV
G1820   MFIVDLT-MRSWLKAEENKRHTLQKSDISNAVASSFTYDFLLD---VVPKDES--IATAD
G1836   MFIMDLT-MRSWLHAQESKRVTLQKSNVDAAVAQTVIFDFLLDDDIEVKRESV--AAAAD
G489    MFILELT-LRSWNHTEENKRRTLQKNDIAAAVTRTDIFDFLVD---IVPREDLRDEVLGS
G3429   EFVGFVG-DEASEKAKAEHRRTVAPEDYLGSFGDLGFDRYVDPMDAYIHGYREFERAGGN
```

Fig. 3C

```
G1364   SAKGGDP-------------NAKKDG--------------QSSQNGQFSQLAHQG------PY
G2345   SGKGGES------------SAKRDG-------------QPSQVSQFSQVPQQGSFSQ-GPY
G3470   SARSGDG----------SARPD--------------QVGLAGQNAQLVHQGSLNYIGLQ
G3471   SARSGDG-----------SATPD--------------QVGLAGQNSQLVHQGSLNYIGLQ
G481    SGKSGDG-----------SNRDA--------------GGGVSGEE---------------
G3876   TAKSSDG-----------SIKKDA-------------LGHVGA---SSSAAQGMGQQGAY
G3434   STKAGEG-----------SVKKDA-------------ISPHGG---TSSSSNQLVQHGVY
G3396   S-RASEL-----------PVKKDV-------------VLNGDP---GSSFEGM-------
G3397   ASTTGAGTSAAST----TPPQQQH-------------TANAAGGYAGYAAPGAGP--GGM
G3435   ASAGASGSQQQQQ----QGELPRG-------------AANAAG-YAGYGAPGSG---GMM
G3398   GAAGSGGGGAASSGGSGSGSGSHH-------------HQDASRNNGGYGMYGGG---GGM
G3436   TTSASSGPQPPLHR--ETTPSSST-------------HNGAGGPVGGYGMYGGAGGGSGM
G482    LGRPQTG-----------GEVGEH-------------QRDAVGDGGGFYGGGGG-----M
G485    TAGRQGD-----------KEGGGG-------------GGGAGSGSGGAPMYGGG-----M
G3472   MMG---------------RPHERD-------------EGYGH----ATPM--------M
G3474   MMG---------------RPHERD-------------EGYGHGHGHATPM--------M
G3475   AAR---------------DKDAPP-------------PTNATN---SAYE--------S
G3478   AAR---------------DKDAPP-------------LTNATN---SAYE--------S
G3476   AAR---------------DSSK---------------DSASA---SSY----------
G1820   PGFVAMPHP--------DGGGVPQYYYP------PGVVMGTPMVGS-GMYAPS---QAW
G1836   P--VAMPPI--------DDGELP-----------PGMVIGTPVCCSLGIHQPQPQMQAW
G489    IPRGTVPEA--------AAAGYPYGY---------LPAGTAPIGNPGMVMGNPGGAYP
G3429   RRVAPPP----------PAAATP-----------LTPGGPTFTDAELQFLRSVIPSR
```

Fig. 3D

```
G1364    GNSQ---------------------------AQQHMMVPMPGTD-----------------------
G2345    GNSQSLRFGN--------------------SIEHLEVLMSSTRTLFITIFRDSTMPVVSENL
G3470    VQPQH-----------------------------LVMPSMQGHE-----------------------
G3471    VQPQH-----------------------------LVMPSMQSHE-----------------------
G481     ---------------------------------MPSW-----------------------
G3876    NQGMG-----------------------------YMQPQYHNGDISN----------------
G3434    NQGMG-----------------------------YMQPQYHNGET-----------------
G3396    ---------------------------------------
G3397    MMMMGQPMYG--------SPPPPPQQQQQ-QHHHMAMGGRG-GFGHHPG-----GGGGGS
G3435    MMMMGQPMYGGSQPQQQPPPPQPPQQQQQHQQHHMAIGGRG-GFGQQ-------GGGGGS
G3398    IMMMGQPMYGSPPASSAGYAQPPP---PHHHHQMVMGGKG-AYGHG-G-----GGGGGP
G3436    IMMMGQPMYG--------GSPPAASSGSYPHHQMAMGGKGGAYGYG---------GGSS
G482     QYHQHH-----------------------QFLHQQNHMYGATGGGSDS---------GGGA
G485     VTTMGH-----------------------QFSHHFS-----------------------
G3472    -IMMGHQ-------------------------QQQHQGHVYGSGTTTGSASS-----------
G3474    TMMMGHQPQH---------------------QHQHQHQGHVYGS----GSASS-----------
G3475    PSYAAA---P-------------------GGIMMHQGHVYGSAGFHQVAGGAIKGGPVYPGP
G3478    ANYAAAAAVP-----------------GGIMMHQGHVYGSAGFHQVAGGAIKGGPAYPGP
G3476    ---------------------------------HQGHVYGSPAYHHQVP-----GPTYPAP
G1820    P---AAA-----------------------GDGEDDAEDNGGNGGGN-------------
G1836    PGAWTSV-----------------------SGEEEEARGKKGGDDGN-------------
G489     PNPYMGQ-----------------------PMWQQQAPDQPDQEN---------------
G3429    SDDEYS------------------------GSSPAIGGYGYGYGYGKNM----------
```

Fig. 3E

```
G1364    -----------------------------
G2345    SDPLSIDMDCEAIYHHFIGLLILSCK
G3470    ---------------------------------
G3471    ------------------------------
G481     ------------------------------
G3876    ------------------------------
G3434    -----------------------------
G3396    ------------------------------
G3397    SSSSGHGRQNRGA----------------
G3435    SSSSGLGRQDRA-----------------
G3398    SPSSGYGRQDRL----------------
G3436    SSPSGLGR--------------------
G482     AS--GRTRT-------------------
G485     ------------------------------
G3472    ----ARTR--------------------
G3474    ----ARTR--------------------
G3475    GSNAGRPR--------------------
G3478    GSNAGRPR--------------------
G3476    G----RPR--------------------
G1820    -----------------------------
G1836    -----------------------------
G489     -----------------------------
G3429    ---------------------------------
```

Fig. 3F

EP 1 928 226 B1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 2004019927 A
- WO 0177311 A
- WO 2004076638 A
- WO 2005047516 A
- US 6664446 B
- US 20040045049 A
- US 20040098764 A
- US 6717034 B
- US 20010010913 A
- US 5563055 A, Townsend and Thomas
- US 5015580 A, Christou
- US 5322783 A, Tomes
- US 6613962 B
- US 5591616 A
- WO 0216655 A, Harper

## Non-patent literature cited in the description

- **Abe et al.** *Plant Cell,* 1997, vol. 9, 1859-1868
- **Abe et al.** *Plant Cell,* 2003, vol. 15, 63-78
- **Affolter et al.** *Curr. Opin. Cell. Biol.,* 1990, vol. 2, 485-495
- **Agrios, G.N.** Plant Pathology. Academic Press, 1997
- **Aldemita ; Hodges.** *Planta,* 1996, vol. 199, 612-617
- **Alia et al.** *Plant J.,* 1998, vol. 16, 155-161
- **Allen.** *EMBO J.,* 1998, vol. 17, 5484-5496
- **Altschul.** *J Mol. Biol.,* 1990, vol. 215, 403-410
- **Altschul.** *J. Mol. Evol.,* 1993, vol. 36, 290-300
- Quantitative Filter Hybridisation. **Anderson ; Young.** Nucleic Acid Hybridisation, A Practical Approach. IRL Press, 1985, 73-111
- **Anderson et al.** *Plant Cell,* 2004, vol. 16, 3460-3479
- **Aravind ; Landsman.** *Nucleic Acids Res.,* 1998, vol. 26, 4413-4421
- **Arents ; Moudrianakis.** *Proc. Natl. Acad. Sci. USA,* 1995, vol. 92, 11170-11174
- **Atchley ; Fitch.** *Proc. Natl. Acad. Sci. USA,* 1997, vol. 94, 5172-5176
- **Atchley et al.** *J. Mol. Evol.,* 1999, vol. 48, 501-516
- **Ausubel et al.** Short Protocols in Molecular Biology. John Wiley & Sons, 1997
- **Bailey et al.** *Plant Cell,* 2003, vol. 15, 2497-2502
- **Bairoch et al.** *Nucleic Acids Res.,* 1997, vol. 25, 217-221
- **Bänzinger et al.** *Breeding for drought and nitrogen stress tolerance in maize. From theory to practice,* 2000
- **Barthelemy et al.** *Biochem. Biophys. Res. Commun.,* 1996, vol. 224, 870-876
- **Bates et al.** *Plant Soil,* 1973, vol. 39, 205-207
- **Baudino ; Cleveland.** *Mol. Cell. Biol.,* 2001, vol. 21, 691-702
- **Bechtold ; Pelletier.** *Methods Mol. Biol.,* 1998, vol. 82, 259-266
- Guide to Molecular Cloning Techniques. **Berger ; Kimmel.** Methods in Enzymology. Academic Press, Inc, 1987, vol. 152
- **Berger et al.** *Curr. Biol.,* 1998, vol. 8, 421-430
- **Berrocal-Lobo et al.** *Plant J.,* 2002, vol. 29, 23-32
- **Berrocal-Lobo ; Molina.** *Mol. Plant Microbe Interact.,* 2004, vol. 17, 763-770
- **Bevan.** *Nucleic Acids Res.,* 1984, vol. 12, 8711-8721
- **Bezhani et al.** *J. Biol. Chem.,* 2001, vol. 276, 23785-23789
- **Bhattacharjee et al.** *Proc. Natl. Acad. Sci. USA,* 2001, vol. 98, 13790-13795
- **Bi et al.** *J. Biol. Chem.,* 1997, vol. 272, 26562-26572
- **Bianchi ; Beltrame.** *EMBO Rep.,* 2000, vol. 1, 109-114
- **Boter.** *Genes Dev.,* 2004, vol. 18, 1577-1591
- **Borevitz et al.** *Plant Cell,* 2000, vol. 12, 2383-2393
- **Boss ; Thomas.** *Nature,* 2002, vol. 416, 847-850
- **Boyer.** *Annu. Rev. Phytopathol.,* 1995, vol. 33, 251-274
- **Brown et al.** *Plant Physiol.,* 2003, vol. 132, 1020-1032
- **Brownlie et al.** *Structure,* 1997, vol. 5, 509-520
- **Bruce et al.** *Plant Cell,* 2000, vol. 12, 65-79
- **Bucher ; Trifonov.** *J. Biomol. Struct. Dyn.,* 1988, vol. 5, 1231-1236
- **Bucher.** *J. Mol. Biol.,* 1990, vol. 212, 563-578

- **Buck ; Atchley.** *J. Mol. Evol.,* 2003, vol. 56, 742-750
- **Burglin.** *Nucleic Acids Res.,* 1997, vol. 25, 4173-4180
- **Burglin.** *Dev. Genes Evol.,* 1998, vol. 208, 113-116
- **Byrne.** *Nature,* vol. 408, 967-971
- **Caretti et al.** *J. Biol. Chem.,* 2003, vol. 278, 30435-30440
- **Carre ; Kay.** *Plant Cell,* 1995, vol. 7, 2039-2051
- **Carroll.** *Cell,* 2000, vol. 101, 577-580
- **Carson et al.** *Plant J.,* vol. 12, 1231-1240
- **Cassas et al.** *Proc. Natl. Acad. Sci. USA,* 1993, vol. 90, 11212-11216
- **Chae et al.** *Oncogene,* vol. 23, 4084-4088
- **Chakravarthy et al.** *Plant Cell,* 2003, vol. 15, 3033-3050
- **Chang ; Liu.** *J. Biol. Chem.,* 1994, vol. 269, 17893-17898
- **Chase et al.** *Ann. Missouri Bot. Gard.,* 1993, vol. 80, 528-580
- **Chen et al.** *Plant Cell,* 2002, vol. 14, 559-574
- **Chen ; Chen.** *Plant Physiol.,* 2002, vol. 129, 706-716
- **Cheong et al.** *Plant Physiol.,* 2002, vol. 129, 661-677
- **Cheong et al.** *Plant Physiol.,* 2003, vol. 132, 1961-1972
- **Chini et al.** *Plant J.,* 2004, vol. 38, 810-822
- **Chinnusamy et al.** *Genes Dev.,* vol. 17, 1043-1054
- **Chinthapalli et al.** Reviews in Plant Biochemistry and Biotechnology. 2002, 143-159
- **Christou et al.** *Proc. Natl. Acad. Sci. USA,* 1987, vol. 84, 3962-3966
- **Christou.** *Bio/Technol.,* 1991, vol. 9, 957-962
- **Christou et al.** *Plant. J.,* 1992, vol. 2, 275-281
- **Ciarapica et al.** *J. Biol. Chem.,* 2003, vol. 278, 12182-12190
- **Costa ; Dolan.** *Development,* 2003, vol. 130, 2893-2901
- **Coupland.** *Nature,* 1995, vol. 377, 482-483
- **Coustry et al.** *J. Biol. Chem.,* 1995, vol. 270, 468-475
- **Coustry et al.** *J. Biol. Chem.,* 1996, vol. 271, 14485-14491
- **Coustry et al.** *Biochem J.,* 1998, vol. 331, 291-297
- **Coustry et al.** *J. Biol. Chem.,* 2001, vol. 276, 40621-40630
- **Crawford et al.** *Plant Physiol.,* 2004, vol. 135, 244-253
- **Crozatier et al.** *Curr. Biol.,* 1996, vol. 6, 707-718
- **Cubas et al.** *Plant J.,* 1999, vol. 18, 215-222
- **Currie.** *J. Biol. Chem.,* 1997, vol. 272, 30880-30888
- **Daly et al.** *Plant Physiol.,* 2001, vol. 127, 1328-1333
- **Dang et al.** *Proc. Natl. Acad. Sci. USA,* 1992, vol. 89, 599-602
- **Dang et al.** *J. Bacteriol.,* 1996, vol. 178, 1842-1849
- **de Pater et al.** *Nucleic Acids Res.,* 1996, vol. 24, 4624-4631
- **Dellagi et al.** *Mol. Plant Microbe Interact.,* 2000, vol. 13, 1092-110
- **Deshayes et al.** *EMBOJ.,* 1985, vol. 4, 2731-2737
- **D'Halluin et al.** *Plant Cell,* 1992, vol. 4, 1495-1505
- **Di Cristina et al.** *Plant J.,* 1996, vol. 10, 393-402
- **Doebley ; Lukens.** *Plant Cell,* 1998, vol. 10, 1075-1082
- **Donn et al.** *Abstracts of VIIth International Congress on Plant Cell and Tissue Culture IAPTC,* 1990, vol. A2-38, 53
- Computer Methods for Macromolecular Sequence Analysis. Methods in Enzymology. Academic Press, Inc, 1996, vol. 266
- **Draper et al.** *Plant Cell Physiol.,* 1982, vol. 23, 451-458
- **Du ; Chen.** *Plant J.,* 2000, vol. 24, 837-847
- Guidebook to the homeobox genesOxford. University Press, 1994
- **Duckett et al.** *Development,* 1994, vol. 120, 3247-3255
- **Ecker.** *Science,* 1995, vol. 268, 667-675
- **Eddy.** *Curr. Opin. Str. Biol.,* 1996, vol. 6, 361-365
- **Edwards et al.** *Plant Physiol.,* 1998, vol. 117, 1015-1022
- **Eimert et al.** *Plant Cell,* 1995, vol. 7, 1703-1712
- **Ellenberger et al.** *Genes Dev.,* 1994, vol. 8, 970-980
- **Eulgem et al.** *EMBO J.,* 1999, vol. 18, 4689-4699
- **Eulgem.** *Trends Plant Sci.,* 2000, vol. 5, 199-206
- **Ezcurra et al.** *Plant J.,* 2000, vol. 24, 57-66
- **Fairchild et al.** *Genes Dev.,* vol. 14, 2377-2391
- **Fairman et al.** *Proc. Natl. Acad. Sci. USA,* 1993, vol. 90, 10429-10433
- **Falvo et al.** *Cell,* 1995, vol. 83, 1101-1111
- **Feng ; Doolittle.** *J. Mol. Evol.,* vol. 25, 351-360
- **Ferre-D'Amare et al.** *EMBO J.,* 1994, vol. 13, 180-189
- **Finkelstein et al.** *Plant Cell,* 1998, vol. 10, 1043-1054
- **Fischer ; Droge-Laser.** *Mol. Plant Microbe Interact.,* 2004, vol. 17, 1162-1171

- **Fisher ; Goding.** *EMBO J.,* 1992, vol. 11, 4103-4109
- **Fisher ; Caudy.** *Bioessays,* 1998, vol. 20, 298-306
- **Forsburg ; Guarente.** *Genes Dev.,* 1988, vol. 3, 1166-117
- **Forzani et al.** *J. Biol. Chem.,* 2001, vol. 276, 16731-16738
- **Fowler et al.** *Plant Cell,* 2002, vol. 14, 1675-1679
- **Fowler ; Thomashow.** *Plant Cell,* 2002, vol. 14, 1675-1690
- **Frampton et al.** *Protein Eng.,* 1991, vol. 4, 891-901
- **Frank et al.** *Plant Cell,* 2000, vol. 12, 111-124
- **Freeling ; Hake.** *Genetics,* 1985, vol. 111, 617-634
- **Friedrichsen et al.** *Genetics,* 2002, vol. 162, 1445-1456
- **Fromm et al.** *Bio/Technol.,* 1990, vol. 8, 833-839
- **Fu et al.** *Plant Cell,* 2001, vol. 13, 1791-1802
- **Fuji et al.** *Nat. Struct. Biol.,* 2000, vol. 7, 889-893
- **Fujimoto et al.** *Plant Cell,* 2000, vol. 12, 393-404
- **Fujimoto et al.** *Plant Mol. Biol.,* 2004, vol. 56, 225-239
- **Galigniana et al.** *Mol. Endocrinol.,* 1998, vol. 12, 1903-1913
- **Galway et al.** *Dev. Biol.,* 1994, vol. 166, 740-754
- **Gampala et al.** *International Conference on Arabidopsis Research,* 2004
- **Gancedo.** *Microbiol. Mol. Biol. Rev.,* 1998, vol. 62, 334-361
- **Gaxiola et al.** *Proc. Natl. Acad. Sci. U S A,* 2001, vol. 98, 11444-11449
- **Gelinas et al.** *Prog. Clin. Biol. Res.,* 1985, vol. 191, 125-139
- **Gelvin et al.** Plant Molecular Biology Manual. Kluwer Academic Publishers, 1990
- **Gilmour et al.** *Plant J.,* 1998, vol. 16, 433-442
- **Giraudat et al.** *Plant Cell,* 1992, vol. 4, 1251-1261
- Methods in Plant Molecular Biology and Biotechnology. CRC Press, 1993
- **Goff et al.** *Genes Dev.,* 1992, vol. 6, 864-875
- **Good ; Chen.** *Biol Signals,* 1996, vol. 5, 163-169
- **Goodrich et al.** *Cell,* 1993, vol. 75, 519-530
- **Gordon-Kamm et al.** *Plant Cell,* 1990, vol. 2, 603-618
- **Graf.** *Curr. Opin. Genet. Dev.,* 1992, vol. 2, 249-255
- **Grandori et al.** *Ann. Rev. Cell. Dev. Biol.,* 2000, vol. 16, 653-699
- **Grant et al.** *Mol. Plant Microbe Interact.,* 2003, vol. 16, 669-680
- **Grasser.** *Plant J.,* 1995, vol. 7, 185-192
- **Grasser.** *Plant Mol. Biol.,* 2003, vol. 53, 281-295
- **Gruber et al.** Methods in Plant Molecular Biology and Biotechnology. 1993, 89-119
- **Gu et al.** *Plant Cell,* 2000, vol. 12, 771-786
- **Gu et al.** *Plant Cell,* 2002, vol. 14, 817-831
- **Guiltinan et al.** *Science,* 1990, vol. 250, 267-271
- **Guo et al.** *Plant Mol. Biol.,* 2004, vol. 55, 607-618
- **Gupta et al.** *Plant Mol. Biol.,* 1997, vol. 35, 987-992
- **Gupta et al.** *Plant Mol. Biol.,* 1997, vol. 34, 529-536
- **Gusmaroli et al.** *Gene,* 2001, vol. 264, 173-185
- **Gusmaroli et al.** *Gene,* 2002, vol. 283, 41-48
- **Haake et al.** *Plant Physiol.,* 2002, vol. 130, 639-648
- **Hain et al.** *Mol. Gen. Genet.,* 1985, vol. 199, 161-168
- **Hall et al.** *Plant Physiol.,* 2000, vol. 123, 1449-1458
- **Halliday et al.** *Proc. Natl. Acad. Sci. USA,* 1999, vol. 96, 5832-5837
- **Haymes et al.** Nucleic Acid Hybridization: A Practical Approach. IRL Press, 1985
- **Hanes ; Brent.** *Cell,* 1989, vol. 57, 1275-1283
- **Hanes ; Brent.** *Science,* 1991, vol. 251, 426-430
- **Hao et al.** *J. Biol. Chem.,* 1998, vol. 273, 26857-26861
- **Hao.** *Biochemistry,* 2002, vol. 41, 4202-4208
- **Hasegawa et al.** *Annu. Rev. Plant Mol. Plant Physiol.,* 2000, vol. 51, 463-499
- **Hatch.** *Biochim. Biophys. Acta,* 1987, vol. 895, 81-106
- **Hattori et al.** *Genes Dev.,* 1992, vol. 6, 609-618
- **Hayashi ; Scott.** *Cell,* 1990, vol. 63, 883-894
- **He et al.** *Transgenic Res.,* 2000, vol. 9, 223-227
- **He et al.** *Mol. Plant Microbe Interact.,* 2001, vol. 14, 1453-1457
- **Heim et al.** *Mol. Biol. Evol.,* 2003, vol. 20, 735-747
- **Hein.** *Methods Enzymol.,* 1990, vol. 183, 626-645
- **Heisler et al.** *Development,* 2001, vol. 128, 1089-1098
- **Hempel.** *Development,* 1997, vol. 124, 3845-3853
- **Henikoff ; Henikoff.** *Nucleic Acids Res.,* 1991, vol. 19, 6565-6572

- **Herrera-Estrella et al.** *Nature,* 1983, vol. 303, 209
- **Hiei et al.** *Plant J.,* 1994, vol. 6, 271-282
- **Hiei et al.** *Plant Mol. Biol.,* 1997, vol. 35, 205-218
- **Higgins ; Sharp.** *Gene,* 1988, vol. 73, 237-244
- **Higgins et al.** *Methods Enzymol.,* 1996, vol. 266, 383-402
- **Hirano et al.** *Gene,* 2002, vol. 290, 107-114
- **Hobo et al.** *Proc. Natl. Acad. Sci. USA,* 1999, vol. 96, 15348-15353
- **Hoecker et al.** *Genes Dev.,* 1995, vol. 9, 2459-2469
- **Hsieh et al.** *Proc. Natl. Acad. Sci. USA,* 1998, vol. 95, 13965-13970
- **Hu et al.** *Plant Cell,* 2003, vol. 15, 1951-1961
- **Hu et al.** *Cell Res.,* 2004, vol. 14, 8-15
- **Hung et al.** *Plant Physiol.,* 1998, vol. 117, 73-84
- **Huq ; Quail.** *EMBO J.,* 2002, vol. 21, 2441-2450
- **Huth et al.** *Nat. Struct. Biol.,* 1997, vol. 4, 657-665
- **Hwang ; Goodman.** *Plant J.,* 1995, vol. 8, 37-43
- **Ishida.** *Nature Biotechnol.,* 1990, vol. 14, 745-750
- **Ishiguro ; Nakamura.** *Mol. Gen. Genet.,* 1994, vol. 244, 563-571
- **Ito et al.** *Plant Cell Physiol.,* 1995, vol. 36, 1281-1289
- **Jaglo et al.** *Plant Physiol.,* 2001, vol. 127, 910-917
- **Jaglo-Ottosen et al.** *Science,* 1998, vol. 280, 104-106
- **Jakoby et al.** *Trends Plant Sci.,* 2002, vol. 7, 106-111
- **Jang et al.** *Plant Cell,* 1997, vol. 9, 5-19
- **Jofuku et al.** *Plant Cell,* 1994, vol. 6, 1211-1225
- **Johnson ; McKnight.** *Ann. Rev. Biochem.,* 1989, vol. 58, 799-839
- **Johnson et al.** *Plant Cell,* 2002, vol. 14, 1359-1375
- **Kagaya et al.** *Nucleic Acids Res.,* 1999, vol. 27, 470-478
- **Kaiser et al.** *Science,* 1998, vol. 281, 1202-1206
- **Kashima et al.** *Nature,* 1985, vol. 313, 402-404
- **Kasuga et al.** *Nature Biotechnol.,* 1999, vol. 17, 287-291
- **Kehoe et al.** *Plant Cell,* 1994, vol. 6, 1123-1134
- **Keith et al.** *Plant Cell,* 1994, vol. 6, 589-600
- **Kerstetter et al.** *Plant Cell,* 1994, vol. 6, 1877-1887
- **Kerstetter et al.** *Development,* 1997, vol. 124, 3045-3054
- **Kim ; ShefFrey.** *J. Biol. Chem.,* 1990, vol. 265, 13362-13369
- **Kim et al.** *Mol. Cell. Biol .,* 1996, vol. 16, 4003-4013
- **Kim et al.** *Plant J.,* 2001, vol. 25, 247-259
- **Kim.** *Plant Mol. Biol.,* 2004, vol. 55, 883-904
- **Kimmel.** *Methods Enzymol.,* 1987, vol. 152, 507-511
- **Kirik et al.** *Dev. Biol.,* 2004, vol. 268, 506-513
- **Kirik et al.** *Plant Mol. Biol.,* 2004, vol. 55, 389-398
- **Kissinger et al.** *Cell,* 1990, vol. 63, 579-590
- **Klee.** *Bio/Technology,* 1985, vol. 3, 637-642
- **Klein et al.** *Nature,* 1987, vol. 327, 70-73
- **Knight.** *Int. Rev. Cytol.,* 2000, vol. 195, 269-324
- **Koornneef et al.** Tomato Biotechnology. Alan R. Liss, Inc, 1986, 169-178
- **Krizek.** *Dev. Genet.,* 1999, vol. 25, 224-236
- **Ku et al.** *Proc. Natl. Acad Sci. USA,* 2000, vol. 97, 9121-9126
- **Kunst et al.** *Biochem Soc. Trans.,* 2000, vol. 28, 651-654
- **Kusnetsov et al.** *J. Biol. Chem.,* 1999, vol. 274, 36009-36014
- **Kwak et al.** *Science,* 2005, vol. 307, 1111-1113
- **Kwong.** *Plant Cell,* 2002, vol. 15, 5-18
- **Kyozuka ; Shimamoto.** *Plant Cell Physiol.,* 2002, vol. 43, 130-135
- **Lapik ; Kaufman.** *Plant Cell,* 2003, vol. 15, 1578-1590
- **Larkin et al.** *Ann. Rev. Plant Biol.,* 2003, vol. 54, 403-430
- **Lebel et al.** *Plant J.,* 1998, vol. 16, 223-233
- **Ledent ; Vervoort.** *Genome Res.,* 2001, vol. 11, 754-770
- **Lee ; Schiefelbein.** *Cell,* 1999, vol. 99, 473-483
- **Lee et al.** *Genome Res.,* 2002, vol. 12, 493-502
- **Lee ; Schiefelbein.** *Plant Cell,* 2002, vol. 14, 611-618
- **Lee et al.** *Proc. Natl. Acad. Sci. U S A,* 2003, vol. 100, 2152-2156
- **Lee et al.** *Plant Mol. Biol.,* 2004, vol. 55, 61-81
- **Lefstin ; Yamamoto.** *Nature,* 1998, vol. 392, 885-888
- **Leon-Kloosterziel et al.** *Plant Physiol.,* 1996, vol. 110, 233-240
- **Levens.** *Genes Dev.,* 2003, vol. 17, 1071-1077
- **Li et al.** *Nucleic Acids Res.,* 1992, vol. 20, 1087-1091
- **Li et al.** *EMBO J.,* 1998, vol. 17, 6300-6315
- **Lin et al.** *Nature,* 1991, vol. 353, 569-571

- **Lincoln et al.** *Plant Cell,* 1990, vol. 2, 1071-1080
- **Liscum ; Reed.** *Plant Mol. Biol.,* 2002, vol. 49, 387-400
- **Littlewood ; Evan.** Helix-Loon-Helix Transcription Factors. Oxford University Press, 1998
- **Liu ; Zhu.** *Proc. Natl. Acad. Sci. USA,* 1997, vol. 94, 14960-14964
- **Liu et al.** *Eur. J. Biochem.,* 1999, vol. 262, 247-257
- **Livingston et al.** *Economic and policy implications of wind-borne entry of Asian soybean rust into the United States,* 2004, http://www.ers.usda.gov/Features/SoyBeanRust
- **Long et al.** *Nature,* 1996, vol. 379, 66-69
- **Long ; Barton.** *Dev. Biol.,* 2000, vol. 218, 341-353
- **Lorenzo et al.** *Plant Cell,* 2003, vol. 15, 165-178
- **Lorenzo et al.** *Plant Cell,* 2004, vol. 16, 1938-1950
- **Lotan et al.** *Cell,* 1998, vol. 93, 1195-1205
- **Loulergue et al.** *Gene,* 1998, vol. 225, 47-57
- **Ludwig et al.** *Proc. Natl. Acad. Sci. USA,* 1989, vol. 86, 7092-7096
- **Ludwig et al.** *Cell,* 1990, vol. 62, 849-851
- **Luerssen et al.** *Plant J.,* 1998, vol. 15, 755-764
- **Luger et al.** *Nature,* 1997, vol. 389, 251-260
- **Lynch et al.** *Phytopathol.,* 2002, vol. 92, 33
- **Ma et al.** *Cell,* 1994, vol. 77, 451-459
- **Mackay ; Crossley.** *Trends Biochem. Sci.,* 1998, vol. 23, 1-4
- **Maity ; de Crombrugghe.** *Trends Biochem. Sci.,* 1998, vol. 23, 174-178
- **Maleck.** *Nat. Genet.,* 2000, vol. 26, 403-410
- **Mandel.** *Nature,* 1992, vol. 360, 273-277
- **Mandel et al.** *Cell,* 1992, vol. 71, 133-143
- **Mantovani.** *Nucleic Acids Res.,* 1998, vol. 26, 1135-1143
- **Mantovani.** *Gene,* 1999, vol. 239, 15-27
- **Mare et al.** *Plant Mol. Biol.,* 2004, vol. 55, 399-416
- **Martin ; Paz-Ares.** *Trends Genet.,* 1997, vol. 13, 67-73
- **Martinez-Garcia ; Quail.** *Plant J.,* 1999, vol. 18, 173-183
- **Martinez-Garcia et al.** *Science,* 2000, vol. 288, 859-863
- **Masiero et al.** *J. Biol. Chem.,* 2002, vol. 277, 26429-26435
- **Massari ; Murre.** *Mol. Cell. Biol.,* 2000, vol. 20, 429-440
- **Masucci J. et al.** *Development,* 1996, vol. 122, 1253-1260
- **Mazon et al.** *Eur. J. Biochem.,* 1982, vol. 127, 605-608
- **McCarty et al.** *Plant Cell,* 1989, vol. 1, 523-532
- **McCarty et al.** *Cell,* 1991, vol. 66, 895-905
- **McCue ; Hanson.** *Trends Biotechnol.,* 1990, vol. 8, 358-362
- **McNabb et al.** *Genes Dev.,* 1995, vol. 9, 47-58
- **McNabb et al.** *Mol. Cell. Biol.,* 1997, vol. 17, 7008-7018
- **Meijer et al.** *Plant Mol. Biol.,* 1996, vol. 31, 607-618
- **Meinke.** *Science,* 1992, vol. 258, 1647-1650
- **Meinke et al.** *Plant Cell,* 1994, vol. 6, 1049-1064
- **Merlot et al.** *Plant J.,* 2001, vol. 25, 295-303
- **Mewes et al.** *Nucleic Acids Res.,* 2002, vol. 30, 31-34
- **Meyers.** Molecular Biology and Biotechnology. Wiley VCH, 1995, 856-853
- **Miki et al.** Methods in Plant Molecular Biology and Biotechnology. CRC Press, Inc, 1993, 67-88
- **Miles et al.** *Soybean rust: is the U.S. soybean crop at risk?,* http://www.apsnet.org/online/feature/rust
- **Miyoshi et al.** *Plant J.,* 2003, vol. 36, 532-540
- **Mizukama ; Fischer.** *Proc. Natl. Acad. Sci. USA,* 2000, vol. 97, 942-947
- **Mizukami.** *Curr Opinion Plant Biol.,* 2001, vol. 4, 533-539
- **Mohr ; Cahill.** *Functional Plant Biology,* 2003, vol. 30, 461-469
- **Mount.** Bioinformatics: Sequence and Genome Analysis. Cold Spring Harbor Laboratory Press, 2001, 543
- **Müller et al.** *Plant J.,* 2001, vol. 28, 169-179
- **Munkvold.** *Annu. Rev. Phytopathol.,* 2003, vol. 41, 99-116
- **Murre et al.** *Cell,* 1989, vol. 56, 777-783
- **Myers et al.** *Science,* 1986, vol. 232, 613-618
- **Nair ; Burley.** *Nature,* 2000, vol. 404, 715, 717-718
- **Nakshatri.** *J. Biol. Chem.,* 1996, vol. 271, 28784-28791
- **Nambara et al.** *Development,* 1995, vol. 121, 629-636
- **Nandi et al.** *Curr. Biol.,* 2000, vol. 10, 215-218
- **Nath et al.** *Science,* 2003, vol. 299, 1404-1407
- **Nesi et al.** *Plant Cell,* 2000, vol. 12, 1863-1878
- **Ni et al.** *Cell,* 1998, vol. 95, 657-667
- **Nieto-Sotelo ; Quail.** *Biochem. Soc. Symp.,* 1994, vol. 60, 265-275
- *Managing Row Crop Diseases in Drought Years,* 2002, http://www.ag.ndsu.nodak.edu/drought/ds-10-97.htm

- *Small Grain Diseases: Management of Those More Common and Severe in Dry Years,* 2004, http://www.ag.ndsu.nodak.edu/drought/ds-01-02.htm
- **Novillo et al.** *Proc. Natl. Acad. Sci. USA,* 2004, vol. 101, 3985-3990
- **Odell.** *Nature,* 1985, vol. 313, 810-812
- **Ohme-Takagi ; Shinshi.** *Plant Cell,* 1995, vol. 7, 173-182
- **Ohta et al.** *Plant Cell,* 2001, vol. 13, 1959-1968
- **Okamuro et al.** *Proc. Natl. Acad. Sci. USA,* 1997, vol. 94, 7076-7081
- **Olesen ; Guarente.** *Genes Dev.,* 1990, vol. 4, 1714-1729
- **Onate et al.** *Mol. Cell. Biol.,* 1994, vol. 14, 3376-3391
- **Onate-Sanchez ; Singh.** *Plant Physiol.,* 2002, vol. 128, 1313-1322
- **Ooms et al.** *Plant Physiol.,* 1993, vol. 102, 1185-1191
- **Palatnik et al.** *Nature,* 2003, vol. 425, 257-263
- **Parcy ; Giraudat.** *Plant J.,* 1997, vol. 11, 693-702
- **Parcy et al.** *Plant Cell,* 1997, vol. 9, 1265-1277
- **Park.** *Plant Cell,* 2001, vol. 13, 1035-1046
- **Payne et al.** *Genetics,* 2000, vol. 156, 1349-1362
- **Peng et al.** *Genes Development,* 1997, vol. 11, 3194-3205
- **Peng et al.** *Nature,* 1999, vol. 400, 256-261
- **Pinkham ; Guarente.** *Mol. Cell Biol.,* 1985, vol. 5, 3410-3416
- **Pnueli et al.** *Plant J.,* 2002, vol. 31, 319-330
- **Porra et al.** *Biochim. Biophys. Acta,* 1989, vol. 975, 384-394
- **Pourtau et al.** *Planta,* 2004, vol. 219, 765-772
- **Putterill et al.** *Cell,* 1995, vol. 80, 847-857
- **Rajani ; Sundaresan.** *Curr. Biol.,* 2001, vol. 11, 1914-1922
- **Ratcliffe et al.** *Plant Physiol.,* 2001, vol. 126, 122-132
- **Reeves ; Nissen.** *J. Biol. Chem.,* 1990, vol. 265, 8573-8582
- **Reeves.** *Gene,* 2001, vol. 277, 63-81
- **Reeves ; Beckerbauer.** *Biochim Biophys Acta,* 2001, vol. 1519, 13-29
- **Reidt et al.** *Plant J.,* 2000, vol. 21, 401-408
- **Remm et al.** *J. Mol. Biol.,* 2001, vol. 314, 1041-1052
- **Reuber.** *Plant J.,* 1998, vol. 16, 473-485
- **Riechmann ; Meyerowitz.** *Biol. Chem.,* 1998, vol. 379, 633-646
- **Riechmann et al.** *Science,* 2000, vol. 290, 2105-2110
- **Riechmann ; Ratcliffe.** *Curr. Opin. Plant Biol.,* 2000, vol. 3, 423-434
- **Rieger et al.** Glossary of Genetics and Cytogenetics: Classical and Molecular. Springer Verlag, 1976
- **Rieping ; Schoffl.** *Mol. Gen. Genet.,* 1992, vol. 231, 226-232
- **Rigaut et al.** *Nat. Biotechnol.,* 1999, vol. 17, 1030-1032
- **Robatzek ; Somssich.** *Genes Dev.,* 2002, vol. 16, 1139-1149
- **Robinson et al.** *Nucleic Acids Res.,* 2000, vol. 28, 4460-4466
- **Robson et al.** *Plant J.,* 2001, vol. 28, 619-631
- **Rohila et al.** *Plant J.,* 2004, vol. 38, 172-181
- **Romier et al.** *J. Biol. Chem.,* 2003, vol. 278, 1336-1345
- **Rushton et al.** *Plant Mol. Biol.,* 1995, vol. 29, 691-702
- **Rushton et al.** *EMBO J.,* 1996, vol. 15, 5690-5700
- **Sadowski et al.** *Nature,* 1988, vol. 335, 563-564
- **Saijo et al.** *Plant J.,* 2000, vol. 23, 319-327
- **Sakuma et al.** *Biochem. Biophys. Res. Comm.,* 2002, vol. 290, 998-1009
- **Saleki et al.** *Plant Physiol.,* 1993, vol. 101, 839-845
- **Salsi et al.** *J. Biol. Chem.,* 2003, vol. 278, 6642-6650
- **Sambrook et al.** Molecular Cloning: A Laboratory Manual. Cold Spring Harbor Laboratory, 1989
- **Sanchez ; Cejudo.** *Plant Physiol.,* 2003, vol. 132, 949-957
- **Sanders et al.** *Plant Cell,* 1999, vol. 11, 691-706
- **Sanford et al.** *Part. Sci. Technol.,* 1987, vol. 5, 27-37
- **Sanford.** *Methods Enzymol.,* 1993, vol. 217, 483-509
- **Schaffer et al.** *Cell,* 1998, vol. 93, 1219-1229
- **Schellmann et al.** *EMBO J.,* 2002, vol. 21, 5036-5046
- **Schindler et al.** *Plant J.,* 1993, vol. 4, 137-150
- **Schnittger et al.** *Development,* 1998, vol. 125, 2283-2289
- **Schnittger et al.** *Plant Cell,* 1999, vol. 11, 1105-1116
- **Schoof et al.** *Cell,* 2000, vol. 100, 635-644
- **Sessa et al.** Molecular genetic analysis of plant development and metabolism. Springer Verlag, 1994
- **Sharp ; LeNoble.** *J. Exp. Bot.,* 2002, vol. 53, 33-37
- **Sheen.** *Ann. Rev. Plant Physiol. Plant Mol. Biol.,* 1999, vol. 50, 187-217
- **Shimizu et al.** *EMBO J.,* 1997, vol. 16, 4689-4697

- **Shin et al.** *Mol Plant Microbe Interact,* 2002, vol. 15, 983-989
- **Shirakata et al.** *Genes Dev.,* 1993, vol. 7, 2456-2470
- **Shpaer.** *Methods Mol. Biol.,* 1997, vol. 70, 173-187
- **Silver et al.** *Mol. Cell. Biol.,* 2003, vol. 23, 5989-5999
- **Sinha et al.** *Mol. Cell. Biol.,* 1996, vol. 16, 328-337
- **Sivamani et al.** *Plant Science,* 2000, vol. 155, 1-9
- **Smalle et al.** *Proc. Natl. Acad. Sci. USA.,* 1998, vol. 95, 3318-3322
- **Smeekens.** *Curr. Opin. Plant Biol.,* 1998, vol. 1, 230-234
- **Smith et al.** *Protein Engineering,* 1992, vol. 5, 35-51
- **Smolen et al.** *Genetics,* 2002, vol. 161, 1235-1246
- **Soltis et al.** *Ann. Missouri Bot. Gard.,* 1997, vol. 84, 1-49
- **Solano et al.** *Genes Dev.,* 1998, vol. 12, 3703-3714
- **Sonnhammer et al.** *Proteins,* 1997, vol. 28, 405-420
- **Sorensen et al.** *Plant J.,* 2003, vol. 33, 413-423
- **Spencer et al.** *Plant Mol. Biol.,* 1994, vol. 24, 51-61
- **Spollen et al.** *Plant Physiol.,* 2000, vol. 122, 967-976
- **Stockinger et al.** *Proc. Natl. Acad. Sci. USA,* 1997, vol. 94, 1035-1040
- **Stone et al.** *Proc. Natl. Acad. Sci. USA,* 2001, vol. 98, 11806-11811
- **Surpin et al.** *Plant Cell,* 2002, vol. 14, S327-S338
- **Suzuki et al.** *Plant Cell,* 1997, vol. 9, 799-807
- **Suzuki et al.** *Plant J.,* 2001, vol. 28, 409-418
- **Suzuki et al.** *Plant Physiol.,* 2003, vol. 132, 1664-1677
- **Svensson et al.** *Arch. Biochem. Biophys,* 2003, vol. 414, 180-188
- **Tahtiharju ; Palva.** *Plant J,* 2001, vol. 26, 461-470
- **Tamminen et al.** *Plant J.,* 2001, vol. 25, 1-8
- **Tanimoto et al.** *Plant J.,* 1995, vol. 8, 943-948
- **Tasanen et al.** *J. Biol. Chem.,* 1992, vol. 267, 11513-11519
- **Taylor ; Scheuring.** *Mol. Gen. Genet.,* 1994, vol. 243, 148-157
- **Tepperman et al.** *Proc. Natl. Acad. Sci. USA,* 2001, vol. 98, 9437-9442
- **Thaler ; Bostock.** *Ecology,* 2003, vol. 85, 48-58
- **Thoma.** *Plant Physiol.,* 1994, vol. 105, 35-45
- **Thompson et al.** *Nucleic Acids Res.,* 1994, vol. 22, 4673-4680
- **Tiwari et al.** *Plant Cell,* 2001, vol. 13, 2809-2822
- **Tiwari et al.** *Plant Cell,* 2003, vol. 15, 533-543
- **Toledo-Ortiz et al.** *Plant Cell,* 2003, vol. 15, 1749-1770
- **Tournier et al.** *FEBS Lett.,* 2003, vol. 550, 149-154
- **Toyama et al.** *Plant Cell Physiol.,* 1999, vol. 40, 1087-1092
- **Truemit ; Sauer.** *Planta,* 1995, vol. 196, 564-570
- **Tudge.** The Variety of Life. Oxford University Press, 2000, 547-606
- **Ulmasov et al.** *Science,* 1997, vol. 276, 1865-1868
- **Vasil et al.** *Bio/Technol.,* 1992, vol. 10, 667-674
- **Vasil et al.** *Bio/Technol.,* 1993, vol. 11, 1553-1558
- **Vasil.** *Plant Mol. Biol.,* 1994, vol. 25, 925-937
- **Verslues ; Sharp.** *Plant Physiol.,* 1999, vol. 119, 1349-1360
- **Vicient et al.** *J. Exp. Bot.,* 2000, vol. 51, 995-1003
- **Vollbrecht et al.** *Nature,* 1991, vol. 350, 241-243
- **Wada et al.** *Science,* 1997, vol. 277, 1113-1116
- **Wada.** *Development,* 2002, vol. 129, 5409-5419
- **Wahl ; Berger.** *Methods Enzymol.,* 1987, vol. 152, 399-407
- **Wan ; Lemeaux.** *Plant Physiol.,* 1994, vol. 104, 37-48
- **Wang et al.** *Plant Cell,* 1997, vol. 9, 491-507
- **Wang.** *Plant J.,* 1998, vol. 16, 515-522
- **Wanner ; Gruissem.** *Plant Cell,* 1991, vol. 3, 1289-1303
- **Waterhouse et al.** *Trends Plant Sci.,* 2001, vol. 6, 297-301
- **Waterston et al.** *Nature,* 2002, vol. 420, 520-562
- **Weeks et al.** *Plant Physiol.,* 1993, vol. 102, 1077-1084
- **Weigel et al.** *Cell,* 1992, vol. 69, 843-859
- **Weigel.** *Plant Cell,* 1995, vol. 7, 388-389
- **Weigel et al.** *Plant Physiol.,* 2000, vol. 122, 1003-1013
- **Weigel ; Nilsson.** *Nature,* 1995, vol. 377, 482-500
- **Weissbach ; Weissbach.** Methods for Plant Molecular Biology. Academic Press, 1989
- **Wendler et al.** *J. Biol. Chem.,* 1997, vol. 272, 8482-8489
- **Wesley et al.** *Plant J.,* 2001, vol. 27, 581-590
- **West et al.** *Plant Cell,* 1994, vol. 6, 1731-1745

- **Westhoff ; Gowik.** *Ann. Bot. (London),* 2004, vol. 93, 13-23
- **Windhovel.** *Plant Mol. Biol.,* 2001, vol. 45, 201-214
- **Wobus ; Weber.** *Curr. Opin. Plant Biol.,* 1999, vol. 2, 33-38
- **Wolberger et al.** *Cell,* 1991, vol. 67, 517-528
- **Wrather ; Sweets.** *Aflatoxin in Corn.,* 2004, http://aes.missouri.edu/delta/croppest/aflacorn.stm
- **Wu et al.** *Plant Cell,* 1996, vol. 8, 617-627
- **Xin ; Browse.** *Proc. Natl. Acad Sci. USA,* 1998, vol. 95, 7799-7804
- **Xing et al.** *EMBO J.,* 1993, vol. 12, 4647-4655
- **Xiong et al.** *Genes Dev.,* 2001, vol. 15, 1971-1984
- **Xiong ; Zhu.** *Plant Cell Environ.,* 2002, vol. 25, 131-139
- **Xiong ; Yang.** *Plant Cell,* 2003, vol. 15, 745-759
- **Xu et al.** *Plant Physiol.,* 1996, vol. 110, 249-257
- **Xu et al.** *Proc. Natl. Acad Sci USA,* 2001, vol. 98, 15089-15094
- **Yamada et al.** *FEBS Lett.,* 1999, vol. 460, 41-45
- **Yamada et al.** *Biochem. Biophys. Res. Commun.,* 1999, vol. 261, 614-621
- **Yamada et al.** *Biochem J.,* 2003, vol. 373, 167-178
- **Yamaguchi-Shinozaki ; Shinozaki.** *Mol. Gen. Genet.,* 1993, vol. 236, 331-340
- **Yamasaki et al.** *Plant Cell,* 2005, vol. 17, 944-956
- **Yang et al.** *Plant J.,* 1999, vol. 18, 141-149
- **Yi et al.** *Plant Physiol.,* 2004, vol. 136, 2862-2874
- **Yu et al.** *Plant Cell,* 2001, vol. 13, 1527-1540
- **Yun et al.** *J. Biol. Chem.,* 2003, vol. 278, 36966-36972
- **Zhang et al.** *Bio/Technology,* 1991, vol. 9, 996-997
- **Zhang et al.** *Planta,* 2002, vol. 215, 191-194
- **Zhang et al.** *Development,* 2003, vol. 130, 4859-4869
- **Zhang ; Wang.** *BMC Evol. Biol.,* 2005, vol. 5, 1
- **Zhong ; Ye.** *Plant Phys.,* 2001, vol. 126, 549-563
- **Zhou et al.** *Nature,* 1995, vol. 376, 771-774
- **Zhou et al.** *Cell,* 1995, vol. 83, 925-935
- **Zhou et al.** *EMBO J.,* 1997, vol. 16, 3207-3218
- **Zhou ; Lee.** *J. Natl. Cancer Inst.,* 1998, vol. 90, 381-388
- **Zhu et al.** *Plant Cell,* 1998, vol. 10, 1181-1191
- **Zou et al.** *J. Biol. Chem.,* 2004, vol. 279, 55770-5577